(19) Europäisches Patentamt
European Patent Office
Office européen des brevets

(11) EP 3 845 538 B1

# (12) EUROPEAN PATENT SPECIFICATION

(45) Date of publication and mention of the grant of the patent:
**08.11.2023 Bulletin 2023/45**

(21) Application number: **19870148.4**

(22) Date of filing: **30.09.2019**

(51) International Patent Classification (IPC):
***C07D 487/04*** (2006.01) ***C07D 403/04*** (2006.01)
***A61K 31/4985*** (2006.01) ***A61P 35/00*** (2006.01)

(52) Cooperative Patent Classification (CPC):
**A61P 35/00; C07B 59/002; C07D 487/04**

(86) International application number:
**PCT/CN2019/109293**

(87) International publication number:
**WO 2020/073862 (16.04.2020 Gazette 2020/16)**

## (54) DIHYDROIMIDAZOPYRAZINONE COMPOUND, COMPOSITION INCLUDING SAME, AND USE THEREOF

DIHYDROIMIDAZOPYRAZINONVERBINDUNG, ZUSAMMENSETZUNGEN DAMIT UND VERWENDUNGEN DAVON

COMPOSÉ DE DIHYDROIMIDAZOPYRAZINONE, COMPOSITION LE COMPRENANT ET SON UTILISATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **10.10.2018 CN 201811178571**

(43) Date of publication of application:
**07.07.2021 Bulletin 2021/27**

(73) Proprietor: **Shenzhen TargetRx, Inc.**
**Shenzhen, Guangdong 518057 (CN)**

(72) Inventors:
- **WANG, Yihan**
  **Shenzhen, Guangdong 518057 (CN)**
- **REN, Xingye**
  **Shenzhen, Guangdong 518057 (CN)**

(74) Representative: **Novitas Patent AB**
**P.O. Box 55557**
**102 04 Stockholm (SE)**

(56) References cited:
**WO-A1-2017/080979** **CN-A- 107 074 874**
**CN-A- 108 349 983** **US-A1- 2013 203 765**

- **Kang, Fang-yuan et al.: "Synthesis of Deuterated AZD9291", Chinese Journal of Synthetic Chemistry, vol. 24, no. 3, 31 December 2016 (2016-12-31), pages 263-265+276, XP055797464,**
- **Liu, Jie: "Deuterated Drugs Progress", Chemical Engineering Design Communications, vol. 42, no. 4, 30 April 2016 (2016-04-30) , pages 199-238, XP009521404,**
- **SHARMA, RAMAN: "Deuterium Isotope Effects on Drug Pharmacokinetics. I. System-Dependent Effects of Specific Deuteration with Aldehyde Oxidase Cleared Drugs", DRUG METABOLISM AND DISPOSITION, 31 December 2012 (2012-12-31), XP055259415,**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Processed by Luminess, 75001 PARIS (FR)

## Description

### TECHNICAL FIELD

The present disclosure belongs to the pharmaceutical technical field, and particularly relates to a dihydroimidazopyrazinone compound, to a pharmaceutical composition containing the same, and to a use thereof. More specifically, the present disclosure relates to some deuterium substituted (R)-7-(3,4-difluorobenzyl)-6-(methoxymethyl)-2-(5-methyl-2-((1-methyl-1H-pyrazol-5-yl)amino)pyrimidin-4-yl)-6,7-dihydroimidazo[1,2-a]pyrazin-8(5H)-one. These deuterium-substituted compounds and compositions thereof can be used to treat proliferative diseases regulated by RAS/RAF/MEK/ERK kinases, and these deuterium-substituted compounds have better pharmacokinetic properties.

### BACKGROUND OF THE INVENTION

Protein kinases play a key regulatory role in almost every aspect of cell biology. The mammalian MAP kinases consist of cytoplasmic protein serine/threonine kinases that participate in the transduction of cellular signals from the plasma membrane to the nucleus. There are multiple MAPK signalling cascades each consisting of 3 components: a MAP3K, a MAP2K and a MAPK. The activated MAP kinases phosphorylate numerous substrates including other protein kinases, protein phosphatases, transcription factors and other functional proteins. The RAS-RAF-MEK-ERK signalling cascade participates in the regulation of cell cycle progression, cell proliferation, survival, metabolism and transcription.

ERK1 and ERK2 are ubiquitously expressed MAPK kinases that participate in the RAS-RAF-MEK-ERK signalling cascade, which both contain unique N- and C-terminal extensions that provide signalling specificity, in addition to an insertion of 31 amino acid residues within the kinase domain that provides additional functional specificity. ERK1 and ERK2 are activated in a wide variety of cell types by mitogenic and other stimuli, resulting in activation of multiple isoforms of RAS (HRAS, NRAS and KRAS). Activation of RAS leads to recruitment and activation of RAF isoforms (ARAF, BRAF and CRAF) and subsequent activation of MEK1 and MEK2, which are dual-specificity protein kinases that mediate the phosphorylation of tyrosine and threonine of ERK1 and ERK2. ERK1 and ERK2 have a large number of identified cytoplasmic and nuclear substrates (Yoon S, Seger R. The extracellular signal-regulated kinase: multiple substrates regulate diverse cellular functions; Growth Factors 2006, 24, 21-44).

The RAS-RAF-MEK-ERK signalling cascade is deregulated in a variety of diseases including brain injury, cancer, cardiac hypertrophy, diabetes and inflammation. Specifically, mutations in KRAS occur in approximately 58% of pancreatic cancer, 33% of colorectal cancer and 31% of biliary cancers, and NRAS mutations in 18% of melanomas. Oncogenic mutations in RAS result in elevated ERK activity across multiple tumors. In addition, BRAF mutations occur in approximately 40-60% of melanomas, 40% of thyroid cancers and 20% of colorectal cancers. These observations indicate that the RAS-RAF-MEK-ERK signalling cascade is an attractive pathway for anti-cancer therapies against a broad range of human tumors.

AZD0364 (the chemical name is (A)-7-(3,4-difluorobenzyl)-6-(methoxymethyl)-2-(5-methyl-2-((1-methyl-1*H*-pyrazol-5-yl)amino)pyrimidin-4-yl)-6,7-dihydroimidazo[1,2-a]pyrazin-8(5H)-one, with the formula below) is an ERK1/2 inhibitor developed by AstraZeneca. It has high inhibitory activity of ERK over other kinases in the RAS-RAF-MEK-ERK signaling cascade. It is disclosed in International Patent Publication No. WO2017/080979.

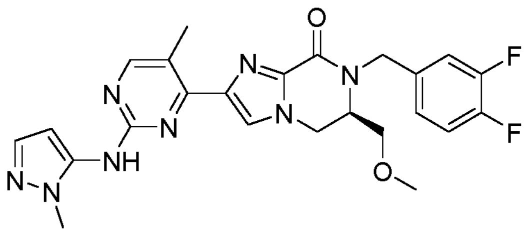

AZD0364

Poor absorption, distribution, metabolism, and/or excretion (ADME) properties are known to be the primary causes of clinical trial failure of many drug candidates. At present, many marketed drugs have limitations on their application due to their poor ADME properties. The rapid metabolism makes many drugs, which could have been effective in treating diseases otherwise, difficult to be used as drugs due to their rapid clearance from the body. Although a frequent or high-dose administration may solve the problem of rapid drug clearance, this approach will bring about problems such as poor compliance of patients, side effects caused by high-dose administration, and increased treatment costs. In addition, drugs that are rapidly metabolized may also expose the patients to undesirable toxic or reactive metabolites.

Therefore, there is still a need in the art to develop compounds that have selective inhibitory activity or better pharmacodynamics/pharmacokinetics for use in the treatment of ERK-mediated disorders. The present disclosure provides a new type of ERK inhibitor based on AZD0364 as the parent compound with deuteration modification, which reduces or eliminates undesirable metabolites; increases the half-life of the parent compound; reduces the number of doses required for the desired effect; reduces the number of doses required to achieve the desired effect; increases the formation of active metabolites (if formed); reduces the production of harmful metabolites in specific tissues; produces drugs that are more effective and/or safer for multi-drugs administration (regardless of whether the multi-drugs are intentional or not) through the deuteration strategy.

## SUMMARY OF THE INVENTION

In view of the above technical problems, the present disclosure discloses a novel deuterium-substituted dihydroimidazopyrazinone compound and a composition containing the compound and a use thereof, which has higher inhibitory activity and selectivity to ERK kinase (especially ERK2 kinase), and has lower side effects, better pharmacokinetic performance at the same time, which can be used for the treatment and/or prevention of proliferative diseases regulated by RAS/RAF/MEK/ERK kinases.

As used herein, the term "compounds of the present disclosure" refers to compounds represented by formulae (I) to (IV) (including subsets of each formula). The term also includes tautomers, stereoisomers, crystal forms, pharmaceutically acceptable salts, hydrates and solvates of the compounds of formulae (I) to (IV).

In this regard, the present disclosure adopts the following technical solutions:

In the first aspect, the present disclosure provides a compound of formula (I):

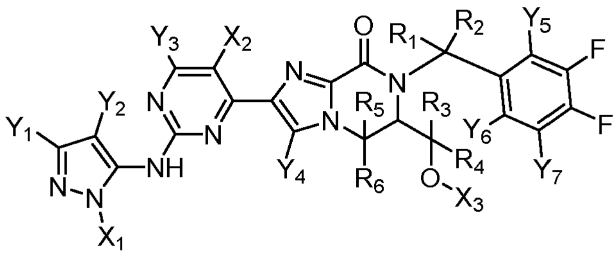

formula (I)

wherein,

$Y_1$, $Y_2$, $Y_3$, $Y_4$, $Y_5$, $Y_6$ and $Y_7$ are each independently selected from hydrogen, deuterium, halogen or trifluoromethyl;
$R_1$, $R_2$, $R_3$, $R_4$, $R_5$ and $R_6$ are each independently selected from hydrogen or deuterium;
$X_1$, $X_2$ and $X_3$ are each independently selected from $CH_3$, $CD_3$, $CHD_2$ or $CH_2D$;
provided that the above compound has at least one deuterium,
or the tautomers, stereoisomers, crystal forms, pharmaceutically acceptable salts, hydrates or solvates thereof.

In another aspect, the present disclosure provides a pharmaceutical composition containing a compound of the present disclosure and pharmaceutically acceptable excipient(s). In a specific embodiment, the compound of the present disclosure is provided in the pharmaceutical composition in an effective amount. In a specific embodiment, the compound of the present disclosure is provided in a therapeutically effective amount. In a specific embodiment, the compound of the present disclosure is provided in a prophylactically effective amount. In a specific embodiment, the pharmaceutical composition further contains additional therapeutic agent(s), and the additional therapeutic agents is(are) selected from MEK inhibitors.

In another aspect, the present disclosure provides a method for preparing the pharmaceutical composition as described above, including the following steps: a pharmaceutically acceptable excipient is mixed with the compound of the present disclosure to form a pharmaceutical composition.

In another aspect, the present disclosure provides the compound of the present disclosure or the above-mentioned composition for use in the treatment and/or prevention of proliferative diseases. In a specific embodiment, the proliferative disease is a disorder characterized by RAS/RAF/MEK/ERK kinase regulation. In a specific embodiment, the proliferative disease is a disorder characterized by ERK kinase mediation. In a specific embodiment, the proliferative disease is a disorder characterized by ERK2 kinase mediation. In a specific embodiment, the proliferative disease is a disorder characterized by KRAS kinase mediation. In a specific embodiment, the proliferative disease is a disorder characterized by BRAF mutant kinase mediation. In a specific embodiment, the proliferative disease is a disorder characterized by BRAF V600E mutant kinase mediation. In specific embodiments, the compound is administered orally, subcutaneously, intravenously, or intramuscularly. In a specific embodiment, the compound is administered chronically.

From the following specific embodiments, examples and claims, other objects and advantages of the present disclosure will be apparent to those skilled in the art.

## DEFINITIONS

Herein, unless otherwise specified, "deuterated" refers to that one or more hydrogens in a compound or group are replaced by deuterium; deuteration can be mono-substitution, disubstitution, multi-substitution or per-substitution. The terms "one or more deuterated" and "deuterated one or more times" are used interchangeably.

Herein, unless otherwise specified, "non-deuterated compound" refers to a compound wherein the content of the deuterium atom is not higher than the natural content of the deuterium isotope (0.015%).

The term "pharmaceutically acceptable salt" refers to those salts which are, within the scope of sound medical judgment, suitable for use in contact with the tissues of humans and lower animals without undue toxicity, irritation, allergic response and the like, and are commensurate with a reasonable benefit/risk ratio. Pharmaceutically acceptable salts are well known in the art. For example, Berge et al., describes pharmaceutically acceptable salts in detail in J. Pharmaceutical Sciences (1977) 66: 1-19. Pharmaceutically acceptable salts of the compounds of the present disclosure include those derived from suitable inorganic and organic acids and inorganic and organic bases.

The compounds of the present disclosure may be in amorphous or crystalline form. In addition, the compounds of the present disclosure may exist in one or more crystalline forms. Therefore, the present disclosure includes all amorphous or crystalline forms of the compounds of the present disclosure within its scope. The term "crystal form" refers to the different arrangement of chemical drug molecules, which is generally presented as the existence form of the drug raw materials in the solid state. A drug may exist in a variety of crystal forms, and different crystal forms of the same drug may have different dissolution and absorption properties in vivo, thereby affecting the dissolution and release of the formulation.

The term "crystal form" refers to the different arrangement of chemical drug molecules, which is generally presented as the existence form of the drug raw materials in the solid state. A drug may exist in a variety of crystal forms, and different crystal forms of the same drug may have different dissolution and absorption properties in vivo, thereby affecting the dissolution and release of the formulation.

As used herein, the term "subject" includes, but is not limited to, humans (*i.e.*, a male or female of any age group, *e.g.,* a pediatric subject (*e.g,* infant, child, adolescent) or adult subject (*e.g.,* young adult, middle-aged adult or elderly adult)) and/or a non-human animal, *e.g.,* a mammal such as primates (*e.g.*, cynomolgus monkeys, rhesus monkeys), cattle, pigs, horses, sheep, goats, rodents, cats, and/or dogs. In certain embodiments, the subject is a human. In certain embodiments, the subject is a non-human animal.

"Disease", "disorder" and "condition" are used interchangeably herein.

As used herein, unless otherwise specified, the terms "treat", "treating", and "treatment" contemplate an action that occurs while a subject is suffering from a particular disease, disorder, or condition, which reduces the severity of the disease, disorder or condition, or retards or slows the progression of the disease, disorder or condition ("therapeutic treatment"). The terms also contemplate an action that occurs before a subject begins to suffer from a specific disease, disorder or condition ("prophylactic treatment").

In general, the "effective amount" of a compound refers to an amount sufficient to elicit the desired biological response. As will be appreciated by those of ordinary skill in this art, the effective amount of a compound disclosed herein may vary depending on such factors as the desired biological endpoint, the pharmacokinetics of the compound, the disease being treated, the mode of administration, and the age, health, and condition of the subject. An effective amount encompasses therapeutically and prophylactically effective amount.

As used herein, unless otherwise specified, a "therapeutically effective amount" of a compound is an amount sufficient to provide a therapeutic benefit in the treatment of a disease, disorder or condition, or to delay or minimize one or more symptoms associated with the disease, disorder or condition. A therapeutically effective amount of a compound means an amount of therapeutic agent, alone or in combination with other therapies, which provides a therapeutic benefit in the treatment of the disease, disorder or condition. The term "therapeutically effective amount" can encompass an amount that improves overall therapy, reduces or avoids symptoms or causes of disease or condition, or enhances the therapeutic efficacy of another therapeutic agent.

As used herein, unless otherwise specified, a "prophylactically effective amount" of a compound is an amount sufficient to prevent a disease, disorder or condition, or one or more symptoms associated with the disease, disorder or condition, or prevent its recurrence. A prophylactically effective amount of a compound means an amount of a therapeutic agent, alone or in combination with other agents, which provides a prophylactic benefit in the prevention of the disease, disorder or condition. The term "prophylactically effective amount" can encompass an amount that improves overall prophylaxis or enhances the prophylactic efficacy of another prophylactic agent.

"Combination" and related terms mean the simultaneous or sequential administration of a compound of the present disclosure. For example, a compound disclosed herein may be administered simultaneously or sequentially with

another therapeutic agent in separate unit dosage forms, or together with another therapeutic agent in a single unit dosage form.

**SPECIFIC EMBODIMENTS**

Compound

In one embodiment, the present disclosure relates to a compound of formula (I), or the tautomers, stereoisomers, crystal forms, pharmaceutically acceptable salts, hydrates or solvates thereof:

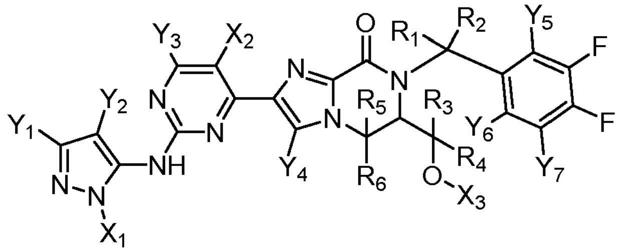

formula (I)

wherein,
$Y_1$, $Y_2$, $Y_3$, $Y_4$, $Y_5$, $Y_6$ and $Y_7$ are each independently selected from hydrogen, deuterium, halogen or trifluoromethyl;
$R_1$, $R_2$, $R_3$, $R_4$, $R_5$ and $R_6$ are each independently selected from hydrogen or deuterium;
$X_1$, $X_2$ and $X_3$ are each independently selected from $CH_3$, $CD_3$, $CHD_2$ or $CH_2D$;
provided that the above compound has at least one deuterium.

In a specific embodiment, the isotope content of deuterium at the deuterated position is at least greater than the 0.015% natural isotope content of deuterium, alternatively greater than 30%, alternatively greater than 50%, alternatively greater than 75%, alternatively greater than 95%, alternatively greater than 99%.

Specifically, for $Y_1$, $Y_2$, $Y_3$, $Y_4$, $Y_5$, $Y_6$, $Y_7$, $R_1$, $R_2$, $R_3$, $R_4$, $R_5$, $R_6$, $X_1$, $X_2$ and $X_3$ in the present disclosure, the isotope content of deuterium at each deuterated position is at least 5%, alternatively 10%, alternatively 15%, alternatively 20%, alternatively 25%, alternatively 30%, alternatively 35%, alternatively 40%, alternatively 45%, alternatively 50%, alternatively 55%, alternatively 60%, alternatively 65%, alternatively 70%, alternatively 75%, alternatively 80%, alternatively 85%, alternatively 90%, alternatively 95%, alternatively 99% greater than the natural isotope content of deuterium.

In another specific embodiment, the compound of the present disclosure contains at least one deuterium atom, alternatively contains two deuterium atoms, alternatively contains three deuterium atoms, alternatively contains four deuterium atoms, alternatively contains five deuterium atoms, alternatively contains six deuterium atoms, alternatively contains seven deuterium atoms, alternatively contains eight deuterium atoms, alternatively contains nine deuterium atoms, alternatively contains ten deuterium atoms, alternatively contains eleven deuterium atoms, alternatively contains twelve deuterium atoms, alternatively contains thirteen deuterium atoms, alternatively contains fourteen deuterium atoms, alternatively contains fifteen deuterium atoms, alternatively contains sixteen deuterium atoms, alternatively contains seventeen deuterium atoms, alternatively contains eighteen deuterium atoms, alternatively contains nineteen deuterium atoms, alternatively contains twenty deuterium atoms, alternatively contains twenty-one deuterium atoms, alternatively contains twenty-two deuterium atoms, alternatively contains twenty-three deuterium atoms.

In the embodiment, "$Y_1$, $Y_2$, $Y_3$, $Y_4$, $Y_5$, $Y_6$ and $Y_7$ are each independently selected from hydrogen, deuterium, halogen or trifluoromethyl" includes the technical solutions wherein, $Y_1$ is selected from hydrogen, deuterium, halogen or trifluoromethyl, $Y_2$ is selected from hydrogen, deuterium, halogen or trifluoromethyl, $Y_3$ is selected from hydrogen, deuterium, halogen or trifluoromethyl, and so on, until $Y_7$ is selected from hydrogen, deuterium, halogen or trifluoromethyl. More specifically, the technical solutions wherein, $Y_1$ is hydrogen, $Y_1$ is deuterium, $Y_1$ is halogen (F, Cl, Br or I), or $Y_1$ is trifluoromethyl, $Y_2$ is hydrogen, $Y_2$ is deuterium, $Y_2$ is halogen (F, Cl, Br or I), or $Y_2$ is trifluoromethyl, $Y_3$ is hydrogen, $Y_3$ is deuterium, $Y_3$ is halogen (F, Cl, Br or I), or $Y_3$ is trifluoromethyl, and so on, until $Y_7$ is hydrogen, $Y_7$ is deuterium, $Y_7$ is halogen (F, Cl, Br or I), or $Y_7$ is trifluoromethyl, are included.

In the embodiment, "$R_1$, $R_2$, $R_3$, $R_4$, $R_5$ and $R_6$ are each independently selected from hydrogen or deuterium" includes the technical solutions wherein, $R_1$ is selected from hydrogen or deuterium, $R_2$ is selected from hydrogen or deuterium, $R_3$ is selected from hydrogen or deuterium, and so on, until $R_6$ is selected from hydrogen or deuterium. More specifically, the technical solutions wherein, $R_1$ is hydrogen or $R_1$ is deuterium, $R_2$ is hydrogen or $R_2$ is deuterium, $R_3$

is hydrogen or $R_3$ is deuterium, and so on, until $R_6$ is hydrogen or $R_6$ is deuterium, are included.

In the embodiment, "$X_1$, $X_2$ and $X_3$ are each independently selected from $CH_3$, $CD_3$, $CHD_2$ or $CH_2D$" includes the technical solutions wherein, $X_1$ is selected from $CH_3$, $CD_3$, $CHD_2$ or $CH_2D$, $X_2$ is selected from $CH_3$, $CD_3$, $CHD_2$ or $CH_2D$ and $X_3$ is selected from $CH_3$, $CD_3$, $CHD_2$ or $CH_2D$. More specifically, the technical solutions wherein, $X_1$ is $CH_3$, $X_1$ is $CD_3$, $X_1$ is $CHD_2$ or $X_1$ is $CH_2D$, $X_2$ is $CH_3$, $X_2$ is $CD_3$, $X_2$ is $CHD_2$ or $X_2$ is $CH_2D$, $X_3$ is $CH_3$, $X_3$ is $CD_3$, $X_3$ is $CHD_2$ or $X_3$ is $CH_2D$, are included.

In one aspect, $Y_1$, $Y_2$, $Y_3$, $Y_4$, $Y_5$, $Y_6$ and $Y_7$ are hydrogen.

In another aspect, $R_1$ and $R_2$ are hydrogen.

In another aspect, $R_1$ and $R_2$ are deuterium.

In another aspect, $R_3$ and $R_4$ are hydrogen.

In another aspect, $R_3$ and $R_4$ are deuterium.

In another aspect, $R_5$ and $R_6$ are hydrogen.

In another aspect, $R_5$ and $R_6$ are deuterium.

In another aspect, $X_1$ is $CH_3$.

In another aspect, $X_1$ is $CD_3$.

In another aspect, $X_2$ is $CH_3$.

In another aspect, $X_2$ is $CD_3$.

In another aspect, $X_3$ is $CH_3$.

In another aspect, $X_3$ is $CD_3$.

In another embodiment, the present disclosure relates to a compound of formula (II):

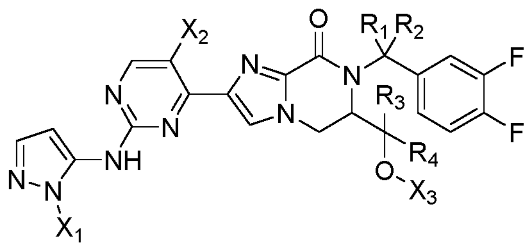

formula (II)

wherein,

$R_1$, $R_2$, $R_3$ and $R_4$ are each independently selected from hydrogen or deuterium;
$X_1$, $X_2$ and $X_3$ are each independently selected from $CH_3$, $CD_3$, $CHD_2$ or $CH_2D$;
provided that the above compound has at least one deuterium.

In the embodiment, alternatively, $R_3$ and $R_4$ are hydrogen, $R_1$ and $R_2$ are each independently selected from hydrogen or deuterium, $X_1$, $X_2$ and $X_3$ are each independently selected from $CH_3$, $CD_3$, $CHD_2$ or $CH_2D$; alternatively, $R_3$ and $R_4$ are hydrogen, $R_1$ and $R_2$ are each independently selected from hydrogen or deuterium, $X_1$, $X_2$ and $X_3$ are each independently selected from $CH_3$ or $CD_3$; alternatively, $R_3$ and $R_4$ are hydrogen, $R_1$ and $R_2$ are each independently selected from hydrogen or deuterium, $X_2$ and $CH_3$, $X_1$ and $X_3$ are each independently selected from $CH_3$ or $CD_3$; and alternatively, the compound described above contains at least one deuterium atom.

In the embodiment, alternatively, $X_2$ is $CH_3$, $R_1$, $R_2$, $R_3$ and $R_4$ are each independently selected from hydrogen or deuterium, $X_1$ and $X_3$ are each independently selected from $CH_3$, $CD_3$, $CHD_2$ or $CH_2D$; alternatively, $X_2$ is $CH_3$, $R_1$, $R_2$, $R_3$ and $R_4$ are each independently selected from hydrogen or deuterium, $X_1$ and $X_3$ are each independently selected from $CH_3$ or $CD_3$; alternatively, $X_2$ is $CH_3$, $R_1$ and $R_2$ are each independently selected from hydrogen or deuterium, $R_3$ and $R_4$ are hydrogen, $X_1$ and $X_3$ are each independently selected from $CH_3$ or $CD_3$; and alternatively, the compound described above contains at least one deuterium atom.

In the embodiment, alternatively, $X_1$ is $CH_3$, $R_1$, $R_2$, $R_3$ and $R_4$ are each independently selected from hydrogen or deuterium, $X_2$ and $X_3$ are each independently selected from $CH_3$, $CD_3$, $CHD_2$ or $CH_2D$; alternatively, $X_1$ is $CH_3$, $R_1$, $R_2$, $R_3$ and $R_4$ are each independently selected from hydrogen or deuterium, $X_2$ and $X_3$ are each independently selected from $CH_3$ or $CD_3$; alternatively, $X_1$ is $CH_3$, $R_1$ and $R_2$ are each independently selected from hydrogen or deuterium, $R_3$ and $R_4$ are hydrogen, $X_2$ and $X_3$ are each independently selected from $CH_3$ or $CD_3$; alternatively, $X_1$ is $CH_3$, $R_1$ and $R_2$ are each independently selected from hydrogen or deuterium, $R_3$ and $R_4$ are hydrogen, $X_2$ is $CH_3$, $X_3$ is selected from $CH_3$ or $CD_3$; and alternatively, the compound described above contains at least one deuterium atom.

In the embodiment, alternatively, $X_1$ is $CD_3$, $R_1$, $R_2$, $R_3$ and $R_4$ are each independently selected from hydrogen or deuterium, $X_2$ and $X_3$ are each independently selected from $CH_3$, $CD_3$, $CHD_2$ or $CH_2D$; alternatively, $X_1$ is $CD_3$, $R_1$, $R_2$, $R_3$ and $R_4$ are each independently selected from hydrogen or deuterium, $X_2$ and $X_3$ are each independently selected from $CH_3$ or $CD_3$; alternatively, $X_1$ is $CD_3$, $R_1$ and $R_2$ are each independently selected from hydrogen or deuterium, $R_3$ and $R_4$ are hydrogen, $X_2$ and $X_3$ are each independently selected from $CH_3$ or $CD_3$; and alternatively, $X_1$ is $CD_3$, $R_1$ and $R_2$ are each independently selected from hydrogen or deuterium, $R_3$ and $R_4$ are hydrogen, $X_2$ is $CH_3$, $X_3$ is selected from $CH_3$ or $CD_3$. In the embodiment, alternatively, $X_3$ is $CH_3$, $R_1$, $R_2$, $R_3$ and $R_4$ are each independently selected from hydrogen or deuterium, $X_1$ and $X_2$ are each independently selected from $CH_3$, $CD_3$, $CHD_2$ or $CH_2D$; alternatively, $X_3$ is $CH_3$, $R_1$, $R_2$, $R_3$ and $R_4$ are each independently selected from hydrogen or deuterium, $X_1$ and $X_2$ are each independently selected from $CH_3$ or $CD_3$; alternatively, $X_3$ is $CH_3$, $R_1$ and $R_2$ are each independently selected from hydrogen or deuterium, $R_3$ and $R_4$ are hydrogen, $X_1$ and $X_2$ are each independently selected from $CH_3$ or $CD_3$; alternatively, $X_3$ is $CH_3$, $R_1$ and $R_2$ are each independently selected from hydrogen or deuterium, $R_3$ and $R_4$ are hydrogen, $X_1$ is selected from $CH_3$ or $CD_3$, $X_2$ is $CH_3$; and alternatively, the compound described above contains at least one deuterium atom.

In the embodiment, alternatively, $X_3$ is $CD_3$, $R_1$, $R_2$, $R_3$ and $R_4$ are each independently selected from hydrogen or deuterium, $X_1$ and $X_2$ are each independently selected from $CH_3$, $CD_3$, $CHD_2$ or $CH_2D$; alternatively, $X_3$ is $CD_3$, $R_1$, $R_2$, $R_3$ and $R_4$ are each independently selected from hydrogen or deuterium, $X_1$ and $X_2$ are each independently selected from $CH_3$ or $CD_3$; alternatively, $X_3$ is $CD_3$, $R_1$ and $R_2$ are each independently selected from hydrogen or deuterium, $R_3$ and $R_4$ are hydrogen, $X_1$ and $X_2$ are each independently selected from $CH_3$ or $CD_3$; and alternatively, $X_3$ is $CD_3$, $R_1$ and $R_2$ are each independently selected from hydrogen or deuterium, $R_3$ and $R_4$ are hydrogen, $X_1$ is selected from $CH_3$ or $CD_3$, $X_2$ is $CH_3$. In the embodiment, alternatively, $R_1$ and $R_2$ are hydrogen, $R_3$ and $R_4$ are each independently selected from hydrogen or deuterium, $X_1$, $X_2$ and $X_3$ are each independently selected from $CH_3$, $CD_3$, $CHD_2$ or $CH_2D$; alternatively, $R_1$ and $R_2$ are hydrogen, $R_3$ and $R_4$ are each independently selected from hydrogen or deuterium, $X_1$, $X_2$ and $X_3$ are each independently selected from $CH_3$ or $CD_3$; alternatively, $R_1$ and $R_2$ are hydrogen, $R_3$ and $R_4$ are hydrogen, $X_1$, $X_2$ and $X_3$ are each independently selected from $CH_3$ or $CD_3$; alternatively, $R_1$ and $R_2$ are hydrogen, $R_3$ and $R_4$ are hydrogen, $X_2$ is $CH_3$, $X_1$ and $X_3$ are each independently selected from $CH_3$ or $CD_3$; and alternatively, the compound described above contains at least one deuterium atom.

In the embodiment, alternatively, $R_1$ and $R_2$ are deuterium, $R_3$ and $R_4$ are each independently selected from hydrogen or deuterium, $X_1$, $X_2$ and $X_3$ are each independently selected from $CH_3$, $CD_3$, $CHD_2$ or $CH_2D$; alternatively, $R_1$ and $R_2$ are deuterium, $R_3$ and $R_4$ are each independently selected from hydrogen or deuterium, $X_1$, $X_2$ and $X_3$ are each independently selected from $CH_3$ or $CD_3$; alternatively, $R_1$ and $R_2$ are deuterium, $R_3$ and $R_4$ are hydrogen, $X_1$, $X_2$ and $X_3$ are each independently selected from $CH_3$ or $CD_3$; and alternatively, $R_1$ and $R_2$ are deuterium, $R_3$ and $R_4$ are hydrogen, $X_2$ is $CH_3$, $X_1$ and $X_3$ are each independently selected from $CH_3$ or $CD_3$.

In another embodiment, the present disclosure relates to a compound of formula (III):

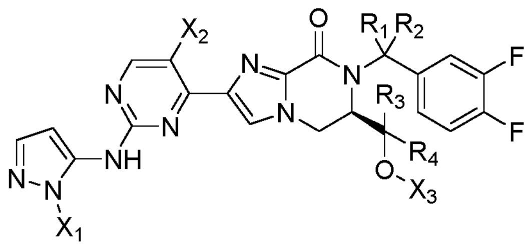

formula (III)

wherein,

$R_1$, $R_2$, $R_3$ and $R_4$ are each independently selected from hydrogen or deuterium;
$X_1$, $X_2$ and $X_3$ are each independently selected from $CH_3$, $CD_3$, $CHD_2$ or $CH_2D$;
provided that the above compound has at least one deuterium.

In the embodiment, alternatively, $R_3$ and $R_4$ are hydrogen, $R_1$ and $R_2$ are each independently selected from hydrogen or deuterium, $X_1$, $X_2$ and $X_3$ are each independently selected from $CH_3$, $CD_3$, $CHD_2$ or $CH_2D$; alternatively, $R_3$ and $R_4$ are hydrogen, $R_1$ and $R_2$ are each independently selected from hydrogen or deuterium, $X_1$, $X_2$ and $X_3$ are each independently selected from $CH_3$ or $CD_3$; alternatively, $R_3$ and $R_4$ are hydrogen, $R_1$ and $R_2$ are each independently selected from hydrogen or deuterium, $X_2$ is $CH_3$, $X_1$ and $X_3$ are each independently selected from $CH_3$ or $CD_3$; and alternatively, the compound described above contains at least one deuterium atom.

In the embodiment, alternatively, $X_2$ is $CH_3$, $R_1$, $R_2$, $R_3$ and $R_4$ are each independently selected from hydrogen or deuterium, $X_1$ and $X_3$ are each independently selected from $CH_3$, $CD_3$, $CHD_2$ or $CH_2D$; alternatively, $X_2$ is $CH_3$, $R_1$, $R_2$, $R_3$ and $R_4$ are each independently selected from hydrogen or deuterium, $X_1$ and $X_3$ are each independently selected from $CH_3$ or $CD_3$; alternatively, $X_2$ is $CH_3$, $R_1$ and $R_2$ are each independently selected from hydrogen or deuterium, $R_3$ and $R_4$ are hydrogen, $X_1$ and $X_3$ are each independently selected from $CH_3$ or $CD_3$; and alternatively, the compound described above contains at least one deuterium atom.

In the embodiment, alternatively, $X_1$ is $CH_3$, $R_1$, $R_2$, $R_3$ and $R_4$ are each independently selected from hydrogen or deuterium, $X_2$ and $X_3$ are each independently selected from $CH_3$, $CD_3$, $CHD_2$ or $CH_2D$; alternatively, $X_1$ is $CH_3$, $R_1$, $R_2$, $R_3$ and $R_4$ are each independently selected from hydrogen or deuterium, $X_2$ and $X_3$ are each independently selected from $CH_3$ or $CD_3$; alternatively, $X_1$ is $CH_3$, $R_1$ and $R_2$ are each independently selected from hydrogen or deuterium, $R_3$ and $R_4$ are hydrogen, $X_2$ and $X_3$ are each independently selected from $CH_3$ or $CD_3$; alternatively, $X_1$ is $CH_3$, $R_1$ and $R_2$ are each independently selected from hydrogen or deuterium, $R_3$ and $R_4$ are hydrogen, $X_2$ is $CH_3$, $X_3$ is selected from $CH_3$ or $CD_3$; and alternatively, the compound described above contains at least one deuterium atom.

In the embodiment, alternatively, $X_1$ is $CD_3$, $R_1$, $R_2$, $R_3$ and $R_4$ are each independently selected from hydrogen or deuterium, $X_2$ and $X_3$ are each independently selected from $CH_3$, $CD_3$, $CHD_2$ or $CH_2D$; alternatively, $X_1$ is $CD_3$, $R_1$, $R_2$, $R_3$ and $R_4$ are each independently selected from hydrogen or deuterium, $X_2$ and $X_3$ are each independently selected from $CH_3$ or $CD_3$; alternatively, $X_1$ is $CD_3$, $R_1$ and $R_2$ are each independently selected from hydrogen or deuterium, $R_3$ and $R_4$ are hydrogen, $X_2$ and $X_3$ are each independently selected from $CH_3$ or $CD_3$; and alternatively, $X_1$ is $CD_3$, $R_1$ and $R_2$ are each independently selected from hydrogen or deuterium, $R_3$ and $R_4$ are hydrogen, $X_2$ is $CH_3$, $X_3$ is selected from $CH_3$ or $CD_3$. In the embodiment, alternatively, $X_3$ is $CH_3$, $R_1$, $R_2$, $R_3$ and $R_4$ are each independently selected from hydrogen or deuterium, $X_1$ and $X_2$ are each independently selected from $CH_3$, $CD_3$, $CHD_2$ or $CH_2D$; alternatively, $X_3$ is $CH_3$, $R_1$, $R_2$, $R_3$ and $R_4$ are each independently selected from hydrogen or deuterium, $X_1$ and $X_2$ are each independently selected from $CH_3$ or $CD_3$; alternatively, $X_3$ is $CH_3$, $R_1$ and $R_2$ are each independently selected from hydrogen or deuterium, $R_3$ and $R_4$ are hydrogen, $X_1$ and $X_2$ are each independently selected from $CH_3$ or $CD_3$; alternatively, $X_3$ is $CH_3$, Ri and $R_2$ are each independently selected from hydrogen or deuterium, $R_3$ and $R_4$ are hydrogen, $X_1$ is selected from $CH_3$ or $CD_3$, $X_2$ is $CH_3$; and alternatively, the compound described above contains at least one deuterium atom.

In the embodiment, alternatively, $X_3$ is $CD_3$, $R_1$, $R_2$, $R_3$ and $R_4$ are each independently selected from hydrogen or deuterium, $X_1$ and $X_2$ are each independently selected from $CH_3$, $CD_3$, $CHD_2$ or $CH_2D$; alternatively, $X_3$ is $CD_3$, $R_1$, $R_2$, $R_3$ and $R_4$ are each independently selected from hydrogen or deuterium, $X_1$ and $X_2$ are each independently selected from $CH_3$ or $CD_3$; alternatively, $X_3$ is $CD_3$, $R_1$ and $R_2$ are each independently selected from hydrogen or deuterium, $R_3$ and $R_4$ are hydrogen, $X_1$ and $X_2$ are each independently selected from $CH_3$ or $CD_3$; and alternatively, $X_3$ is $CD_3$, $R_1$ and $R_2$ are each independently selected from hydrogen or deuterium, $R_3$ and $R_4$ are hydrogen, $X_1$ is selected from $CH_3$ or $CD_3$, $X_2$ is $CH_3$. In the embodiment, alternatively, $R_1$ and $R_2$ are hydrogen, $R_3$ and $R_4$ are each independently selected from hydrogen or deuterium, $X_1$, $X_2$ and $X_3$ are each independently selected from $CH_3$, $CD_3$, $CHD_2$ or $CH_2D$; alternatively, $R_1$ and $R_2$ are hydrogen, $R_3$ and $R_4$ are each independently selected from hydrogen or deuterium, $X_1$, $X_2$ and $X_3$ are each independently selected from $CH_3$ or $CD_3$; alternatively, $R_1$ and $R_2$ are hydrogen, $R_3$ and $R_4$ are hydrogen, $X_1$, $X_2$ and $X_3$ are each independently selected from $CH_3$ or $CD_3$; alternatively, $R_1$ and $R_2$ are hydrogen, $R_3$ and $R_4$ are hydrogen, $X_2$ is $CH_3$, $X_1$ and $X_3$ are each independently selected from $CH_3$ or $CD_3$; and alternatively, the compound described above contains at least one deuterium atom.

In the embodiment, alternatively, $R_1$ and $R_2$ are deuterium, $R_3$ and $R_4$ are each independently selected from hydrogen or deuterium, $X_1$, $X_2$ and $X_3$ are each independently selected from $CH_3$, $CD_3$, $CHD_2$ or $CH_2D$; alternatively, $R_1$ and $R_2$ are deuterium, $R_3$ and $R_4$ are each independently selected from hydrogen or deuterium, $X_1$, $X_2$ and $X_3$ are each independently selected from $CH_3$ or $CD_3$; alternatively, $R_1$ and $R_2$ are deuterium, $R_3$ and $R_4$ are hydrogen, $X_1$, $X_2$ and $X_3$ are each independently selected from $CH_3$ or $CD_3$; and alternatively, $R_1$ and $R_2$ are deuterium, $R_3$ and $R_4$ are hydrogen, $X_2$ is $CH_3$, $X_1$ and $X_3$ are each independently selected from $CH_3$ or $CD_3$.

In another embodiment, the present disclosure relates to a compound of formula (IV):

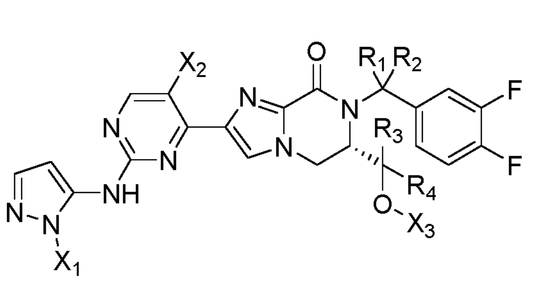

formula (IV)

wherein,

$R_1$, $R_2$, $R_3$ and $R_4$ are each independently selected from hydrogen or deuterium;
$X_1$, $X_2$ and $X_3$ are each independently selected from $CH_3$, $CD_3$, $CHD_2$ or $CH_2D$;
provided that the above compound has at least one deuterium.

In the embodiment, alternatively, $R_3$ and $R_4$ are hydrogen, $R_1$ and $R_2$ are each independently selected from hydrogen or deuterium, $X_1$, $X_2$ and $X_3$ are each independently selected from $CH_3$, $CD_3$, $CHD_2$ or $CH_2D$; alternatively, $R_3$ and $R_4$ are hydrogen, $R_1$ and $R_2$ are each independently selected from hydrogen or deuterium, $X_1$, $X_2$ and $X_3$ are each independently selected from $CH_3$ or $CD_3$; alternatively, $R_3$ and $R_4$ are hydrogen, $R_1$ and $R_2$ are each independently selected from hydrogen or deuterium, $X_2$ is $CH_3$, $X_1$ and $X_3$ are each independently selected from $CH_3$ or $CD_3$; and alternatively, the compound described above contains at least one deuterium atom.

In the embodiment, alternatively, $X_2$ is $CH_3$, $R_1$, $R_2$, $R_3$ and $R_4$ are each independently selected from hydrogen or deuterium, $X_1$ and $X_3$ are each independently selected from $CH_3$, $CD_3$, $CHD_2$ or $CH_2D$; alternatively, $X_2$ is $CH_3$, $R_1$, $R_2$, $R_3$ and $R_4$ are each independently selected from hydrogen or deuterium, $X_1$ and $X_3$ are each independently selected from $CH_3$ or $CD_3$; alternatively, $X_2$ is $CH_3$, $R_1$ and $R_2$ are each independently selected from hydrogen or deuterium, $R_3$ and $R_4$ are hydrogen, $X_1$ and $X_3$ are each independently selected from $CH_3$ or $CD_3$; and alternatively, the compound described above contains at least one deuterium atom.

In the embodiment, alternatively, $X_1$ is $CH_3$, $R_1$, $R_2$, $R_3$ and $R_4$ are each independently selected from hydrogen or deuterium, $X_2$ and $X_3$ are each independently selected from $CH_3$, $CD_3$, $CHD_2$ or $CH_2D$; alternatively, $X_1$ is $CH_3$, $R_1$, $R_2$, $R_3$ and $R_4$ are each independently selected from hydrogen or deuterium, $X_2$ and $X_3$ are each independently selected from $CH_3$ or $CD_3$; alternatively, $X_1$ is $CH_3$, $R_1$ and $R_2$ are each independently selected from hydrogen or deuterium, $R_3$ and $R_4$ are hydrogen, $X_2$ and $X_3$ are each independently selected from $CH_3$ or $CD_3$; alternatively, $X_1$ is $CH_3$, $R_1$ and $R_2$ are each independently selected from hydrogen or deuterium, $R_3$ and $R_4$ are hydrogen, $X_2$ is $CH_3$, $X_3$ is selected from $CH_3$ or $CD_3$; and alternatively, the compound described above contains at least one deuterium atom.

In the embodiment, alternatively, $X_1$ is $CD_3$, $R_1$, $R_2$, $R_3$ and $R_4$ are each independently selected from hydrogen or deuterium, $X_2$ and $X_3$ are each independently selected from $CH_3$, $CD_3$, $CHD_2$ or $CH_2D$; alternatively, $X_1$ is $CD_3$, $R_1$, $R_2$, $R_3$ and $R_4$ are each independently selected from hydrogen or deuterium, $X_2$ and $X_3$ are each independently selected from $CH_3$ or $CD_3$; alternatively, $X_1$ is $CD_3$, $R_1$ and $R_2$ are each independently selected from hydrogen or deuterium, $R_3$ and $R_4$ are hydrogen, $X_2$ and $X_3$ are each independently selected from $CH_3$ or $CD_3$; and alternatively, $X_1$ is $CD_3$, $R_1$ and $R_2$ are each independently selected from hydrogen or deuterium, $R_3$ and $R_4$ are hydrogen, $X_2$ is $CH_3$, $X_3$ is selected from $CH_3$ or $CD_3$. In the embodiment, alternatively, $X_3$ is $CH_3$, $R_1$, $R_2$, $R_3$ and $R_4$ are each independently selected from hydrogen or deuterium, $X_1$ and $X_2$ are each independently selected from $CH_3$, $CD_3$, $CHD_2$ or $CH_2D$; alternatively, $X_3$ is $CH_3$, $R_1$, $R_2$, $R_3$ and $R_4$ are each independently selected from hydrogen or deuterium, $X_1$ and $X_2$ are each independently selected from $CH_3$ or $CD_3$; alternatively, $X_3$ is $CH_3$, $R_1$ and $R_2$ are each independently selected from hydrogen or deuterium, $R_3$ and $R_4$ are hydrogen, $X_1$ and $X_2$ are each independently selected from $CH_3$ or $CD_3$; alternatively, $X_3$ is $CH_3$, $R_1$ and $R_2$ are each independently selected from hydrogen or deuterium, $R_3$ and $R_4$ are hydrogen, $X_1$ is selected from $CH_3$ or $CD_3$, $X_2$ is $CH_3$; and alternatively, the compound described above contains at least one deuterium atom.

In the embodiment, alternatively, $X_3$ is $CD_3$, $R_1$, $R_2$, $R_3$ and $R_4$ are each independently selected from hydrogen or deuterium, $X_1$ and $X_2$ are each independently selected from $CH_3$, $CD_3$, $CHD_2$ or $CH_2D$; alternatively, $X_3$ is $CD_3$, $R_1$, $R_2$, $R_3$ and $R_4$ are each independently selected from hydrogen or deuterium, $X_1$ and $X_2$ are each independently selected from $CH_3$ or $CD_3$; alternatively, $X_3$ is $CD_3$, $R_1$ and $R_2$ are each independently selected from hydrogen or deuterium, $R_3$ and $R_4$ are hydrogen, $X_1$ and $X_2$ are each independently selected from $CH_3$ or $CD_3$; and alternatively, $X_3$ is $CD_3$, $R_1$ and $R_2$ are each independently selected from hydrogen or deuterium, $R_3$ and $R_4$ are hydrogen, $X_1$ is selected from $CH_3$ or $CD_3$, $X_2$ is $CH_3$. In the embodiment, alternatively, $R_1$ and $R_2$ are hydrogen, $R_3$ and $R_4$ are each independently selected from hydrogen or deuterium, $X_1$, $X_2$ and $X_3$ are each independently selected from $CH_3$, $CD_3$, $CHD_2$ or $CH_2D$; alternatively, $R_1$ and $R_2$ are hydrogen, $R_3$ and $R_4$ are each independently selected from hydrogen or deuterium, $X_1$, $X_2$ and $X_3$ are each independently selected from $CH_3$ or $CD_3$; alternatively, $R_1$ and $R_2$ are hydrogen, $R_3$ and $R_4$ are hydrogen, $X_1$, $X_2$ and $X_3$ are each independently selected from $CH_3$ or $CD_3$; alternatively, $R_1$ and $R_2$ are hydrogen, $R_3$ and $R_4$ are hydrogen, $X_2$ is $CH_3$, $X_1$ and $X_3$ are each independently selected from $CH_3$ or $CD_3$; and alternatively, the compound described above contains at least one deuterium atom.

In the embodiment, alternatively, $R_1$ and $R_2$ are deuterium, $R_3$ and $R_4$ are each independently selected from hydrogen or deuterium, $X_1$, $X_2$ and $X_3$ are each independently selected from $CH_3$, $CD_3$, $CHD_2$ or $CH_2D$; alternatively, $R_1$ and $R_2$ are deuterium, $R_3$ and $R_4$ are each independently selected from hydrogen or deuterium, $X_1$, $X_2$ and $X_3$ are each independently selected from $CH_3$ or $CD_3$; alternatively, $R_1$ and $R_2$ are deuterium, $R_3$ and $R_4$ are hydrogen, $X_1$, $X_2$ and $X_3$ are each independently selected from $CH_3$ or $CD_3$; and alternatively, $R_1$ and $R_2$ are deuterium, $R_3$ and $R_4$ are hydrogen, $X_2$ is $CH_3$, $X_1$ and $X_3$ are each independently selected from $CH_3$ or $CD_3$.

In an alternative embodiment, the compound of the present disclosure is selected from the following compounds:

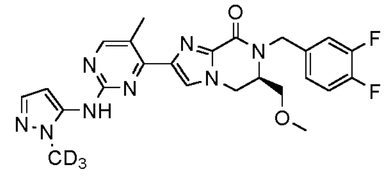,
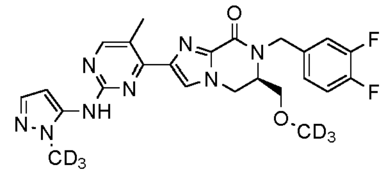,
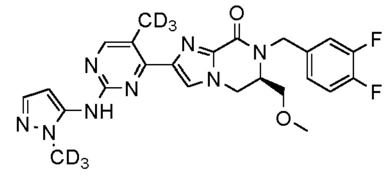,

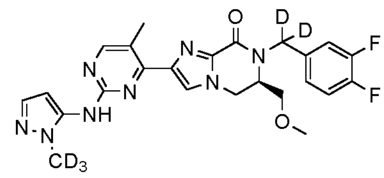,
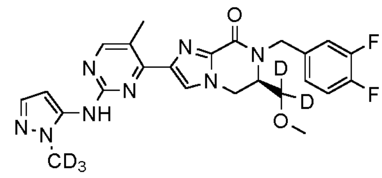,
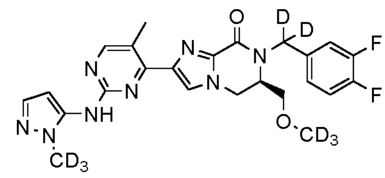,

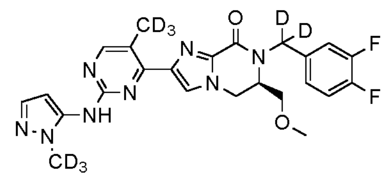,
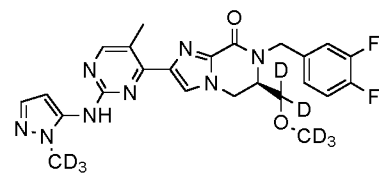,
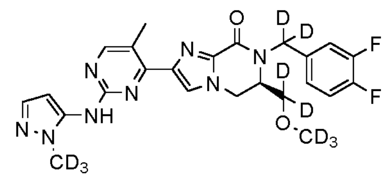,

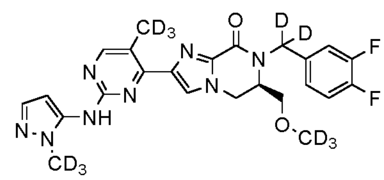,
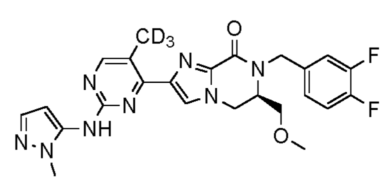,
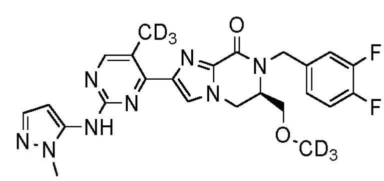,

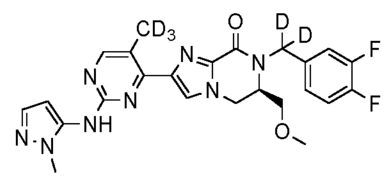,
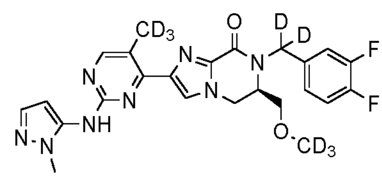,
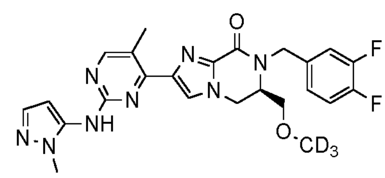,

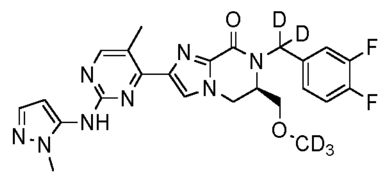,
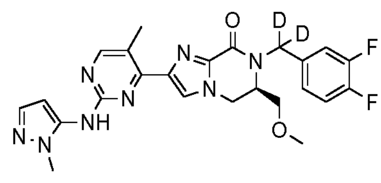,
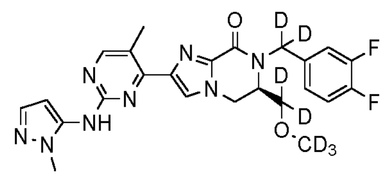,

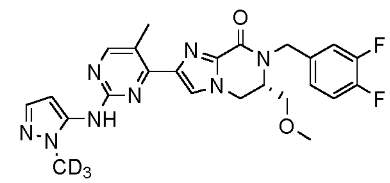,
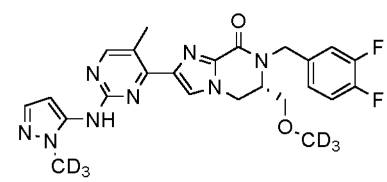,
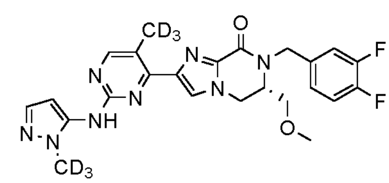,

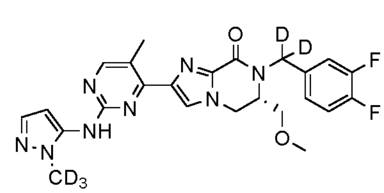,
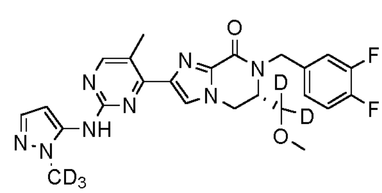,
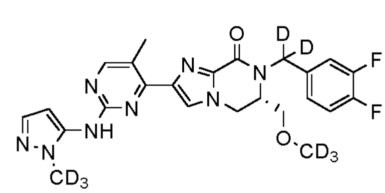,

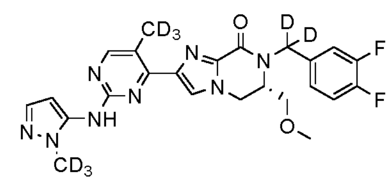,
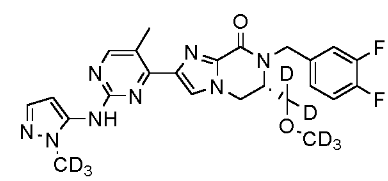,
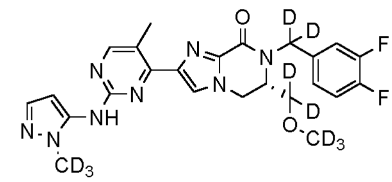,

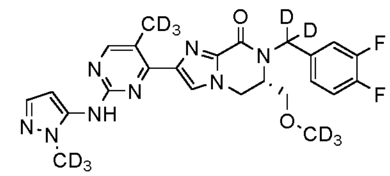,
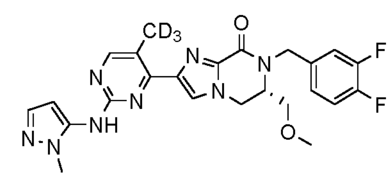,
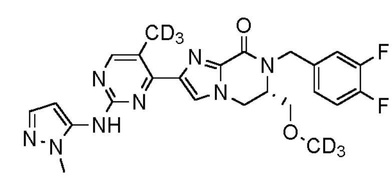,

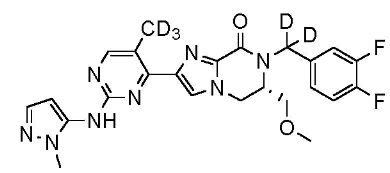,
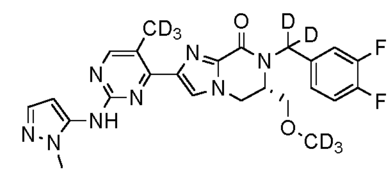,
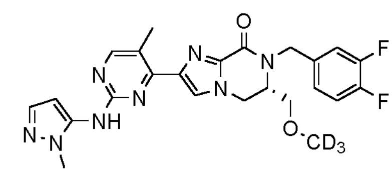,

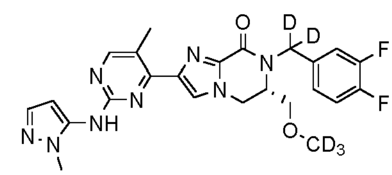,
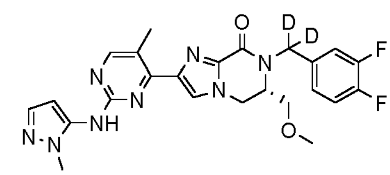,
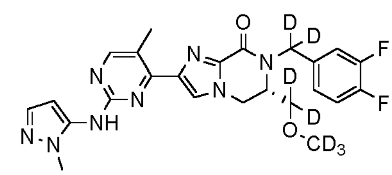,

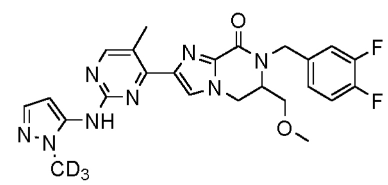,
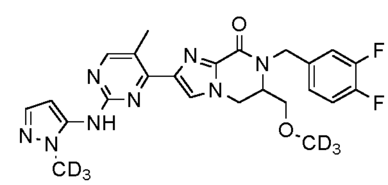,
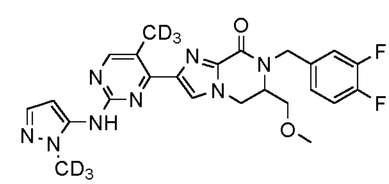,

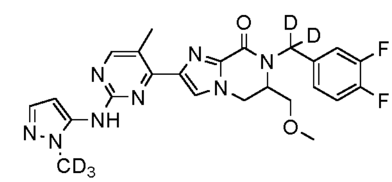,
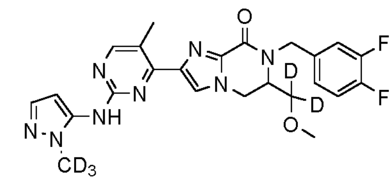,
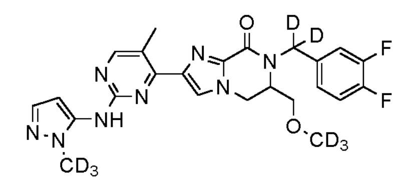,

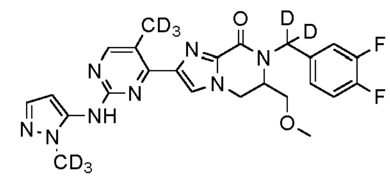,
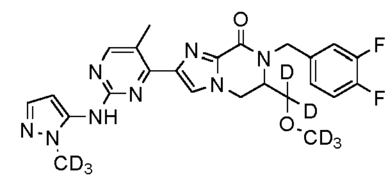,
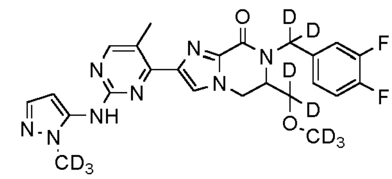,

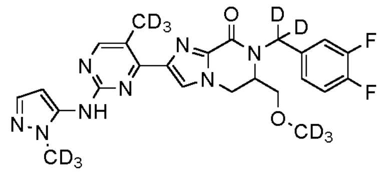, 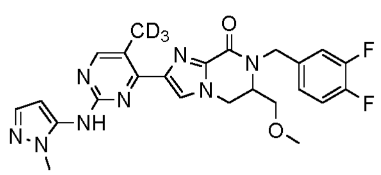, 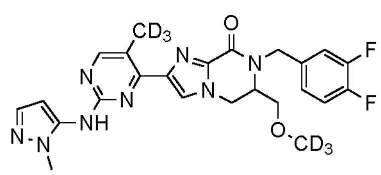,

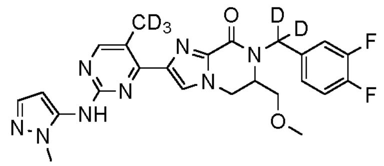, 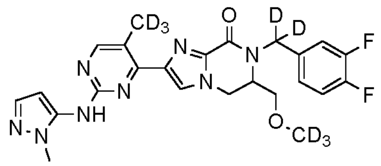, 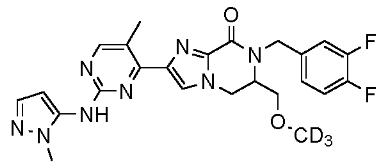,

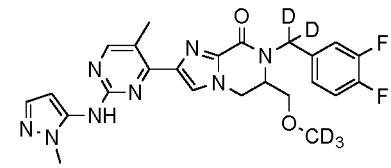, 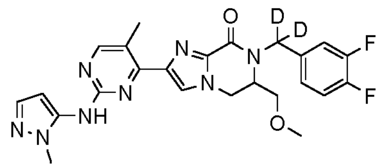, 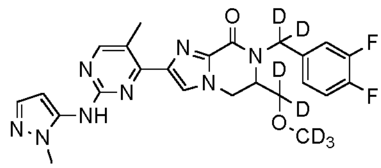.

In an alternative embodiment, the compound of the present disclosure is selected from the following compounds:

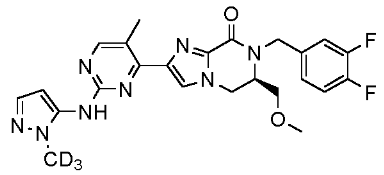, 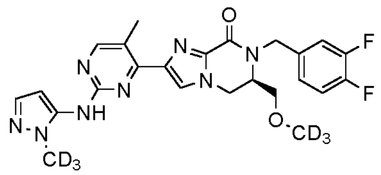, 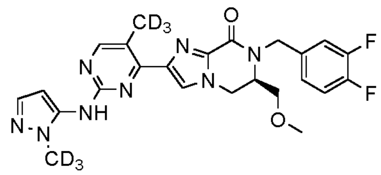,

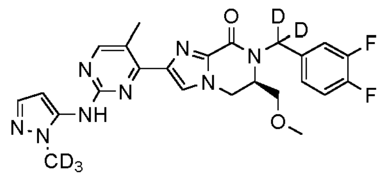, 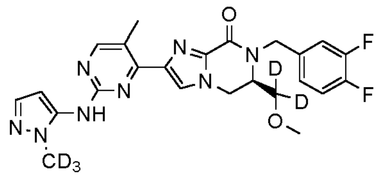, 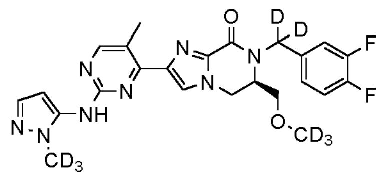,

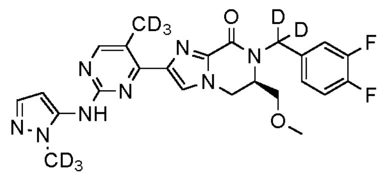, 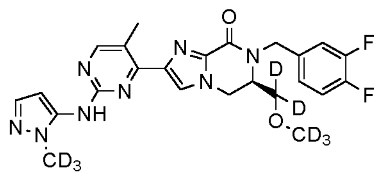, 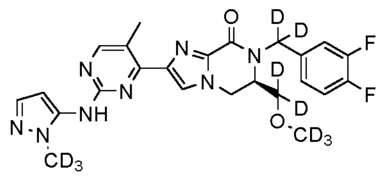,

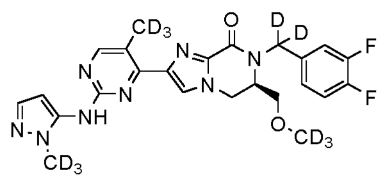, 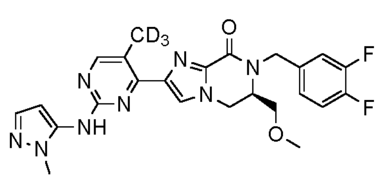, 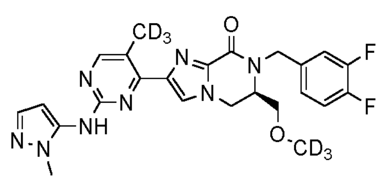,

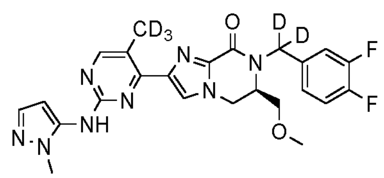, 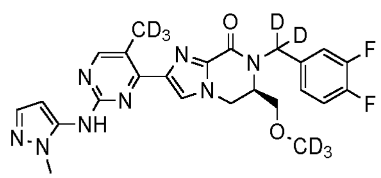, 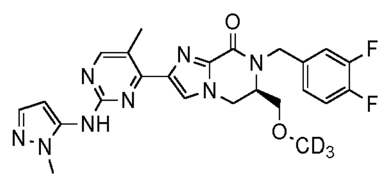,

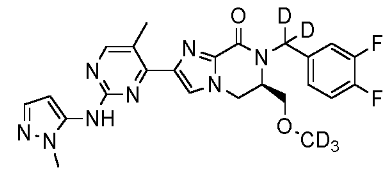 , 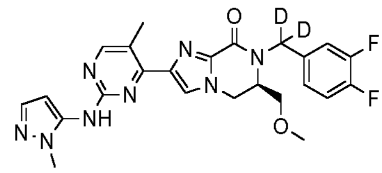 , 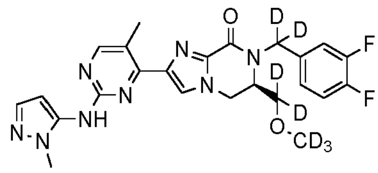 .

In an alternative embodiment, the compound of the present disclosure is selected from the following compounds:

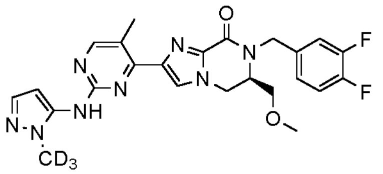 , 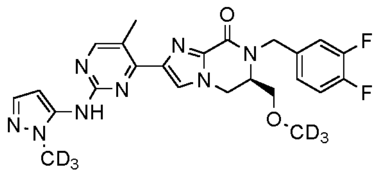 , 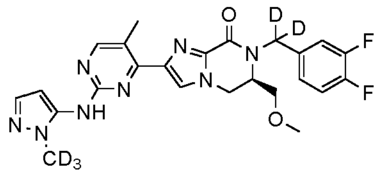 ,

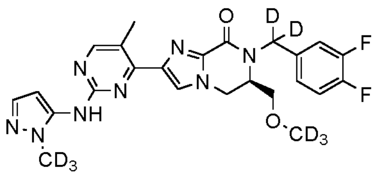 , 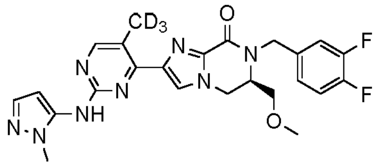 , 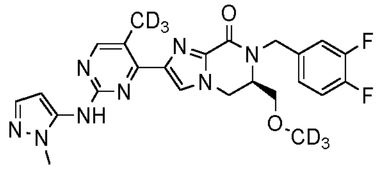 ,

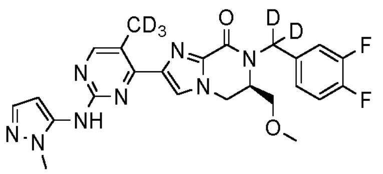 , 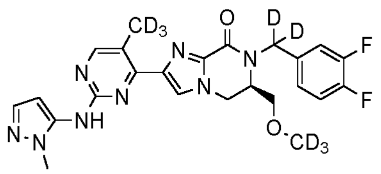 , 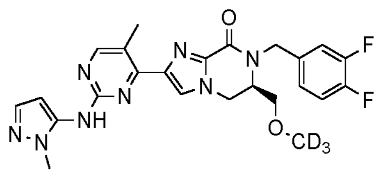 ,

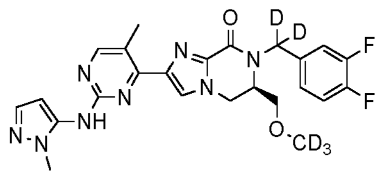 , 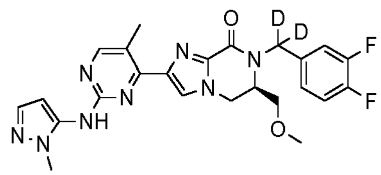 .

Some compounds of Formulae (I) to (IV) have chiral centre(s) and it will be recognised that such compound of Formulae (I) to (IV) may be prepared, isolated and/or supplied with or without the presence, in addition, of one or more of the other 2 possible enantiomeric isomers of the compound of Formulae (I) to (IV) in any relative proportions. The preparation of enantiomeric enriched/enantiomeric pure compounds may be carried out by standard techniques of organic chemistry that are well known in the art, for example by synthesis from enantiomeric enriched or enantiomeric pure starting materials, use of an appropriate enantiomeric enriched or enantiomeric pure catalyst during synthesis, and/or by resolution of a racemic or partially enriched mixture of stereoisomers, for example via chiral chromatography.

For use in a pharmaceutical context it may be preferable to provide the compound of Formulae (I) to (IV) or pharmaceutically acceptable salt thereof without large amounts of the other stereoisomeric forms exist.

Accordingly, in one embodiment there is provided a composition comprising a compound of Formulae (I) to (IV) or a pharmaceutically acceptable salt thereof, optionally together with one or more of the other stereoisomeric forms of the compound of Formulae (I) to (IV) or pharmaceutically acceptable salt thereof, wherein the compound of Formulae (I) to (IV) or pharmaceutically acceptable salt thereof is present in the composition with an enantiomeric excess (ee%) of ≥ 90%.

In a further embodiment the %ee in the above-mentioned composition is ≥ 95%.

In a further embodiment the %ee in the above-mentioned composition is ≥ 98%.

In a further embodiment the %ee in the above-mentioned composition is ≥ 99%.

Those skilled in the art will appreciate that organic compounds can form complexes with solvents that react in or precipitate or crystallize from the solvent. These complexes are referred to as "solvates." When the solvent is water, the complex is referred to as a "hydrate." The present disclosure encompasses all solvates of the compounds disclosed herein.

The term "solvate" refers to forms of a compound or a salt thereof, which are associated with a solvent, usually by a solvolysis reaction. This physical association may include hydrogen bonding. Conventional solvents include water, methanol, ethanol, acetic acid, DMSO, THF, diethyl ether, etc. The compounds described herein can be prepared, for example, in crystalline form, and can be solvated. Suitable solvates include pharmaceutically acceptable solvates and further include both stoichiometric solvates and non-stoichiometric solvates. In some cases, the solvates will be capable of isolation, for example, when one or more solvent molecules are incorporated into the crystal lattice of a crystalline solid. "Solvate" includes both solution-phase and isolatable solvates. Representative solvates include hydrates, ethanolates and methanolates.

The term "hydrate" refers to a compound that is associated with water. Generally, the number of water molecules contained in a hydrate of a compound is in a definite ratio to the number of the compound molecules in the hydrate. Therefore, hydrates of a compound can be represented, for example, by a general formula $R \cdot x\ H_2O$, wherein R is the compound, and x is a number greater than 0. Given compounds can form more than one type of hydrates, including, for example, monohydrates (x is 1), lower hydrates (x is a number greater than 0 and smaller than 1, for example, hemihydrates ($R \cdot 0.5\ H_2O$)) and polyhydrates (x is a number greater than 1, for example, dihydrates ($R \cdot 2\ H_2O$) and hexahydrates ($R \cdot 6\ H_2O$)).

Compounds of the present disclosure may be in an amorphous or a crystalline form (polymorph). Furthermore, the compounds of the present disclosure may exist in one or more crystalline forms. Therefore, the present disclosure includes all amorphous or crystalline forms of the compounds of the present disclosure within its scope. The term "polymorph" refers to a crystalline form of a compound (or a salt, hydrate or solvate thereof) in a particular crystal packing arrangement. All polymorphs have the same elemental composition. Different crystalline forms generally have different X-ray diffraction patterns, infrared spectra, melting points, density, hardness, crystal shapes, optical and electrical properties, stability, and solubility. Recrystallization solvents, rate of crystallization, storage temperatures, and other factors may cause one crystalline form to dominate. Various polymorphs of a compound can be prepared by crystallization under different conditions.

Also disclosed herein are isotopically labeled compounds, which are equivalent to those described above, but one or more atoms are replaced by atoms having an atom mass or mass number that are different from that of atoms that are common in nature. Examples of isotopes that can be listed in compounds disclosed herein include hydrogen, carbon, nitrogen, oxygen, phosphorus, sulfur, fluorine and chlorine isotopes, such as $^{2}H$, $^{3}H$, $^{13}C$, $^{11}C$, $^{14}C$, $^{15}N$, $^{18}O$, $^{17}O$, $^{31}P$, $^{32}P$, $^{35}S$, $^{18}F$ and $^{36}Cl$, respectively. A compound disclosed herein containing the above isotope and/or other isotope of other atoms, or a pharmaceutically acceptable salt of said compound are all within the scope disclosed herein. Certain isotopically labeled compounds disclosed herein, such as those incorporating radioisotopes (e.g., $^{3}H$ and $^{14}C$), can be used in the tissue distribution experiments of drugs and/or substrates. Tritium, i.e., $^{3}H$, and carbon-14, i.e., $^{14}C$, are particularly preferred, because they are easier to be prepared and detected. In addition, substitution with heavier isotopes such as deuterium, i.e., $^{2}H$, may provide therapeutic benefits due to the higher metabolic stability, for example, increased half-life in vivo or reduced dosage, and thus priority may be given in some cases. Isotopically-labeled compounds of formula (I) disclosed herein can be prepared using the following schemes and/or the procedures disclosed in the examples and preparation examples by replacing the non-isotopic reagents with readily available isotopically labeled reagents.

Method of preparing the compound disclosed herein

The compounds disclosed herein, including salts thereof, can be prepared using known organic synthetic techniques and can be synthesized according to any of various possible synthetic routes, such as those in the scheme below. The reaction for preparing compounds disclosed herein can be carried out in a suitable solvent, which can be easily selected by those skilled in the art of organic synthesis. The suitable solvent can be substantially unreactive with starting materials (reactants), intermediates or products at the temperature at which the reaction is carried out (for example, at temperatures ranging from the freezing temperature to boiling temperature of the solvent). A given reaction can be carried out in one solvent or a mixture of two or more solvents. Depending on the particular reaction step, the skilled person can select the solvent for it.

The preparation of the compounds disclosed herein may involve protection and deprotection of different chemical groups. One skilled in the art can readily determine the need for protection and deprotection and the choice of appropriate protective groups. The chemical properties of protective groups can be found, for example, in Wuts and Greene, Protective Groups in Organic Synthesis, 4th Ed., John Wiley & Sons: New Jersey, (2006).

The compound of the present disclosure can be prepared as an individual stereoisomer thereof by reacting a racemic mixture of the compound with an optically active resolving agent to form a pair of diastereomeric compounds, separating the diastereomers, and recovering the optically pure enantiomer. The resolution of enantiomers can be carried out using a diastereomeric derivative of the compound disclosed herein, or alternatively, the dissociable complexes (for example, crystalline diastereomeric salts). Diastereomers have significantly different physical properties (for example,

melting point, boiling point, solubility, reactivity, etc.), and can be easily separated by taking advantage of these dissimilarities. Diastereomers can be separated by chromatography, or alternatively by separation/resolution techniques based on differences in solubility. The optically pure enantiomer is then recovered, along with the resolving reagent, by any practical means that would not result in racemization. A more detailed description of the techniques applicable to the resolution of a racemic mixture to obtain stereoisomers of a compound can be found in Jean Jacques, Andre Collet, Samue1 H. Wilen, "Enantiomers, Racemates and Resolutions", John Wiley And Sons, Inc., 1981.

The reaction can be monitored according to any suitable method known in the art. For example, product formation can be monitored by spectroscopic means (such as nuclear magnetic resonance (NMR) spectroscopy (e.g., $^1H$ or $^{13}C$), infrared (IR) spectroscopy, spectrophotometry (e.g., UV-visible), mass spectrometry (MS)) or by chromatographic methods (such as high performance liquid chromatography (HPLC) or thin layer chromatography (TLC)).

The following general preparation route can be used to synthesize the compound of structural formula (I) of the present disclosure. The synthetic route is as follows:

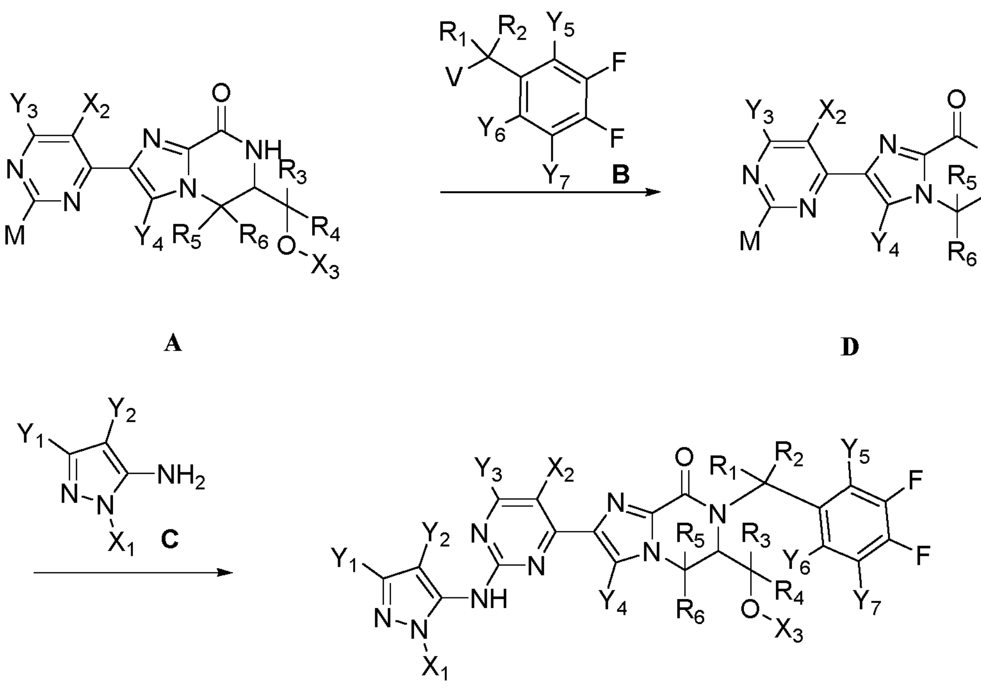


A

D

I

wherein $Y_1$, $Y_2$, $Y_3$, $Y_4$, $Y_5$, $Y_6$, $Y_7$, $R_1$, $R_2$, $R_3$, $R_4$, $R_5$, $R_6$, $X_1$, $X_2$ and $X_3$ are as defined in the summary of the present disclosure, M is a leaving group (e.g., iodine, bromine, chlorine, trifluoromethanesulfonyloxy, etc.) and V is a leaving group (e.g., iodine, bromine, chlorine, trifluoromethanesulfonyloxy, etc.).

The compound of formula (I) can be prepared by reacting a compound of formula (D) with a compound of formula (C). The reaction is carried out at a temperature ranging from about 80 °C to about 150 °C in a suitable solvent (e.g., DME or dioxane) in the presence of a transition metal catalyst (e.g., XantPhos-Pd-G2) and a suitable base (e.g., cesium carbonate, potassium carbonate or sodium carbonate). The compound of formula (D) can be prepared by reacting a compound of formula (A) with a compound of formula (B) in the presence of a suitable base (e.g., sodium hydride or potassium hydride, etc.) and a suitable anhydrous solvent (e.g., DMF or DMA, etc.). The reaction is carried out at a temperature ranging from about 20 °C to 60 °C and can take about 2-4 hours to complete.

## Pharmaceutical compositions, formulations and kits

In another aspect, provided herein is a pharmaceutical composition comprising the compound disclosed herein (also referred to as "active component") and pharmaceutically acceptable excipient(s). In some embodiments, the pharmaceutical composition comprises an effective amount of the active component. In some embodiments, the pharmaceutical composition comprises a therapeutically effective amount of the active component. In some embodiments, the pharmaceutical composition comprises a prophylactically effective amount of the active component.

The "pharmaceutically acceptable excipient" for use in the present disclosure refers to a non-toxic carrier, adjuvant or vehicle that does not destroy the pharmacological activity of the compound formulated together. Pharmaceutically acceptable carriers, adjuvants, or vehicles that can be used in the compositions disclosed herein include, but are not limited to, ion exchangers, alumina, aluminum stearate, lecithin, serum proteins (e.g., human serum albumin), buffer substances (such as phosphate), glycine, sorbic acid, potassium sorbate, a mixture of partial glycerides of saturated plant fatty acids, water, salt or electrolyte (such as protamine sulfate), disodium hydrogen phosphate, potassium hydrogen phosphate, sodium chloride, zinc salt, silica gel, magnesium trisilicate, polyvinyl pyrrolidone, cellulose-based substances, polyethylene glycol, sodium carboxymethyl cellulose, polyacrylate, wax, polyethylene-polyoxypropylene block polymer, polyethylene glycol and lanolin.

The present disclosure also includes a kit (e.g., pharmaceutical packs). The kit provided may include compounds disclosed herein, other therapeutic agents, and first and second containers containing the compounds disclosed herein and other therapeutic agents (e.g., vials, ampoules, bottles, syringes, and/or dispersible packages or other suitable containers). In some embodiments, the kit provided can also optionally include a third container containing a pharmaceutically acceptable excipient for diluting or suspending compounds disclosed herein and/or other therapeutic agents. In some embodiments, the compounds disclosed herein and other therapeutic agents provided in a first container and a second container are combined to form a unit dosage form.

The pharmaceutical composition provided herein can be administered by a variety of routes including, but not limited to, oral administration, parenteral administration, inhalation administration, topical administration, rectal administration, nasal administration, buccal cavity administration, vaginal administration, administration by implant or other means of administration. For example, the parenteral administration as used herein includes subcutaneous administration, intradermal administration, intravenous administration, intramuscular administration, intra-articular administration, intra-arterial administration, intrasynovial administration, intra sternal administration, intracerebroventricular administration, intralesional administration, and intracranial injection or infusion techniques.

Generally, the compounds provided herein are administered in an effective amount. The amount of the compound actually administered will typically be determined by a physician, in the light of the relevant circumstances, including the condition to be treated, the chosen route of administration, the actual compound administered, the age, weight, and response of the individual patient, the severity of the patient's symptoms, and the like.

When used to prevent the condition disclosed herein, the compounds provided herein will be administered to a subject at risk for developing the condition, typically on the advice and under the supervision of a physician, at the dosage levels described above. Subjects at risk for developing a particular condition generally include those that have a family history of the condition, or those who have been identified by genetic testing or screening to be particularly susceptible to developing the condition.

The pharmaceutical compositions provided herein can also be administered chronically ("chronic administration"). Chronic administration refers to the administration of a compound or pharmaceutical composition thereof over an extended period of time, for example, over 3 months, 6 months, 1 year, 2 years, 3 years, 5 years, etc., or may be continued indefinitely, for example, for the rest of the subject's life. In some embodiments, the chronic administration is intended to provide a constant level of the compound in the blood, e.g., within the therapeutic window over the extended period of time.

The pharmaceutical compositions disclosed herein may be further delivered using a variety of dosing methods. For example, in some embodiments, the pharmaceutical composition may be given as a bolus, e.g., in order to rapidly raise the concentration of the compound in the blood to an effective level. The placement of the bolus dose depends on the systemic levels of the active ingredient desired, e.g., an intramuscular or subcutaneous bolus dose allows a slow release of the active ingredient, while a bolus delivered directly to the veins (e.g., through an IV drip) allows a much faster delivery which quickly raises the concentration of the active ingredient in the blood to an effective level. In other embodiments, the pharmaceutical composition may be administered as a continuous infusion, e.g., by IV drip, to provide maintenance of a steady-state concentration of the active ingredient in the subject's body. Furthermore, in still yet other embodiments, the pharmaceutical composition may be administered as first as a bolus dose, followed by continuous infusion.

The compositions for oral administration can take the form of bulk liquid solutions or suspensions, or bulk powders. More commonly, however, the compositions are presented in unit dosage forms to facilitate accurate dosing. The term "unit dosage forms" refers to physically discrete units suitable as unitary dosages for human subjects and other mammals, each unit containing a predetermined quantity of active material calculated to produce the desired therapeutic effect, in association with a suitable pharmaceutical excipient. Typical unit dosage forms include prefilled, premeasured ampules or syringes of the liquid compositions or pills, tablets, capsules or the like in the case of solid compositions. In such compositions, the compound is usually a minor component (from about 0.1% to about 50% by weight or alternatively from about 1% to about 40% by weight) with the remainder being various vehicles or excipients and processing aids helpful for forming the desired dosing form.

With oral dosing, one to five and especially two to four and typically three oral doses per day are representative regimens. Using these dosing patterns, each dose provides from about 0.01 mg/kg to about 20 mg/kg of the compound disclosed herein, with preferred doses each providing from about 0.1 mg/kg to about 10 mg/kg, and especially about 1

mg/kg to about 5 mg/kg.

Transdermal doses are generally selected to provide similar or lower blood levels than are achieved using injection doses, generally in an amount ranging from about 0.01% to about 20% by weight, alternatively from about 0.1% to about 20% by weight, alternatively from about 0.1% to about 10% by weight, or yet alternatively from about 0.5% to about 15% by weight.

Injection dose levels range from about 0.1 mg/kg/hour to at least 10 mg/kg/hour, all for from about 1 to about 120 hours and especially 24 to 96 hours. A preloading bolus of from about 0.1 mg/kg to about 10 mg/kg or more may also be administered to achieve adequate steady state levels. The maximum total dose is not expected to exceed about 2 g/day for a 40 to 80 kg human patient.

Liquid forms suitable for oral administration may include a suitable aqueous or nonaqueous vehicle with buffers, suspending and dispensing agents, colorants, flavors and the like. Solid forms may include, for example, any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate; a glidant such as colloidal silicon dioxide; a sweetening agent such as sucrose or saccharin; or a flavoring agent such as peppermint, methyl salicylate, or orange flavoring.

Injectable compositions are typically based upon injectable sterile saline or phosphate-buffered saline or other injectable excipients known in the art. As mentioned before, the active compound in such compositions is typically a minor component, often being from about 0.05% to 10% by weight with the remainder being the injectable excipient and the like.

Transdermal compositions are typically formulated as a topical ointment or cream containing the active ingredient(s). When formulated as an ointment, the active ingredients will typically be combined with either a paraffinic or a water-miscible ointment base. Alternatively, the active ingredients may be formulated in a cream with, for example an oil-in-water cream base. Such transdermal formulations are well-known in the art and generally include additional ingredients to enhance the stable dermal penetration of the active ingredients or formulation. All such known transdermal formulations and ingredients are included within the scope provided herein.

The compounds disclosed herein can also be administered by a transdermal device. Accordingly, transdermal administration can be accomplished using a reservoir or a patch in porous membrane type or with various solid matrixes.

The above-described components for orally administrable, injectable or topically administrable compositions are merely representative. Other materials as well as processing techniques and the like are set forth in Part 8 of Remington's Pharmaceutical Sciences, 17th edition, 1985, Mack Publishing Company, Easton, Pennsylvania.

The compounds disclosed herein can also be administered in sustained release forms or from sustained release drug delivery systems. A description of representative sustained release materials can be found in Remington's Pharmaceutical Sciences.

The present disclosure also relates to the pharmaceutically acceptable formulations of a compound disclosed herein. In one embodiment, the formulation comprises water. In another embodiment, the formulation comprises a cyclodextrin derivative. The most common cyclodextrins are α-, β- and γ-cyclodextrins consisting of 6, 7 and 8 α-1,4-linked glucose units, respectively, optionally comprising one or more substituents on the linked sugar moieties, which include, but are not limited to, methylated, hydroxyalkylated, acylated, and sulfoalkylether substitution. In some embodiments, the cyclodextrin is a sulfoalkyl ether β-cyclodextrin, e.g., sulfobutyl ether β-cyclodextrin, also known as Captisol. See, e.g., U.S. 5,376,645. In some embodiments, the formulation comprises hexapropyl-β-cyclodextrin (e.g., 10% to 50% in water).

Indications

In another aspect, the present disclosure provides a compound of formulae (I) to (IV) or a pharmaceutically acceptable salt thereof as defined above, for use in the prevention and/or treatment of the proliferative diseases or disorders regulated by RAS/RAF/MEK/ERK kinases.

In another aspect, the present disclosure provides a compound of formulae (I) to (IV) or a pharmaceutically acceptable salt thereof as defined above, for use in the prevention and/or treatment of the proliferative diseases or disorders mediated by ERK.

In another aspect, there is provided a compound of formulae (I) to (IV) or a pharmaceutically acceptable salt thereof as defined above, for use in the prevention and/or treatment of those tumors that are sensitive to the inhibition of ERK.

In another aspect, there is provided a compound of formulae (I) to (IV) or a pharmaceutically acceptable salt thereof as defined above, for use in providing inhibition of ERK.

In another aspect, there is provided a compound of formulae (I) to (IV) or a pharmaceutically acceptable salt thereof as defined above, for use in providing inhibition of ERK2.

The compound of formulae (I) to (IV) or a pharmaceutically acceptable salt thereof can effectively treat any

cancer in which the RAS/RAF/MEK/ERK kinase pathway is activated. Examples of cancers that have been reported to have such activation include acute myelogenous leukemia (AML), chronic myelomonocytic leukemia, multiple myeloma, chronic myelogenous leukemia, colorectal cancer, breast cancer, bladder cancer, head and neck cancer, brain cancer, malignant glioma, neuroblastoma, non-Hodgkin's lymphoma, pancreatic cancer, ovarian cancer, testicular cancer, thyroid cancer, non-small cell lung cancer, small cell lung cancer, melanoma, neurofibromatosis type I, or biliary tract cancer.

In one aspect, the compound can effectively treat cancers selected from non-small cell lung cancer, pancreatic cancer, colorectal cancer, melanoma, uveal melanoma, neurofibromatosis type I in children, differentiated thyroid carcinoma or biliary tract cancer.

In one aspect, the compound can effectively treat KRAS or BRAF mutant cancers.

In one aspect, the compound can effectively treat MAPK pathway-dependent cancers, for example, non-small cell lung cancer, pancreatic cancer and colorectal cancer.

In another aspect, the compound can effectively treat BRAF mutant melanoma.

In another aspect, the compound can effectively treat NRAS mutant melanoma, uveal melanoma, neurofibromatosis type I in children, differentiated thyroid carcinoma or biliary tract cancer.

Combination therapy

The present disclosure provides a compound of formulae (I) to (IV) or a pharmaceutically acceptable salt thereof, together with additional therapeutic agent(s), for use in the combined treatment of cancer.

Additional therapeutic agents include one or more of the following:

(i) antiproliferative/antineoplastic drugs and combinations thereof, as used in medical oncology, such as alkylating agents (for example cis-platin, oxaliplatin, carboplatin, cyclophosphamide, nitrogen mustard, melphalan, chlorambucil, busulphan, temozolamide and nitrosoureas); antimetabolites (for example gemcitabine and antifolates such as fluoropyrimidines like 5-fluorouracil and tegafur, raltitrexed, methotrexate, cytosine arabinoside, and hydroxyurea); antitumor antibiotics (for example anthracyclines like adriamycin, bleomycin, doxorubicin, daunomycin, epirubicin, idarubicin, mitomycin-C, dactinomycin and mithramycin); antimitotic agents (for example vinca alkaloids like vincristine, vinblastine, vindesine and vinorelbine and taxoids like taxol and taxotere and polokinase inhibitors); and topoisomerase inhibitors (for example epipodophyllotoxins like etoposide and teniposide, amsacrine, topotecan and camptothecin);

(ii) antihormonal agents such as antioestrogens (for example tamoxifen, fulvestrant, toremifene, raloxifene, droloxifene and iodoxyfene), antiandrogens (for example bicalutamide, flutamide, nilutamide and cyproterone acetate), LHRH antagonists or LHRH agonists (for example goserelin, leuprorelin and buserelin), progestogens (for example megestrol acetate), aromatase inhibitors (for example as anastrozole, letrozole, vorazole and exemestane) and inhibitors of 5$\alpha$-reductase such as finasteride;

(iii) inhibitors of growth factor function and their downstream signalling pathways:

included are Ab modulators of any growth factor or growth factor receptor targets, reviewed by Stern et al. Critical Reviews in Oncology/Haematology, 2005, 54, pp11-29); also included are small molecule inhibitors of such targets, for example kinase inhibitors - examples include the anti-erbB2 antibody trastuzumab [Herceptin™], the anti-EGFR antibody panitumumab, the anti-EGFR antibody cetuximab [Erbitux, C225] and tyrosine kinase inhibitors including inhibitors of the erbB receptor family, such as epidermal growth factor family receptor (EGFR/erbB 1), tyrosine kinase inhibitors such as gefitinib or erlotinib, erbB2 tyrosine kinase inhibitors such as lapatinib, and mixed erb 1/2 inhibitors such as afatanib; similar strategies are available for other classes of growth factors and their receptors, for example inhibitors of the hepatocyte growth factor family or their receptors including c-met and ron; inhibitors of the insulin and insulin growth factor family or their receptors (IGFR, IR), inhibitors of the platelet-derived growth factor family or their receptors (PDGFR), and inhibitors of signalling mediated by other receptor tyrosine kinases such as c-kit, AnLK, and CSF-1R;

Also included are inhibitors of serine/threonine kinases not listed above, for example raf inhibitors such as vemurafenib, MEK inhibitors such as selumetinib (AZD6244, ARRY-142886), cobimetinib or GDC-0623 (see for example WO2015/0832840), Abl inhibitors such as imatinib or nilotinib, Btk inhibitors such as ibrutinib, Syk inhibitors such as fostamatinib, aurora kinase inhibitors (for example AZD1152), inhibitors of other ser/thr kinases such as JAKs, STATs and IRAK4, and cyclin dependent kinase inhibitors;

(iv) modulators of DNA damage signalling pathways, for example PARP inhibitors (e.g. Olaparib), ATR inhibitors or ATM inhibitors;

(v) modulators of apoptotic and cell death pathways such as Bel family modulators (e.g. ABT-263/Navitoclax, ABT-199);

(vi) antiangiogenic agents such as those which inhibit the effects of vascular endothelial growth factor, [for example the anti-vascular endothelial cell growth factor antibody bevacizumab (Avastin™) and for example, a VEGF receptor tyrosine kinase inhibitor such as sorafenib, axitinib, pazopanib, sunitinib and vandetanib (and compounds that work by other mechanisms (for example linomide, inhibitors of integrin $\alpha v\beta 3$ function and angiostatin))];
(vii) vascular damaging agents, such as Combretastatin A4;
(viii) anti-invasion agents, for example c-Src kinase family inhibitors like (dasatinib, J. Med. Chem., 2004, 47, 6658-6661) and bosutinib (SKI-606), and metalloproteinase inhibitors like marimastat, inhibitors of urokinase plasminogen activator receptor function or antibodies to Heparanase];
(ix) immunotherapy approaches, including for example ex-vivo and in-vivo approaches to increase the immunogenicity of patient tumor cells, such as transfection with cytokines such as interleukin 2, interleukin 4 or granulocyte-macrophage colony stimulating factor, approaches to decrease T-cell anergy, approaches using transfected immune cells such as cytokine-transfected dendritic cells, approaches using cytokine-transfected tumor cell lines and approaches using anti-idiotypic antibodies. Specific examples include monoclonal antibodies targeting PD-1 (e.g. BMS-936558), PDL-1 or CTLA4 (e.g. ipilimumab and tremelimumab);
(x) Antisense or RNAi based therapies, for example those which are directed to the targets listed.
(xi) gene therapy approaches, including for example approaches to replace aberrant genes such as aberrant p53 or aberrant BRCA1 or BRCA2, GDEPT (gene-directed enzyme prodrug therapy) approaches such as those using cytosine deaminase, thymidine kinase or a bacterial nitroreductase enzyme and approaches to increase patient tolerance to chemotherapy or radiotherapy such as multi-drug resistance gene therapy.

According to this aspect there is provided a combination suitable for use in the treatment of cancer comprising a compound of Formulae (I) to (IV) or a pharmaceutically acceptable salt thereof as defined hereinbefore and another anti-tumor agent, in particular any one of the anti-tumor agents listed under (i) to (xi) above. In particular, the anti-tumor agent listed under (i) to (xi) above is the standard of care for the specific cancer to be treated; the person skilled in the art will understand the meaning of "standard of care".
Therefore in a further aspect there is provided a compound of Formula (I) to (IV) or a pharmaceutically acceptable salt thereof in combination with another anti-tumor agent, in particular an anti-tumor agent selected from one listed under (i) to (xi) herein above.
According to a further aspect there is provided a pharmaceutical composition which comprises a compound of Formula (I) to (IV) or a pharmaceutically acceptable salt thereof in combination with an anti-tumor agent selected from one listed under (i) to (xi) herein above, in association with pharmaceutically acceptable diluent(s) or carrier(s).
According to a further aspect there is provided a pharmaceutical composition which comprises a compound of Formula (I) to (IV) or a pharmaceutically acceptable salt thereof in combination with an anti-tumor agent selected from one listed under (i) to (xi) herein above, in association with pharmaceutically acceptable diluent(s) or carrier(s) for use in treating proliferative disease.

Examples

The present disclosure is further illustrated below in conjunction with specific examples. It is to be understood that the examples are used to illustrate the present disclosure, and not intended to limit the scope of present disclosure. In the following examples, the experimental methods wherein the particular conditions are not specified are usually in accordance with conventional conditions or according to the conditions recommended by the manufacturer. Parts and percentages are parts by weight and percentage by weight unless otherwise stated.
The abbreviations used herein have the following meanings:

| | |
|---|---|
| APCI | Atmospheric pressure chemical ionization |
| Boc | Tert-butoxycarbonyl |
| MSCl | Methanesulfonyl chloride |
| SEMCl | 2-(Trimethylsilyl)ethoxymethyl chloride |
| NBS | N-Bromosuccinimide |
| $B_2(pin)_2$ | Bis(pinacolato)diboron |
| X-Phos | 2-Dicyclohexylphosphino-2',4',6'-triisopropylbiphenyl |
| $Pd_2(dba)_3CHCl_3$ | Tris(dibenzylideneacetone)dipalladium-chloroform adduct |
| $Pd(PPh_3)_4$ | Tetrakis(triphenylphosphine)palladium |
| XantPhos-Pd-G2 | Chloro[(4,5-bis(diphenylphosphino)-9,9-dimethylxanthene)-2-(2'-amino-1,1'-biphenyl)]palladium(II) |
| $NaBH_4$ | Sodium borohydride |
| $LiAlD_4$ | Lithium aluminum deuteride |

| | |
|---|---|
| NaH | Sodium hydride |
| KOAc | Potassium acetate |
| $Cs_2CO_3$ | Cesium carbonate |
| $K_2CO_3$ | Potassium carbonate |
| DIPEA | N,N-Diisopropylethylamine |
| TEA | triethylamine |
| TFA | trifluoroacetic acid |
| HCl | hydrochloric acid |
| THF | tetrahydrofuran |
| DCM | dichloromethane |
| DMF | N,N-Dimethylformamide |
| Dioxane | 1,4-Dioxane |
| EtOAc | Ethyl acetate |
| PE | Petroleum ether |
| $CDCl_3$ | Deuterated chloroform |
| DMSO | Dimethylsulphoxide |
| MeOH | Methanol |
| MeOD | Deuterated methanol |

**Example 1 Preparation of (R)-2-(2-chloro-5-methylpyrimidin-4-yl)-6-(methoxymethyl)-6,7-dihydroimidazo[1,2-a]pyrazin-8(5H)-one (Intermediate A-1).**

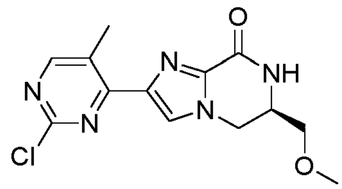

A-1

The following route was used for the synthesis:

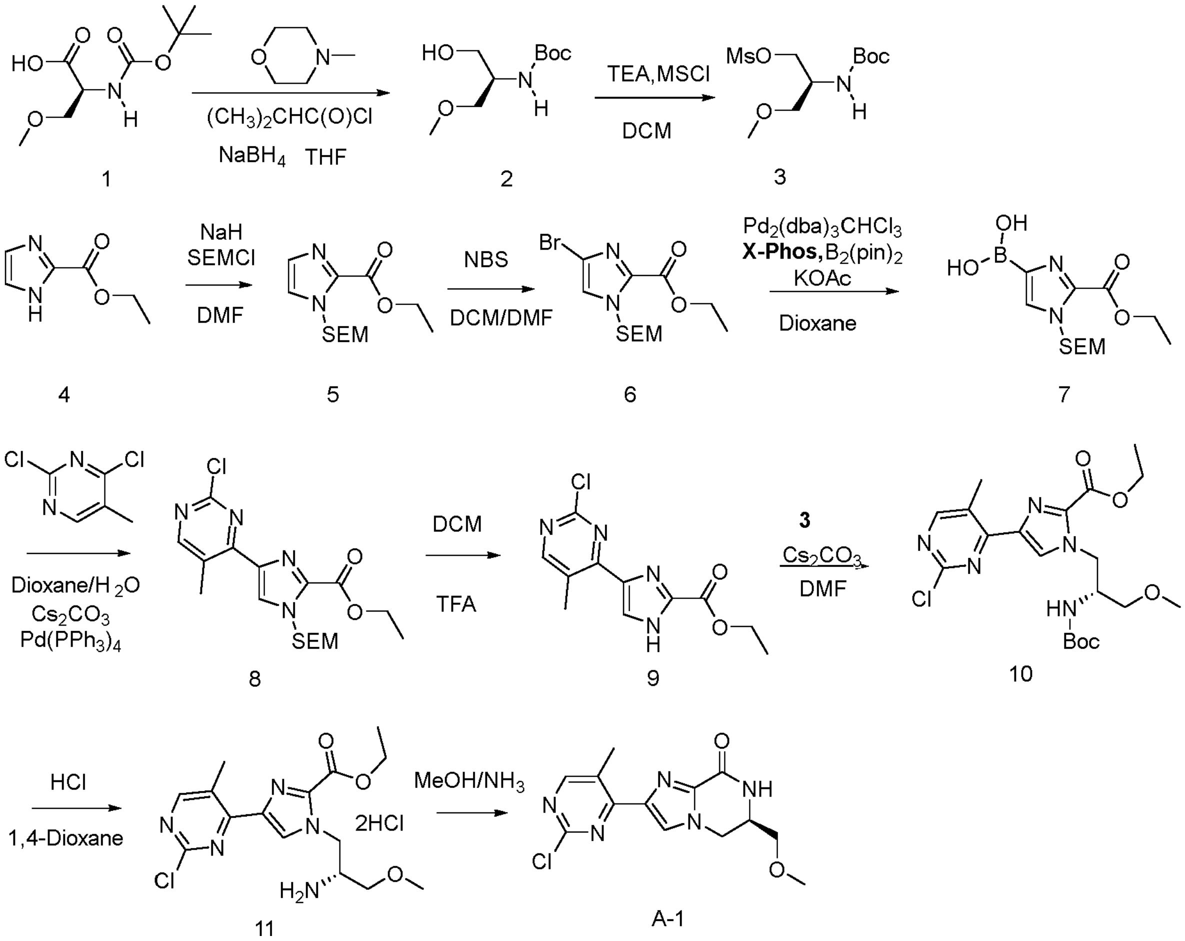


1 2 3 4 5 6 7 8 9 10 11 A-1

Step 1 Synthesis of compound 2

At -15 °C, a solution of isobutyryl chloride (14.96 g, 110 mol) in anhydrous THF (20 mL) was slowly added dropwise to a solution of compound 1 (21.8 g, 99.54 mmol) and N-methylmorpholine (11.2 g, 110 mmol) in anhydrous THF (100 mL), and after the addition, the reaction was continued for 20 min at -15 °C.

A solution of $NaBH_4$ (12.00 g, 318 mmol) in $H_2O$ (20 mL) was slowly added to the above reaction solution, and then reacted at -15 °C for 45 min. EtOAc (700 mL) was added to dilute the reaction solution, and the system was adjusted to neutral with dilute hydrochloric acid (2 M). The organic layer was separated, washed with saturated saline solution (100 mL), dried over anhydrous sodium sulfate, and concentrated under reduced pressure. The concentrate was purified by column chromatography (PE/EtOAc= 20%) to give 13 g of light yellow oil. Yield: 62.83%. $^1$H NMR (300 MHz, $CDCl_3$) δ 5.19 (s, 1H), 3.83 - 3.60 (m, 3H), 3.59 - 3.44 (m, 2H), 3.35 (s, 3H), 2.96 (s, 1H), 1.43 (s, 9H).

Step 2 Synthesis of compound 3

In an ice bath, a solution of MSCl (2.27 g, 19.83 mmol) in anhydrous DCM (20 mL) was slowly added to a solution of compound 2 (3.4 g, 16.58 mmol) and TEA (2.06 g, 20.4 mmol) in anhydrous DCM (100 ml), and the reaction solution was stirred and reacted for 2.5 h at room temperature. Water (30 mL) was added to quench the reaction, and the organic layer was separated, washed with saturated aqueous solution of sodium bicarbonate, dried over anhydrous sodium sulfate, and concentrated under reduced pressure to afford light yellow oil which was directly used in the reaction of the next step. Yield: 100%.

Step 3 Synthesis of compound 5

In an ice bath, NaH (6.42 g, 160.5 mmol) was added to a solution of compound 4 (15 g, 107.04 mmol) in anhydrous DMF (75 mL), and after stirring for 30 min, SEMCl (28.47 mL, 160.56 mmol) was added to the reaction solution, which was stirred at room temperature overnight. The reaction was quenched by adding water (100 mL), and extracted with ethyl acetate (200 mL × 3). The organic layers were combined, washed with saturated saline solution

(100 mL × 4), dried over anhydrous sodium sulfate, and concentrated under reduced pressure. The concentrate was purified by column chromatography (PE/EtOAc= 10%) to give 17 g of light yellow oil. Yield: 58.9%. $^1$H NMR (500 MHz, $CDCl_3$) δ 7.26 (d, *J* = 1.0 Hz, 1H), 7.20 (d, *J*= 1.0 Hz, 1H), 5.79 (s, 2H), 4.42 (q, *J*= 7.1 Hz, 2H), 3.58 - 3.54 (m, 2H), 1.43 (t, *J*= 7.1 Hz, 3H), 0.94 - 0.90 (m, 2H), -0.03 (s, 9H).

Step 4 Synthesis of compound 6

In an ice bath, NBS (4.93 g, 27.70 mmol) was added to a solution of compound 5 (6.8 g, 25.18 mmol) in the mixture of anhydrous DMF (30 mL) and anhydrous DCM (30 mL) in batches, and the reaction was reacted at room temperature for 24 h. The reaction was quenched by adding water (100 mL), and DCM was removed under reduced pressure. The reaction solution was extracted with ethyl acetate (150 mL × 3), and the organic layers were combined, washed with saturated saline solution (100 mL × 4), dried over anhydrous sodium sulfate, and concentrated under reduced pressure. The concentrate was purified by column chromatography (PE/EtOAc= 10%) to give 5 g of light yellow oil. Yield: 57%. $^1$H NMR (400 MHz, DMSO-$d_6$) δ 7.82 (d, *J* = 14.5 Hz, 1H), 5.66 (s, 2H), 4.30 (q, *J* = 7.1 Hz, 2H), 3.58 - 3.44 (m, 2H), 1.30 (t, *J* = 7.1 Hz, 3H), 0.89 - 0.74 (m, 2H), -0.06 (s, 9H).

Step 5 Synthesis of compound 7

Under the protection of nitrogen, X-phos (410 mg) and $Pd_2(dba)_3CHCl_3$ (370 mg) were added to a solution of compound 6 (5.00 g, 14.36 mmol), $B_2(pin)_2$ (4.36 g) and potassium acetate (4.20 g) in anhydrous dioxane (60 mL), and the reaction was heated to 80 °C and reacted overnight. The reaction was cooled to room temperature, filtered with celite, and the filter cake was washed with dioxane. The filtrate was concentrated under reduced pressure to give light yellow oil, which was directly used in the reaction of the next step.

Step 6 Synthesis of compound 8

The above compound 7 was dissolved in a mixed solvent of dioxane (90 mL) and water (15 mL), to which compound 2,4-dichloro-5-methylpyrimidine (2.60 g, 15.80 mmol) and $Cs_2CO_3$ (9.34 g, 28.72 mmol) were added, the atmosphere was replaced with nitrogen, and $Pd(PPh_3)_4$ (1.00g) was added under nitrogen protection to the reaction solution, which was reacted at 85 °C for 2.5 h. The reaction was cooled to room temperature, filtered with celite, and dioxane was removed from filtrate under reduced pressure. The reaction was quenched by adding 50 mL water, extracted with ethyl acetate (200 mL × 3), the organic layers were combined, washed with saturated saline solution, dried over anhydrous sodium sulfate, and concentrated under reduced pressure. The concentrate was purified by column chromatography (PE/EtOAc= 5%) to give 3.72 g of light yellow solid. Yield: 65.4%. LC-MS (APCI): m/z = 397.0$(M+1)^+$. $^1$H NMR (300 MHz, $CDCl_3$) δ 8.42 (s, 1H), 8.13 (s, 1H), 5.82 (s, 2H), 4.45 (q, *J* = 7.1 Hz, 2H), 3.71 - 3.50 (m, 2H), 2.70 (s, 3H), 1.45 (t, *J* = 7.1 Hz, 3H), 0.98 - 0.91 (m, 2H), -0.01 (s, 9H).

Step 7 Synthesis of compound 9

TFA(13.0 mL) was added to a solution of compound 8 (3.7g, 9.34 mmol) in DCM (13 mL), and the reaction was reacted at room temperature overnight. The reaction solution was concentrated under reduced pressure, to which saturated sodium bicarbonate solution (30 mL) was added, and stirred for 2 h. The solid was filtered and washed with water, and dried in vacuum to give 2.25 g of light yellow solid. Yield: 91.0%. LC-MS (APCI): m/z = 267.1$(M+1)^+$. $^1$H NMR (300 MHz, DMSO-$d_6$) δ 13.96 (s, 1H), 8.59 (s, 1H), 8.11 (s, 1H), 4.34 (q, *J* = 7.1 Hz, 2H), 2.59 (s, 3H), 1.32 (t, *J* = 7.1 Hz, 3H).

Step 8 Synthesis of compound 10

$Cs_2CO_3$ (2.70, 8.28 mmol (2-3 eq.)) was added to a solution of compound 9 (1.10 g, 4.14 mmol, 1 eq.) and compound 3 (3.0-4 eq.) in anhydrous DMF (15 mL), and under the protection of nitrogen, the reaction solution was reacted for 20 h at 100 °C. The reaction was cooled to room temperature, quenched by adding water (30 mL), and extracted with ethyl acetate (50 mL × 4). The organic layers were combined, washed with saturated saline solution (30 mL × 4), dried over anhydrous sodium sulfate, and concentrated under reduced pressure. The concentrate was purified by column chromatography (PE/EtOAc= 20%) to give 820 mg of light yellow solid. Yield: 40.0%. LC-MS (APCI): m/z = 454.1$(M+1)^+$, $^1$H NMR (300 MHz, $CDCl_3$) δ 8.39 (s, 1H), 7.95 (s, 1H), 5.09 (d, *J* = 8.8 Hz, 1H), 4.70 (dd, *J* = 13.4, 4.3 Hz, 1H), 4.60 - 4.33 (m, 3H), 4.22 (s, 1H), 3.49 (dd, *J* = 6.8, 3.3 Hz, 2H), 3.37 (s, 3H), 2.69 (s, 3H), 1.45 (t, *J* = 7.1 Hz, 3H), 1.30 (s, 9H).

Step 9 Synthesis of compound 11

A solution of HCl (15 mL) in dioxane was added to compound 10 (770 mg, 1.70 mmol), which was stirred overnight at room temperature. The solid was filtered, washed with ethyl acetate (15 mL), and dried in vacuum. The resulted product was directly used in the reaction of the next step. LC-MS (APCI): m/z = 354.1(M+1)$^+$.

Step 10 Synthesis of compound A-1

The above compound 11 was added to the MeOH/$NH_3$ (30 mL, 7N) solution, which was stirred overnight at room temperature. The reaction solution was concentrated under reduced pressure, and the concentrate was purified by column chromatography (DCM/MeOH= 5%) to give 465 mg of light yellow solid. Yield of the two steps: 87.4%. LC-MS (APCI): m/z = 308.0(M+1)$^+$.

**Example 2 Preparation of 2-(2-chloro-5-methylpyrimidin-4-yl)-6-((methoxy-*d*$_3$)methyl)-6,7-dihydroimidazo[1,2-a]pyrazin-8(5H)-one (Intermediate A-2).**

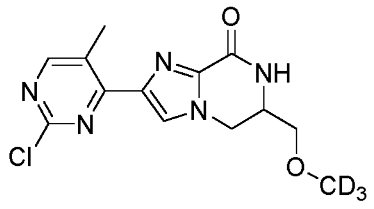

A-2

The following route was used for the synthesis:

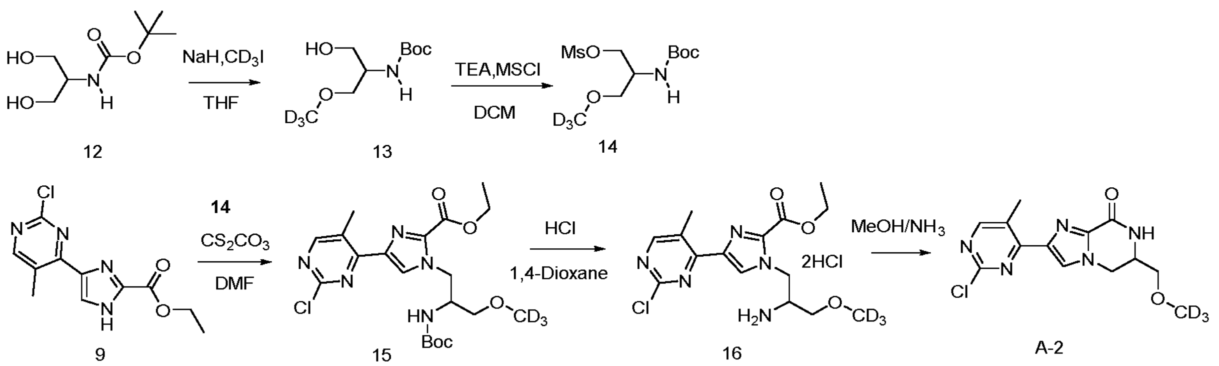


Step 1 Synthesis of compound 13

In an ice bath, NaH (2.67 g, 66.75 mol) was added to a solution of compound 12 (12.18 g, 63.69 mmol) in anhydrous THF (200 mL), and after addition, the reaction was continued for 30 min at room temperature.

In an ice bath, a solution of deuterated methyl iodide (8.70 g, 60.00 mmol) in anhydrous THF (20 mL) was slowly added to the above reaction solution, and reacted at room temperature for 3 h. The reaction was quenched by adding water (200 mL). THF was removed under reduced pressure, EtOAc (700 mL) was added to dilute the reaction solution, and the system was adjusted to neutral with dilute hydrochloric acid (2 M). The organic layer was separated and washed with saturated saline solution (100 mL), dried over anhydrous sodium sulfate, and concentrated under reduced pressure. The concentrate was purified by column chromatography (PE/EtOAc= 20%) to give 3.1 g of light yellow oil. Yield: 20.1%.

Step 2 Synthesis of compound 14

In an ice bath, a solution of MsCl (1.40 g) in anhydrous DCM (10 mL) was slowly added to a solution of compound 13 (2.1 g, 10.24 mmol) and TEA (1.27 g) in anhydrous DCM (70 ml), and the reaction solution was stirred and reacted at room temperature for 2.5 h. Water (30 mL) was added to quench the reaction, and the organic layer was separated,

and washed with saturated aqueous solution of sodium bicarbonate, dried over anhydrous sodium sulfate, and concentrated under reduced pressure to afford light yellow oil, which was directly used in the reaction of next step. Yield: 100%.

Step 3 Synthesis of compound 15

$Cs_2CO_3$ (3.05 g, 9.38 mmol) was added to a solution of compound 9 (1.00 g, 3.75 mmol, 1 eq.) and the above compound 14 (10.24 mmol) in anhydrous DMF (15 mL), and under the protection of nitrogen, the reaction solution was reacted for 20 h at 100 °C. The reaction was cooled to room temperature, quenched by adding water (30 mL), and extracted with ethyl acetate (50 mL × 4) The organic layers were combined, washed with saturated saline solution (30 mL × 4), dried over anhydrous sodium sulfate, and concentrated under reduced pressure. The concentrate was purified by column chromatography (PE/EtOAc= 20%) to give 760 mg of light yellow solid. Yield: 44.4%. LC-MS (APCI): m/z = 457.1$(M+1)^+$.

Step 4 Synthesis of compound 16

A solution of HCl (15 mL) in dioxane was added to compound 15 (760 mg, 1.66 mol), and the mixture was stirred at room temperature overnight. The solid was filtered, washed with ethyl acetate (15 mL), and dried in vacuum. The resulted product was directly used in the reaction of the next step. LC-MS (APCI): m/z = 357.1$(M+1)^+$.

Step 5 Synthesis of compound A-2

The above compound 16 was added to the $MeOH/NH_3$ (30 mL, 7N) solution, stirred at room temperature overnight. The reaction was concentrated under reduced pressure, and the concentrate was purified by column chromatography (DCM/MeOH= 5%) to give 530 mg of light yellow solid. Yield of the two steps: 100%. LC-MS (APCI): m/z = 311.1$(M+1)^+$.

**Example 3 Preparation of 4-(bromomethyl-*d*$_2$)-1,2-difluorobenzene (Intermediate B-1).**

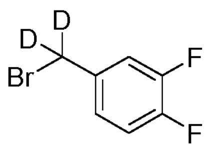
B-1

The following route was used for the synthesis:

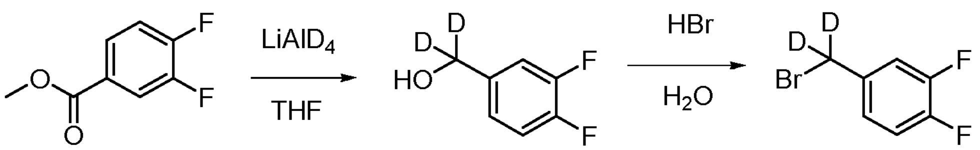


B-1

Step 1 Synthesis of compound (3,4-difluorophenyl)methan-*d*$_2$-ol

In an ice bath, a solution of compound methyl 3,4-difluorobenzoate (2.00 g, 11.62 mmol) in anhydrous THF (10 mL) was slowly added dropwise to a solution of $LiAlD_4$ (490 mg, 11.62 mmol) in anhydrous THF (40 mL), and after the addition, the reaction was continued for 60 min at room temperature. In an ice bath, heavy water (3 mL) was added to the above reaction solution and stirred for another 30 min. The reaction was filtered with celite, and the filter cake was washed with THF. The filtrate was dried over anhydrous sodium sulfate, and concentrated under reduced pressure to afford light yellow liquid, which was directly used in the reaction of the next step.

Step 2 Synthesis of compound 4-(bromomethyl-*d*$_2$)-1,2-difluorobenzene (Intermediate B-1)

HBr (33%, 10 mL) was added to the above compound (3,4-difluorophenyl)methan-*d*$_2$-ol, and the reaction solution was refluxed for 1 h. The reaction was cooled to room temperature, extracted with DCM (50 mL × 2), and the organic

layer was washed with saturated saline solution (50 mL), dried over anhydrous sodium sulfate, and concentrated under reduced pressure. The concentrate was purified by column chromatography (PE/EtOAc= 1%) to give 1.7 g of light yellow liquid. Yield: 80%. $^1H$ NMR (300 MHz, $CDCl_3$) $\delta$ 7.26 - 7.17 (m, 1H), 7.15 - 7.08 (m, 2H).

**Example 4 Preparation of 1-(methyl-*$d_3$*)-1*H*-pyrazol-5-amine (Intermediate C-1).**

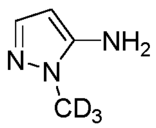

C-1

The following route was used for the synthesis:

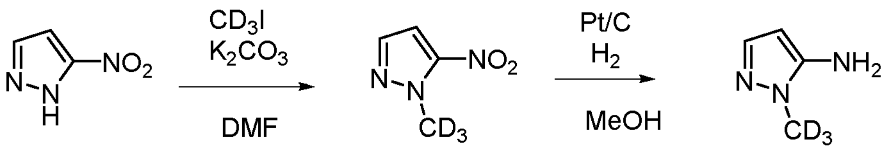


C-1

Step 1 Synthesis of compound 1-(methyl-*$d_3$*)-5-nitro-1H-pyrazole

Deuterated methyl iodide (2.90 g, 20.0 mmol) was added to a solution of compound 5-nitro-1H-pyrazole (1.13 g, 10.00 mmol) and $K_2CO_3$ (4.14 g, 30.00 mmol) in anhydrous DMF (40 mL), and under the protection of nitrogen, the reaction solution was reacted at 45 °C for 24 h. The reaction was cooled to room temperature, was quenched by adding water (100 mL), and extracted with ethyl acetate (150 mL $\times$ 3). The organic layers were combined, washed with saturated saline solution (100 mL $\times$ 3), dried over anhydrous sodium sulfate, and concentrated under reduced pressure. The concentrate was purified by column chromatography (PE/EtOAc= 35%) to give 100 mg of light yellow oil.

Step 2 Synthesis of compound 1-(methyl-*$d_3$*)-1*H*-pyrazol-5-amine (Intermediate C-1)

5% Pt-C (50 mg) was added to compound 1-(methyl-*$d_3$*)-5-nitro-1*H*-pyrazole (100 mg) in anhydrous methanol, and reacted under hydrogen atmosphere for 4 h. The reaction solution was filtered with celite, and the filtrate was concentrated under reduced pressure. The concentrate was purified by column chromatography (DCM/MeOH= 10%) to give 70 mg of light yellow solid. $^1H$ NMR (500 MHz, DMSO-*$d_6$*) $\delta$ 6.96 (d, *J* = 1.7 Hz, 1H), 5.22 (d, *J* = 1.8 Hz, 1H), 5.08 (s, 2H).

**Example 5 Preparation of (R)-7-(3,4-difluorobenzyl)-6-(methoxymethyl)-2-(5-methyl-2-**((1-(methyl-*$d_3$*)-1*H*-pyrazol-5-yl)amino)pyrimidin-4-yl)-6,7-dihydroimidazo[1,2-a]pyrazin-8(5H)-one (Compound 1-1).

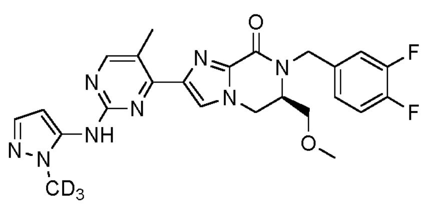

I-1

The following route was used for the synthesis:

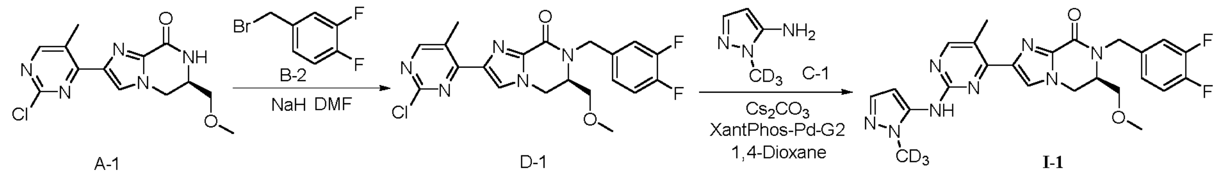


Step 1 Synthesis of compound D-1

At room temperature, compound B-2 (460 mg, 2.22 mmol) was added to a solution of compound A-1 (456 mg, 1.48 mmol) and NaH (152 mg, 3.82 mmol) in anhydrous DMF (10 mL), and reacted at room temperature for 2.5 h. Water (30 mL) was added to quench the reaction, which was extracted with ethyl acetate (50 mL × 3), and the organic layers were combined, washed with saturated saline solution (30 mL × 3), dried over anhydrous sodium sulfate, and concentrated under reduced pressure. The concentrate was purified by column chromatography (dichloromethane/methanol= 4%) to give 620 mg of light yellow solid. Yield: 96.75%. LC-MS (APCI): m/z = 434.2(M+1)$^+$. $^1$H NMR (500 MHz, $CDCl_3$) δ 8.42 (d, *J* = 0.5 Hz, 1H), 7.94 (s, 1H), 7.25 - 7.19 (m, 1H), 7.18 - 7.08 (m, 2H), 5.33 (d, *J* = 15.0 Hz, 1H), 4.42 (dd, *J* = 13.2, 1.0 Hz, 1H), 4.28 - 4.17 (m, 2H), 3.85 - 3.78 (m, 1H), 3.38 (dd, *J* = 9.4, 4.9 Hz, 1H), 3.31 - 3.25 (m, 4H), 2.76 (s, 3H).

Step 2 Synthesis of compound I-1

Under the protection of nitrogen, XantPhos-Pd-G2 (15 mg, 0.015 mmol) was added to a solution of compound D-1 (120 mg, 0.27 mmol), compound C-1 (64 mg, 0.64 mmol) and $Cs_2CO_3$ (190 mg, 0.58 mmol) in anhydrous dioxane (8 mL), and reacted at 100 °C overnight. The reaction was cooled to room temperature, filtered with celite, and the filtrate was concentrated under reduced pressure, added with DCM (200 mL), washed with saturated saline solution (50 mL × 3), dried over anhydrous sodium sulfate, and concentrated under reduced pressure. The concentrate was purified by column chromatography (dichloromethane/methanol= 5%) to give 125 mg of brown solid. Yield: 92.9%. Purity: 96.78%(HPLC); LC-MS (APCI): m/z = 498.3(M+1)$^+$. $^1$H NMR (300 MHz, DMSO-*d*$_6$) δ 9.20 (s, 1H), 8.30 (s, 1H), 7.92 (s, 1H), 7.50 - 7.34 (m, 2H), 7.31 (d, *J*= 1.9 Hz, 1H), 7.25 (d, *J* = 4.2 Hz, 1H), 6.28 (d, *J* = 1.8 Hz, 1H), 5.06 (d, *J* = 15.4 Hz, 1H), 4.56 - 4.29 (m, 3H), 4.10 - 3.93 (m, 1H), 3.42 - 3.34 (m, 2H), 3.16 (s, 3H), 2.49 (s, 3H).

**Example 6 Preparation of (*R*)-7-((3,4-difluorophenyl)methyl-*d*$_2$)-6-(methoxymethyl)-2-(5-methyl-2-((1-methyl-1*H*-pyrazol-5-yl)amino)pyrimidin-4-yl)-6,7-dihydroimidazo[1,2-a]pyrazin-8(5H)-one (Compound 1-2).**

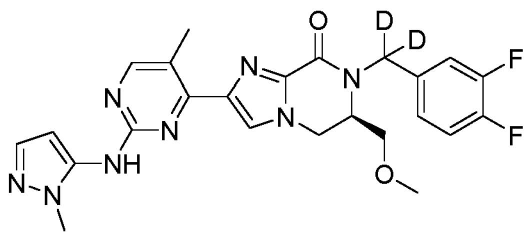

I-2

The following route was used for the synthesis:

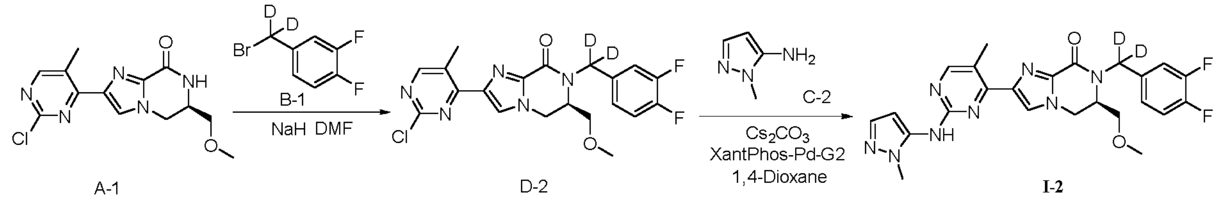


Step 1 Synthesis of compound D-2

At room temperature, compound B-1 (205 mg, 0.98 mmol) was added to a solution of compound A-1 (200 mg, 0.65 mmol) and NaH (65 mg, 1.63 mmol) in anhydrous DMF (8 mL), and reacted at room temperature for 2.5 h. Water

(30 mL) was added to quench the reaction, which was extracted with ethyl acetate (50 mL × 3), and the organic layers were combined, washed with saturated saline solution (30 mL × 3), dried over anhydrous sodium sulfate, and concentrated under reduced pressure. The concentrate was purified by column chromatography (dichloromethane/methanol= 4%) to give 190 mg of light yellow solid. Yield: 67.2%. LC-MS (APCI): m/z = 436.2$(M+1)^+$.

Step 2 Synthesis of compound I-2

Under the protection of nitrogen, XantPhos-Pd-G2 (10 mg) was added to a solution of compound D-2 (95 mg, 0.22 mmol), compound C-2 (50 mg, 0.54 mmol) and $Cs_2CO_3$ (142 mg, 0.44 mmol) in anhydrous dioxane (6 mL), and reacted at 100 °C overnight. The reaction was cooled to room temperature, filtered with celite, and the filtrate was concentrated under reduced pressure, added with DCM (100 mL), washed with saturated saline solution (25 mL × 3), dried over anhydrous sodium sulfate, and concentrated under reduced pressure. The concentrate was purified by column chromatography (dichloromethane/methanol= 6%) to give 90 mg of brown solid. Yield: 83.2%. Purity: 98.69%(HPLC); LC-MS (APCI): m/z = 497.2$(M+1)^+$. $^1$H NMR (300 MHz, DMSO-$d_6$) δ 9.20 (s, 1H), 8.30 (s, 1H), 7.92 (s, 1H), 7.51 - 7.34 (m, 2H), 7.33 - 7.29 (m, 1H), 7.29 - 7.17 (m, 1H), 6.28 (d, *J* = 1.8 Hz, 1H), 4.58 - 4.33 (m, 2H), 4.07 - 3.92 (m, 1H), 3.68 (s, 3H), 3.41 - 3.36 (m, 1H), 3.31 - 3.25 (m, 1H), 3.16 (s, 3H), 2.49 (s, 3H).

**Example 7 Preparation of (R)-7-((3,4-difluorophenyl)methyl-*d*$_2$)-6-(methoxymethyl)-2-(5-methyl-2-((1-(methyl-*d*$_3$)-1H-pyrazol-5-yl)amino)pyrimidin-4-yl)-6,7-dihydroimidazo[1,2-a]pyrazin-8(5H)-one (Compound 1-3).**

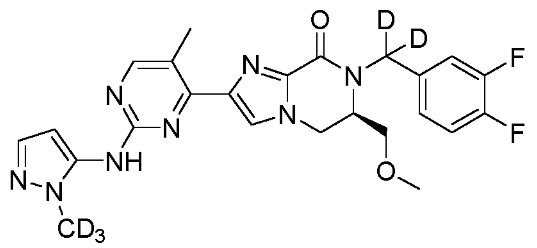

I-3

The following route was used for the synthesis:

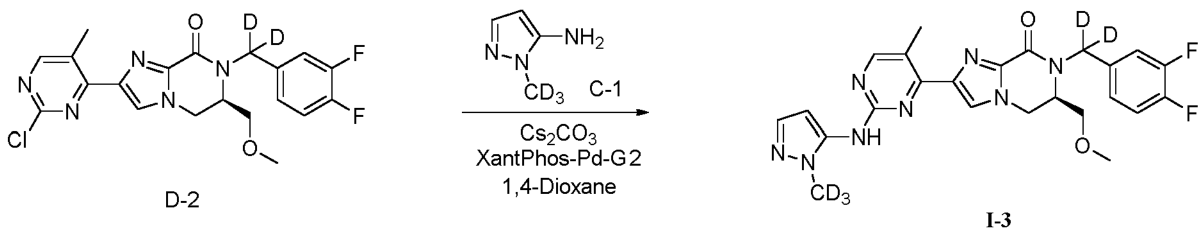


D-2

I-3

Under the protection of nitrogen, XantPhos-Pd-G2 (20 mg) was added to a solution of compound D-2 (110 mg, 0.25 mmol), compound C-1 (60 mg, 0.63 mmol) and $Cs_2CO_3$ (165 mg, 0.50 mmol) in anhydrous dioxane (8 mL), and reacted at 100 °C overnight. The reaction was cooled to room temperature, filtered with celite, and the filtrate was concentrated under reduced pressure, added with DCM (120 mL), washed with saturated saline solution (25 mL × 3), dried over anhydrous sodium sulfate, and concentrated under reduced pressure. The concentrate was purified by column chromatography (dichloromethane/methanol= 6%) to give 115 mg of brown solid. Yield: 92.0%. Purity: 99.19%(HPLC); LC-MS (APCI): m/z = 500.2$(M+1)^+$. $^1$H NMR (300 MHz, DMSO-$d_6$) δ 9.21 (s, 1H), 8.30 (s, 1H), 7.92 (s, 1H), 7.54 - 7.34 (m, 2H), 7.31 (s, 1H), 7.27 - 7.17 (m, 1H), 6.28 (s, 1H), 4.57 - 4.35 (m, 2H), 4.09 - 3.94 (m, 1H), 3.40 - 3.36 (m, 2H), 3.15 (s, 3H), 2.49 (s, 3H).

**Example 8 Preparation of 7-(3,4-difluorobenzyl)-6-((methoxy-*d*$_3$)-methyl)-2-(5-methyl-2-((1-methyl-1H-pyrazol-5-yl)amino)pyrimidin-4-yl)-6,7-dihydroimidazo [1,2-a] pyrazin-8(5H)-one (Compound 1-4), (*R*)-7-(3,4-difluorobenzyl)-6-((methoxy-*d*$_3$)-methyl)-2-(5-methyl-2-((1-methyl-1H-pyrazol-5-yl)amino)pyrimidin-4-yl)-6,7-dihydroimidazo [1,2-a]pyrazin-8(5H)-one (Compound 1-4-1) and (*S*)-7-(3,4-difluorobenzyl)-6-((methoxy-*d*$_3$)-methyl)-2-(5-methyl-2-((1-methyl-1H-pyrazol-5-yl)amino)pyrimidin-4-yl)-6,7-dihydroimidazo[1,2-a]pyrazin-8(5H)-one (Compound 1-4-2).**

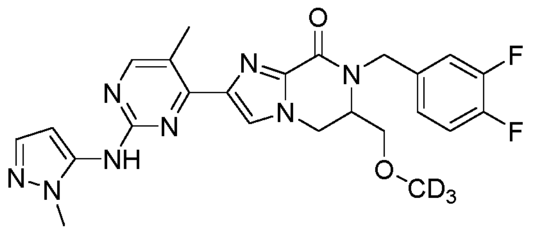

I-4,

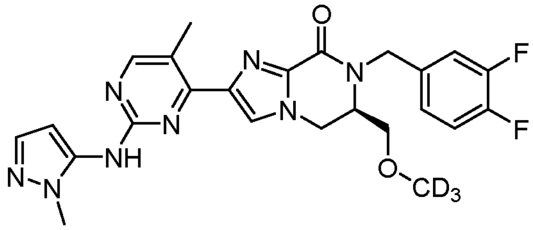

I-4-1,

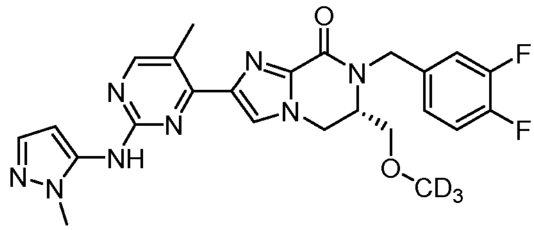

I-4-2

The following route was used for the synthesis:

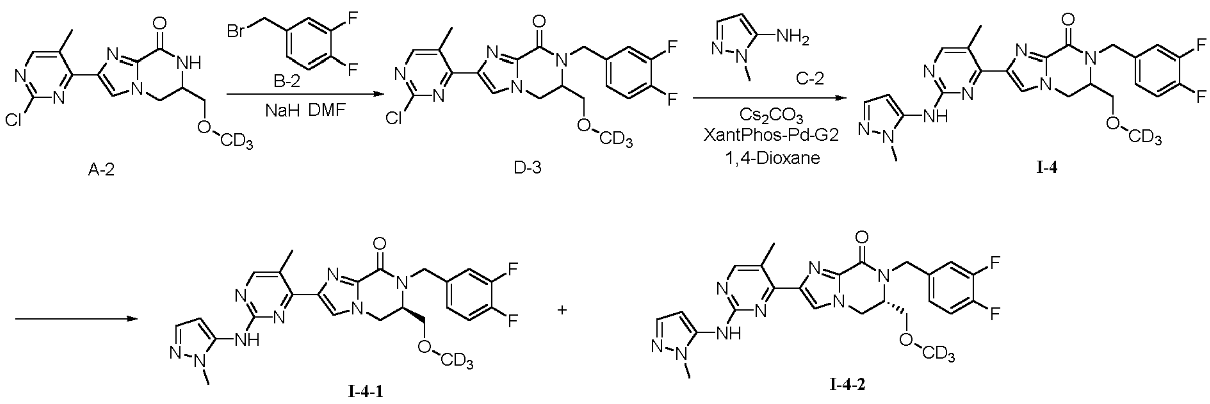


Step 1 Synthesis of compound D-3

At room temperature, Compound B-2 (536 mg, 2.59 mmol) was added to a solution of compound A-2 (530 mg, 1.72 mmol) and NaH (172 mg, 4.30 mmol) in anhydrous DMF (10 mL), and reacted at room temperature for 2.5 h. Water (30 mL) was added to quench the reaction, which was extracted with ethyl acetate (50 mL × 3), and the organic layers were combined, washed with saturated saline solution (30 mL × 3), dried over anhydrous sodium sulfate, and concentrated under reduced pressure. The concentrate was purified by column chromatography (dichloromethane/methanol= 4%) to give 660 mg of light yellow solid. Yield: 87.8%. LC-MS (APCI): m/z = 437.1(M+1)$^+$.

Step 2 Synthesis of compound I-4

Under the protection of nitrogen, XantPhos-Pd-G2 (44 mg) was added to a solution of compound D-3 (220 mg, 0.50 mmol), compound C-2 (121.4 mg, 1.25 mmol) and $Cs_2CO_3$ (325 mg, 1.00 mmol) in anhydrous dioxane (10 mL), and reacted at 100 °C overnight. The reaction was cooled to room temperature, filtered with celite, and the filtrate was concentrated under reduced pressure, added with DCM (200 mL), washed with saturated saline solution (50 mL × 3), dried over anhydrous sodium sulfate, and concentrated under reduced pressure. The concentrate was purified by column chromatography (dichloromethane/methanol= 5%) to give 205 mg of brown solid, as racemic compound I-4. LC-MS (APCI): m/z = 498.3(M+1)$^+$.

Step 3 Synthesis of compound I-4-1 and compound I-4-2

The racemic compound I-4 was resolved by a chiral column to afford compound I-4-1 and compound I-4-2.

**Example 9 Preparation of 7-(3,4-difluorobenzyl)-6-((methoxy-*d₃*)-methyl)-2-(5-methyl-2-((1-(methyl-*d₃*)-1*H*-pyrazol-5-yl)amino)pyrimidin-4-yl)-6,7-dihydroimidazo[1,2-a]pyrazin-8(5H)-one (Compound 1-5),**

**(*R*)-7-(3,4-difluorobenzyl)-6-((methoxy-*d₃*)-methyl)-2-(5-methyl-2-((1-(methyl-*d₃*)-1*H*-pyrazol-5-yl)amino)pyrimidin-4-yl)-6,7-dihydroimidazo [1,2-a] pyrazin-8(SH)-one (Compound 1-5-1),**

**(*S*)-7-(3,4-difluorobenzyl)-6-((methoxy-*d₃*)-methyl)-2-(5-methyl-2-((1-(methyl-*d₃*)-1*H*-pyrazol-5-yl)amino)pyrimidin-4-yl)-6,7-dihydroimidazo [1,2-a] pyrazin-8(SH)-one (Compound 1-5-2).**

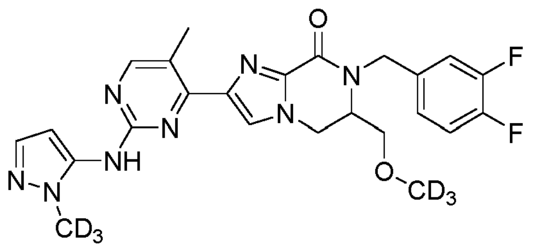

I-5,

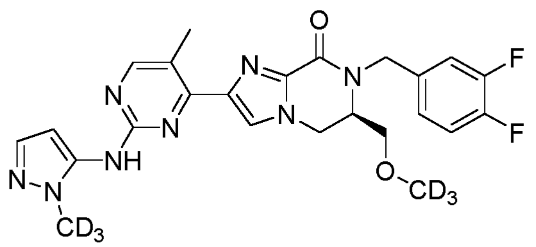

I-5-1,

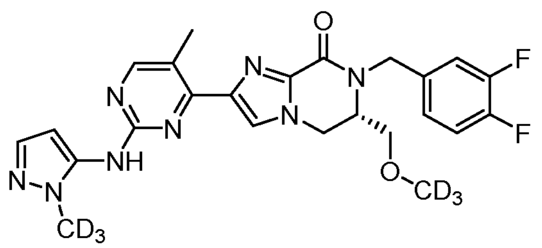

I-5-2

The following route was used for the synthesis:

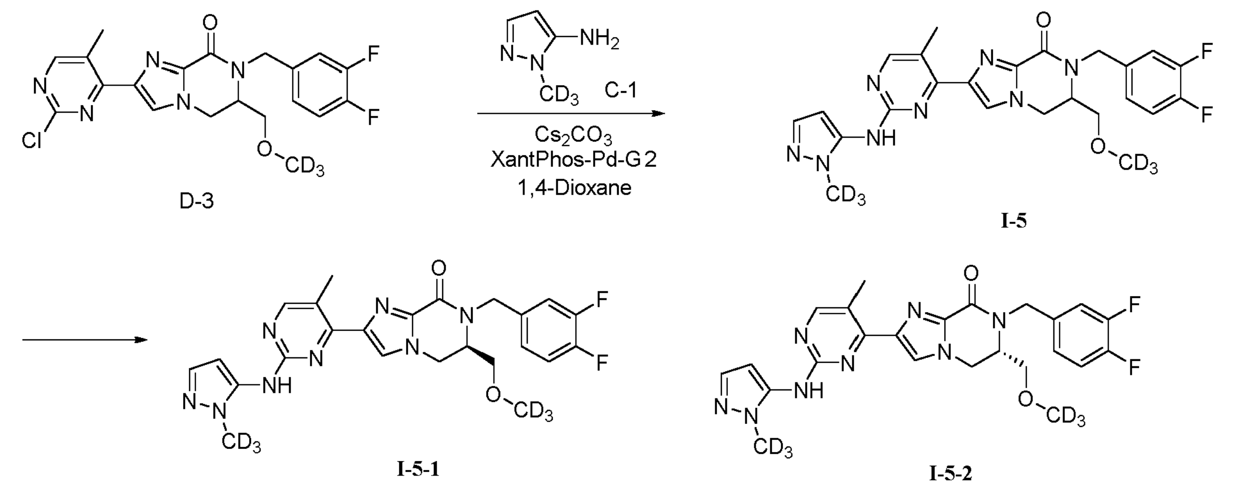


D-3 I-5 I-5-1 I-5-2

Step 1 Synthesis of compound I-5

Under the protection of nitrogen, XantPhos-Pd-G2 (44 mg) was added to a solution of compound D-3 (220 mg, 0.50 mmol), compound C-1 (125 mg, 1.25 mmol) and $Cs_2CO_3$ (325 mg, 1.00 mmol) in anhydrous dioxane (10 mL), and reacted at 100 °C overnight. The reaction was cooled to room temperature, filtered with celite, and the filtrate was concentrated under reduced pressure, added with DCM (200 mL), washed with saturated saline solution (50 mL × 3), dried over anhydrous sodium sulfate, and concentrated under reduced pressure. The concentrate was purified by column chromatography (dichloromethane/methanol= 5%) to give 225 mg of a brown solid, racemic compound I-5. LC-MS (APCI): m/z = 501.3$(M+1)^+$.

Step 2 Synthesis of compound I-5-1 and compound I-5-2

The racemic compound I-5 was resolved by a chiral column to obtain compound I-5-1 and compound I-5-2.

**Biological activity assay**

(1) Metabolic stability evaluation

Microsome assay: human liver microsomes: 0.5 mg/mL, Xenotech; coenzyme (NADPH/NADH): 1 mM, Sigma Life Science; magnesium chloride: 5 mM, 100 mM phosphate buffer (pH is 7.4).

Preparation of stock solution: a certain amount of powder of the example compound and the reference compound were accurately weighed, and dissolved to 5 mM with DMSO, respectively.

Preparation of phosphate buffer (100mM, pH7.4): Pre-formulated 150 mL 0.5 M potassium dihydrogen phosphate and 700 mL 0.5 M dipotassium hydrogen phosphate solution were mixed, then the pH of the mixture was adjusted to 7.4 with 0.5 M dipotassium hydrogen phosphate solution. Before use, 5-fold dilution was made with ultra-pure water, and magnesium chloride was added to afford phosphate buffer (100 mM), which containing 100 mM potassium phosphate, 3.3 mM magnesium chloride, pH is 7.4.

NADPH regeneration system solution (containing 6.5 mM NADP, 16.5 mM G-6-P, 3 U/mL G-6-P D, 3.3 mM magnesium chloride) was prepared and placed on wet ice before use.

Preparation of stop solution: Acetonitrile solution containing 50 ng/mL propranolol hydrochloride and 200 ng/mL tolbutamide (internal standard). 25057.5 μL phosphate buffer (pH 7.4) was taken into a 50 mL centrifuge tube, and 812.5 μL human liver microsome was added, mixed well to afford liver microsome diluent with a protein concentration of 0.625 mg/mL.

Incubation of the sample: The stock solutions of the corresponding compounds were diluted to 0.25 mM with an aqueous solution containing 70% acetonitrile as working solutions for use. 398 μL of human liver microsome diluent was taken and added to a 96-well incubation plate (N=2), respectively, and 2 μL of 0.25 mM working solution was then added respectively, and mixed well.

Determination of metabolic stability: 300 μL of pre-chilled stop solution was added to each well of a 96-well deep well plate and the plate was placed on ice as a stop plate. The 96-well incubation plate and NADPH regeneration system were placed in a 37 °C water bath box, shook at 100 rpm, and pre-incubated for 5 min. 80 μL of incubation solution was taken from each well of the incubation plate and added to the stop plate, mixed well, and 20 μL of NADPH

regeneration system solution was supplemented, the resulted solution was considered as sample at 0 min. Then 80 μL of NADPH regeneration system solution was added to each well of the incubation plate to start the reaction and started timing. The reaction concentration of the corresponding compound is 1 μM, and the protein concentration is 0.5 mg/mL. 100 μL reaction solution was taken at 10, 30, 90 min of reaction, respectively, and added into stop plate, the reaction was stopped with 3 min vortex. The stop plate was centrifuged at 5000×g, 4 °C for 10 min. 100 μL of supernatant was taken to a 96-well plate where 100 μL of distilled water was added in advance, mixed well, sample analysis was subjected by LC-MS/MS.

Data analysis: LC-MS/MS system was used to detect peak area of corresponding compound and internal standard, peak area ratio of compound and internal standard was calculated. The slope was measured by plotting the natural logarithm of the remaining percentage of the compound against time, $t_{1/2}$ and $CL_{int}$ were calculated according to the following formula, wherein V/M equivalented to 1/ protein concentration.

$$t_{1/2} = -\frac{0.693}{\text{Slope}}, \quad CL_{\text{int}} = \frac{0.693}{t_{1/2}} \bullet \frac{V}{M}, \ t_{1/2}(\text{min});\ CL_{\text{int}}(\mu\text{L/min/mg}).$$

The compounds of the present disclosure and the compound without deuteration were tested at the same time and compared to evaluate their metabolic stability in human liver microsomes. The non-deuterated compound AZD0364 was used as a control. In the human liver microsome experiments, by comparing with the non-deuterated compound AZD0364, the compounds of the present disclosure can significantly improve the metabolic stability. The results of the liver microsome experiments of representative example compounds are shown in Table 1 below.

Table 1:

| Example compounds | HLM | |
|---|---|---|
| | $t_{1/2}$(min) | $CL_{int}$(μL/min/mg) |
| AZD0364 | 143.2 | 9.7 |
| I-1 | 144.0 | 9.6 |
| I-2 | 153.8 | 9.0 |
| I-3 | 216.7 | 6.4 |
| I-4-1 | 172.1 | 8.1 |
| I-4-2 | 157.0 | 8.8 |
| I-5-1 | 179.0 | 7.7 |
| I-5-2 | 299.5 | 4.6 |

(2) Pharmacokinetic experiment in rats

6 male Sprague-Dawley rats (7-8 weeks old, and weighing approximately 210 g) were divided into 2 groups with 3 rats in each group. The rats were intravenously or orally administered a single dose of compounds (10 mg/kg orally) to compare pharmacokinetic differences.

The rats were fed on standard food and water. Fasting was started 16 hours before the test. The drug was dissolved in PEG400 and dimethyl sulfoxide. The blood samples were collected from eyelids at the time points of 0.083 hour, 0.25 hour, 0.5 hour, 1 hour, 2 hours, 4 hours, 6 hours, 8 hours, 12 hours, and 24 hours after administration.

Rats were briefly anesthetized after inhalation of diethyl ether and 300 μL of blood sample was collected from the eyelids into test tubes. There was 30 μL of 1% heparin salt solution in the test tube. Tubes were dried at 60°C overnight before use. After the blood sample was collected at the last time point, the rats were sacrificed after ether anesthesia.

Immediately after the collection of the blood sample, the test tube was gently inverted at least 5 times to ensure sufficient mixing and then placed on ice. The blood sample was centrifuged at 5000 rpm at 4°C for 5 minutes to separate the plasma from the red blood cells. 100 μL of plasma was aspirated into a clean plastic centrifuge tube with a pipette, marking with the name of the compound and time point. Plasma was stored at -80°C prior to analysis. The concentration of the compound of the present disclosure in plasma was determined by LC-MS/MS. The pharmacokinetic parameters were calculated based on the blood concentration of the drug for each animal at different time points.

Experiments showed that the compounds of the present disclosure have better pharmacokinetic properties in

vivo, and therefore have better pharmacodynamics and treatment effects.

(3) ERK2 kinase inhibition

Reagents and materials:

ERK2 kinase (Promega, catalog No. V1961), RET (V804M), Active (Signalchem, catalog No. R02-12GG), ADP-Glo TM kit (Promega, catalog No. V9102), GDC-0994 (MCE, catalog No. HY-15947), ATP (Promega, catalog No. V910B), DMSO (Sigma, catalog No. D8418), DTT (Invitrogen, catalog No. P2325), 384-well plate (Greiner, catalog No. 784075), polypropylene 384-well plate (Labcyte, catalog No. P-05525-BC), polypropylene 96-well plate (Nunc, catalog No. 249944), plate shaker (Thermo, catalog No. 4625-1 CECN/THZ Q), centrifuge (Eppendorf, catalog No. 5810R), multilabel ELISA reader Envision 2104 multi-label Reader (PerkinElmer, catalog No. 74785), Echo acoustic dispenser (Labcyte, catalog No. 550).

Specific experimental methods:

Compound formulation: The test compounds were dissolved in DMSO to prepare a 10 mM stock solution. Then, the stock solution was diluted 10 times with a 3-fold gradient. When addition, 10-fold dilution was made with buffer solution.

ERK2 kinase assay: In 1 × kinase buffer solution, ERK2 kinase was mixed with pre-formulated and diluted compounds of different concentrations for 10 minutes in duplicate for each concentration. The corresponding substrate and ATP were added thereto, and reacted at room temperature for 20 minutes (both a negative and a positive control were set, wherein the negative is blank control, and the positive control is GDC-0994). Detection reagent was added after the reaction is complete (reagent in ADP-Glo TM kit), and after 40 minutes of incubation at room temperature, Envision 2104 multi-label Reader ELISA reader was used for detection of enzyme activities under the presence of compounds of the present disclosure with different concentrations, and the inhibitory activities of different concentrations of compounds on enzyme activity were calculated. GraphPad Prism 6.0 software was used for data analysis, nonlinear curve regression was used to fit the data to get the dose-response curve, thereby $IC_{50}$ values were calculated.

Compounds of the present disclosure were tested in the above kinase inhibition assay, the compounds of the present disclosure are more potent than AZD0364. Results of representative example compounds were summarized in Table 2 below.

Table 2:

| Example compounds | ERK2 kinase $IC_{50}$ (nM) |
|---|---|
| AZD0364 | 4.01 |
| I-3 | 3.31 |
| I-4-1 | 3.09 |
| I-5-1 | 2.75 |

(4) A375 cell assay

The cells in the logarithmic growth phase were taken and the trypan blue exclusion method was used to detect the cell viability to ensure that the cell viability is above 90%. The cell concentration was adjusted, and 150 μL cell suspension was added to a 96-well plate, which was incubated at 37 °C, 5% $CO_2$ for 16 h. The maximum concentration of the drug to be tested was 10 μM, which was diluted into 10 concentrations with a 3-fold gradient dilution. 50 μL drug solution was added to each well of the 96-well plate in duplicate, and incubation was continued for 4 hours. After removing the medium, 0.2 ml cell lysis solution and 1 ml PMSF were added to each well, which was incubated on ice for 30 min, and centrifuged for 10 min at 4 °C to afford cell lysis solution. PathScan®Phospho-p90RSK (Thr359) Sandwich ELISA Kit (CST, catalog No. 91959C) was used for detecting the inhibitory rate of the compounds of the present disclosure on phospho-p90RSK, to each well 100 μL cell lysis solution was added, and incubated at 4 °C overnight. The plate was washed, added with 100 μL reconstituted Detection Antibody, and incubated at 37 °C for 1 h. The plate was washed, added with 100 μL reconstituted HRP-Linked secondary antibody, and incubated at 37 °C for 30 min. The plate was washed, added with 100 μL TMB Substrate, and after incubating at 25 °C for 30 min, 100 μL STOP Solution was added, and EnVision (Perkin Elmer 2104) was used to detect fluorescence value, and inhibitory rates under the effect of different concentrations of compounds were calculated. The calculation of $IC_{50}$ was carried out using Prism Graphpad 8.0. software.

Phospho-p90RSK cell assay was conducted in A375 cell line, which has human malignant melanoma with BRAF V600E mutation that upregulates the MAPK pathway, and therefore has increased endogenous levels of phospho-ERK and phospho-p90RSK. The experimental results show that, as compared with AZD0364, the compounds of the present disclosure have a better inhibitory effect on phospho-p90RSK. Results of representative example compounds were summarized in Table 3 below.

Table 3:

| Example compounds | A375 $IC_{50}$ (nM) |
|---|---|
| AZD0364 | 38.68 |
| I-1 | 30.15 |
| I-3 | 34.64 |
| I-4-1 | 24.93 |
| I-5-1 | 29.65 |

The above is detailed description of the present disclosure in conjunction with specific embodiments, and it cannot be assumed that the specific implementation of the present disclosure is limited to these descriptions

## Claims

1. A compound of formula (I), or tautomers, stereoisomers, crystal forms, pharmaceutically acceptable salts, hydrates or solvates thereof:

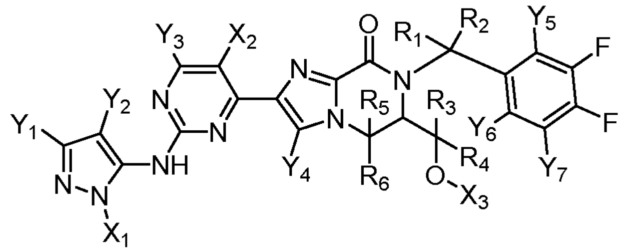

formula (I)

wherein,

$Y_1$, $Y_2$, $Y_3$, $Y_4$, $Y_5$, $Y_6$ and $Y_7$ are each independently selected from hydrogen, deuterium, halogen or trifluoromethyl;
$R_1$, $R_2$, $R_3$, $R_4$, $R_5$ and $R_6$ are each independently selected from hydrogen or deuterium;
$X_1$, $X_2$ and $X_3$ are each independently selected from $CH_3$, $CD_3$, $CHD_2$ or $CH_2D$;
provided that the above compound has at least one deuterium.

2. The compound according to claim 1, which is the compound of formula (II):

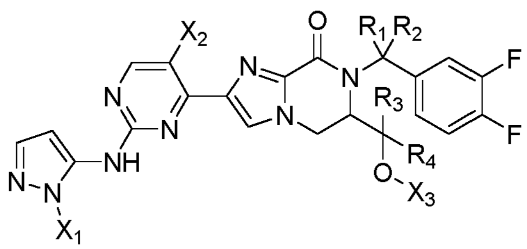

formula (II)

wherein,

$R_1$, $R_2$, $R_3$ and $R_4$ are each independently selected from hydrogen or deuterium;
$X_1$, $X_2$ and $X_3$ are each independently selected from $CH_3$, $CD_3$, $CHD_2$ and $CH_2D$;
provided that the above compound has at least one deuterium;
or the tautomers, stereoisomers, crystal forms, pharmaceutically acceptable salts, hydrates or solvates thereof.

**3.** The compound according to claim 2, which is the compound of formula (III):

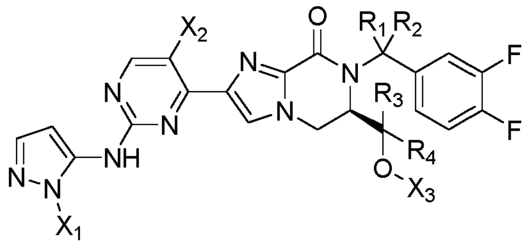

formula (III)

wherein,

$R_1$, $R_2$, $R_3$ and $R_4$ are each independently selected from hydrogen or deuterium;
$X_1$, $X_2$ and $X_3$ are each independently selected from $CH_3$, $CD_3$, $CHD_2$ or $CH_2D$;
provided that the above compound has at least one deuterium;
or the tautomers, stereoisomers, crystal forms, pharmaceutically acceptable salts, hydrates or solvates thereof.

**4.** The compound according to claim 2, which is the compound of formula (IV):

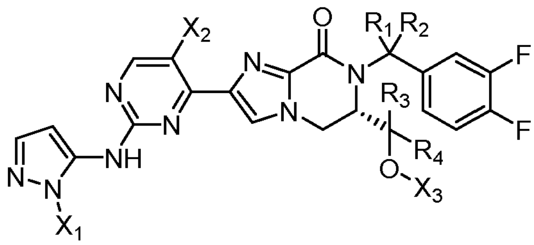

formula (IV)

wherein,

$R_1$, $R_2$, $R_3$ and $R_4$ are each independently selected from hydrogen or deuterium;
$X_1$, $X_2$ and $X_3$ are each independently selected from $CH_3$, $CD_3$, $CHD_2$ or $CH_2D$;
provided that the above compound has at least one deuterium;
or the tautomers, stereoisomers, crystal forms, pharmaceutically acceptable salts, hydrates or solvates thereof.

**5.** The compound according to any one of claims 1-4, wherein, $R_3$ and $R_4$ are hydrogen.

**6.** The compound according to any one of claims 1-5, wherein, $X_2$ is $CH_3$.

**7.** The compound according to any one of claims 1-6, wherein, $R_1$ and $R_2$ are each deuterium.

**8.** The compound according to any one of claims 1-7, wherein, $X_1$ is $CD_3$.

**9.** The compound according to any one of claims 1-8, wherein, $X_2$ is $CD_3$.

**10.** The compound of claim 1, wherein the compound is selected from the following compounds:

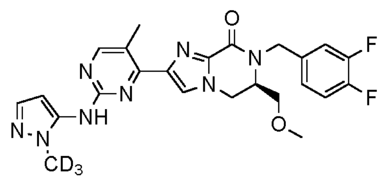,
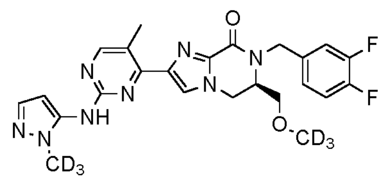,
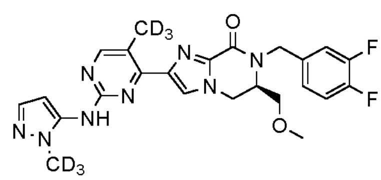,

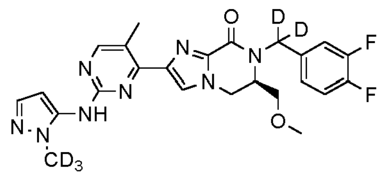,
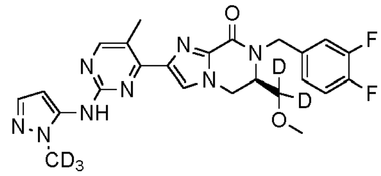,
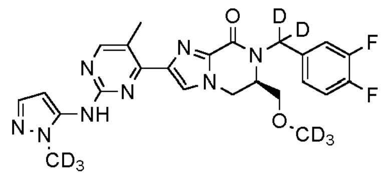,

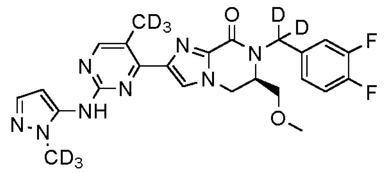,
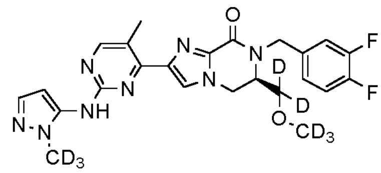,
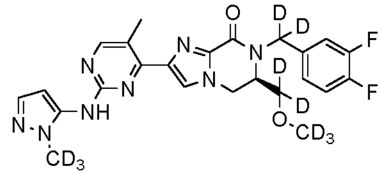,

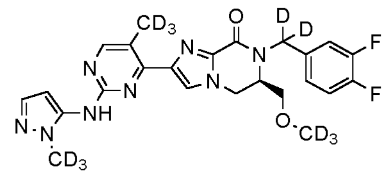,
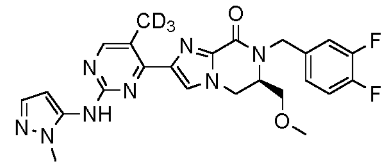,
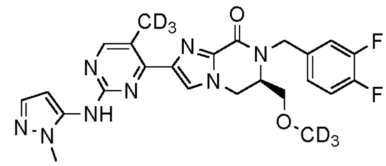,

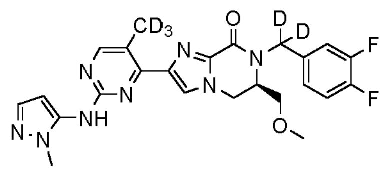,
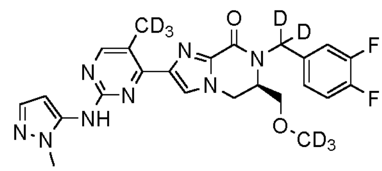,
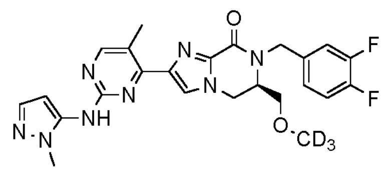,

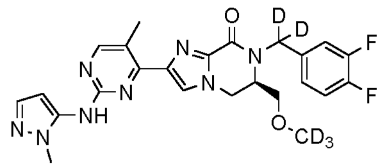,
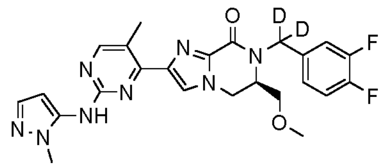,
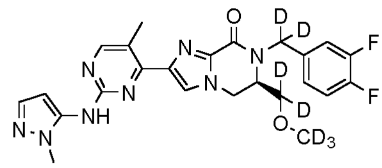,

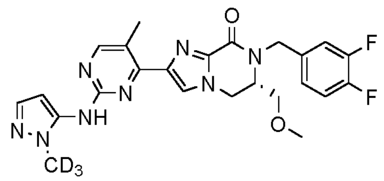,
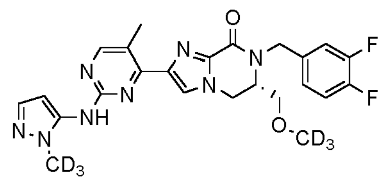,
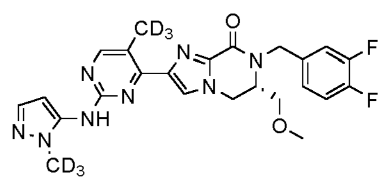,

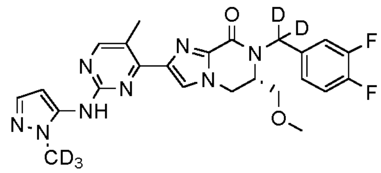 ,
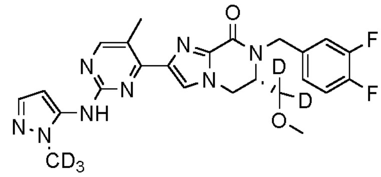 ,
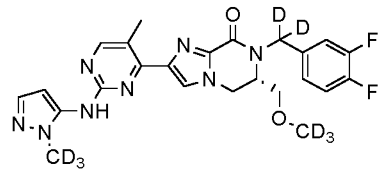 ,

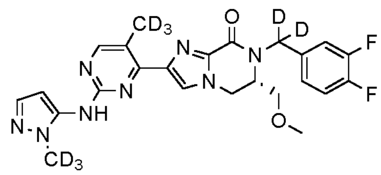 ,
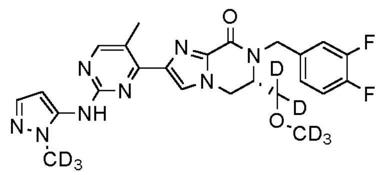 ,
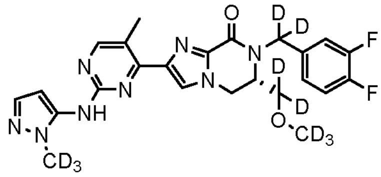 ,

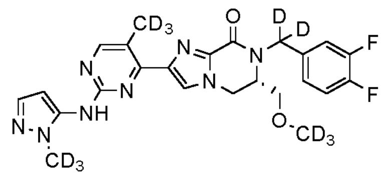 ,
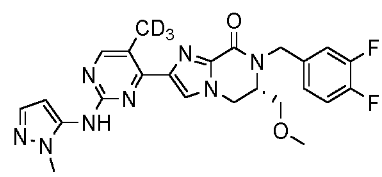 ,
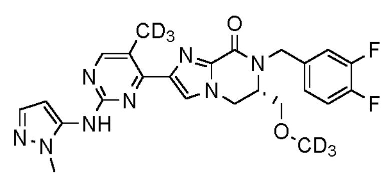 ,

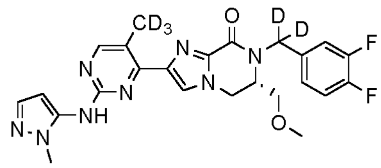 ,
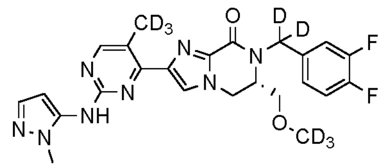 ,
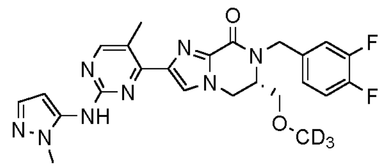 ,

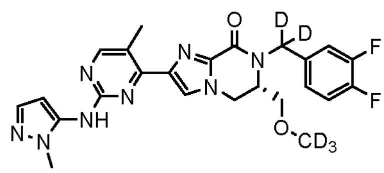 ,
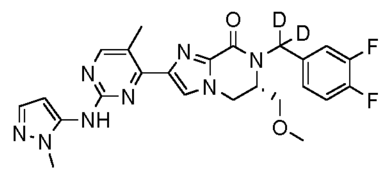 ,
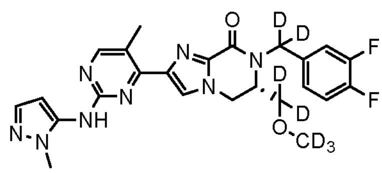 ,

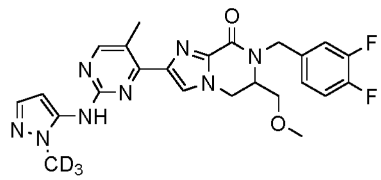 ,
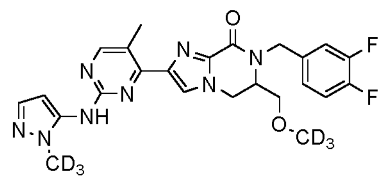 ,
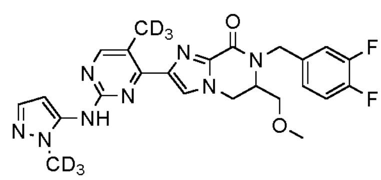 ,

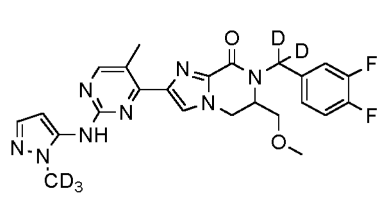 ,
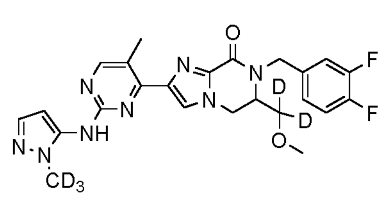 ,
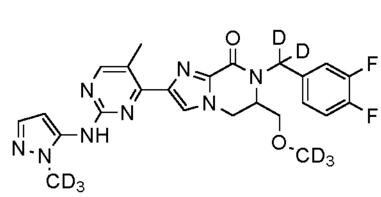 ,

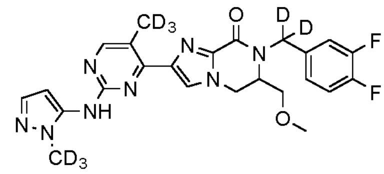, 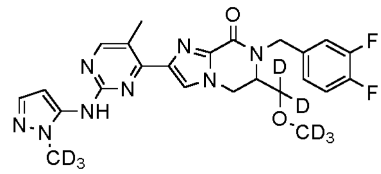, 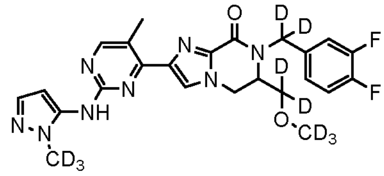,

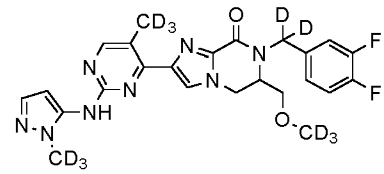, 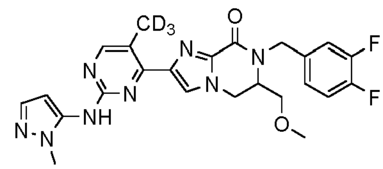, 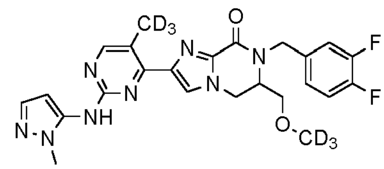,

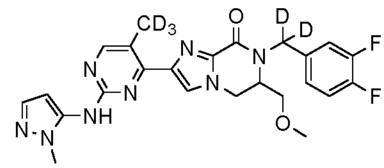, 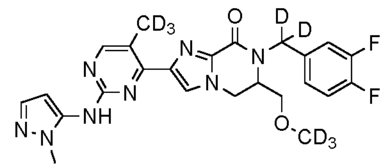, 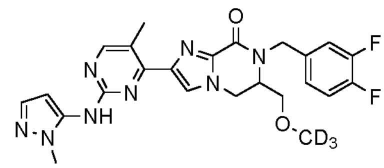,

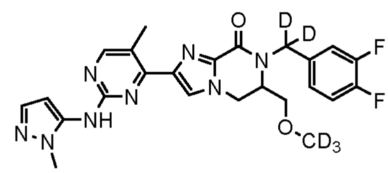, 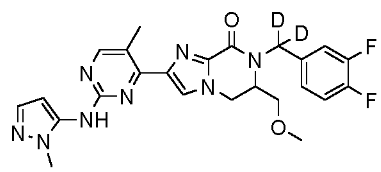, 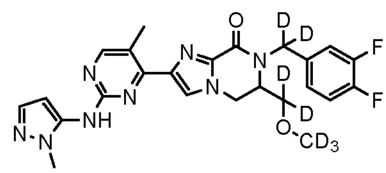.

11. A pharmaceutical composition, comprising pharmaceutically acceptable excipient(s) and a compound or tautomers, stereoisomers, crystal forms, pharmaceutically acceptable salts, hydrates or solvates thereof according to any one of claims 1-10; alternatively, which also comprises other therapeutic agent(s);
alternatively, wherein, the other therapeutic agent(s) is(are) selected from MEK inhibitors.

12. The compound or the tautomers, stereoisomers, crystal forms, pharmaceutically acceptable salts, hydrates or solvates thereof according to any one of claims 1-10 or the composition according to claim 11, for use in the treatment and/or prevention of proliferative diseases selected from non-small cell lung cancer, pancreatic cancer or colorectal cancer.

**Patentansprüche**

1. Verbindung der Formel (I) oder Tautomere, Stereoisomere, Kristallformen, pharmazeutisch verträgliche Salze, Hydrate oder Solvate davon:

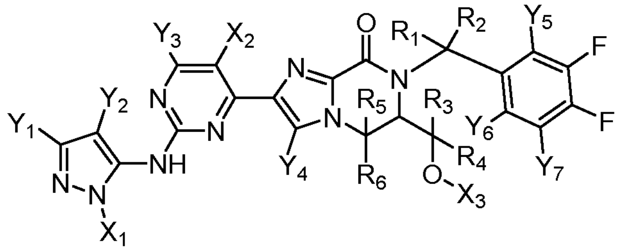

Formel (I)

wobei:

$Y_1$, $Y_2$, $Y_3$, $Y_4$, $Y_5$, $Y_6$ und $Y_7$ jeweils unabhängig aus Wasserstoff, Deuterium, Halogen oder Trifluormethyl ausgewählt sind;
$R_1$, $R_2$, $R_3$, $R_4$, $R_5$ und $R_6$ jeweils unabhängig aus Wasserstoff oder Deuterium ausgewählt sind;
$X_1$, $X_2$ und $X_3$ jeweils unabhängig aus $CH_3$, $CD_3$, $CHD_2$ oder $CH_2D$ ausgewählt sind;
vorausgesetzt, dass die obige Verbindung mindestens ein Deuterium aufweist.

**2.** Verbindung nach Anspruch 1, die die Verbindung der Formel (II) ist:

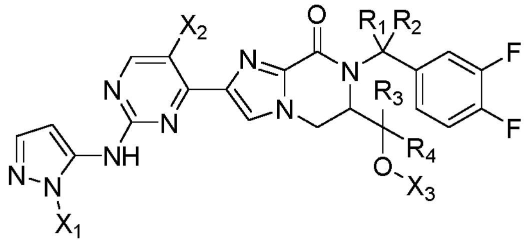

Formel (II)

wobei:

$R_1$, $R_2$, $R_3$ und $R_4$ jeweils unabhängig aus Wasserstoff oder Deuterium ausgewählt sind;
$X_1$, $X_2$ und $X_3$ jeweils unabhängig aus $CH_3$, $CD_3$, $CHD_2$ und $CH_2D$ ausgewählt sind;
vorausgesetzt, dass die obige Verbindung mindestens ein Deuterium aufweist;
oder Tautomere, Stereoisomere, Kristallformen, pharmazeutisch verträgliche Salze, Hydrate oder Solvate davon.

**3.** Verbindung nach Anspruch 2, die die Verbindung der Formel (III) ist:

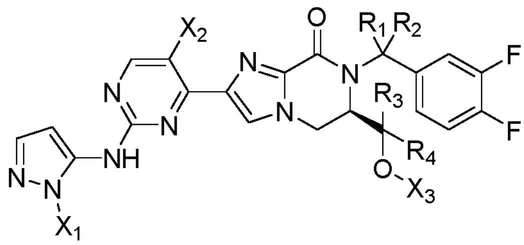

Formel (III)

wobei:

$R_1$, $R_2$, $R_3$ und $R_4$ jeweils unabhängig aus Wasserstoff oder Deuterium ausgewählt sind;
$X_1$, $X_2$ und $X_3$ jeweils unabhängig aus $CH_3$, $CD_3$, $CHD_2$ oder $CH_2D$ ausgewählt sind;
vorausgesetzt, dass die obige Verbindung mindestens ein Deuterium aufweist;
oder Tautomere, Stereoisomere, Kristallformen, pharmazeutisch verträgliche Salze, Hydrate oder Solvate davon.

**4.** Verbindung nach Anspruch 2, die die Verbindung der Formel (IV) ist:

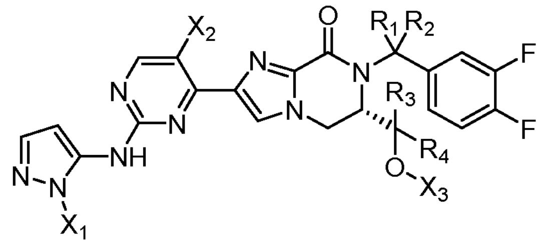

Formel (IV)

wobei:

$R_1$, $R_2$, $R_3$ und $R_4$ jeweils unabhängig aus Wasserstoff oder Deuterium ausgewählt sind;
$X_1$, $X_2$ und $X_3$ jeweils unabhängig aus $CH_3$, $CD_3$, $CHD_2$ oder $CH_2D$ ausgewählt sind;
vorausgesetzt, dass die obige Verbindung mindestens ein Deuterium aufweist;
oder Tautomere, Stereoisomere, Kristallformen, pharmazeutisch verträgliche Salze, Hydrate oder Solvate davon.

**5.** Verbindung nach einem der Ansprüche 1 bis 4, wobei $R_3$ und $R_4$ Wasserstoff sind.

**6.** Verbindung nach einem der Ansprüche 1 bis 5, wobei $X_2$ $CH_3$ ist.

**7.** Verbindung nach einem der Ansprüche 1 bis 6, wobei $R_1$ und $R_2$ jeweils Deuterium sind.

**8.** Verbindung nach einem der Ansprüche 1 bis 7, wobei $X_1$ $CD_3$ ist.

**9.** Verbindung nach einem der Ansprüche 1 bis 8, wobei $X_2$ $CD_3$ ist.

**10.** Verbindung nach Anspruch 1, wobei die Verbindung aus den folgenden Verbindungen ausgewählt ist:

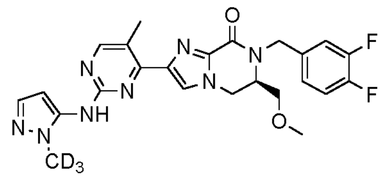 ,
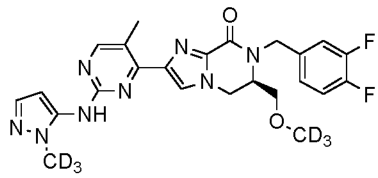 ,
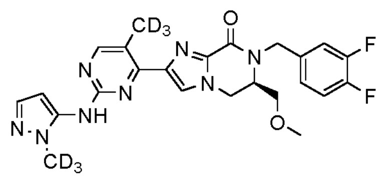 ,

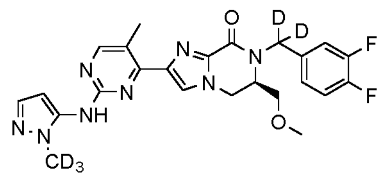 ,
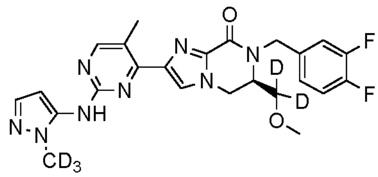 ,
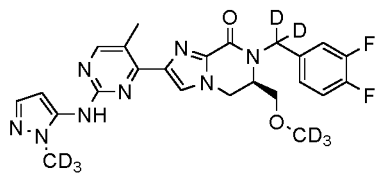 ,

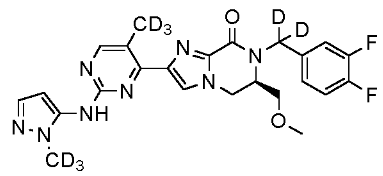 ,
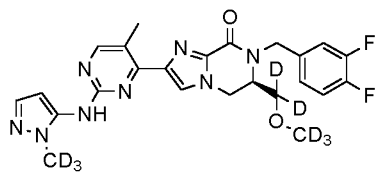 ,
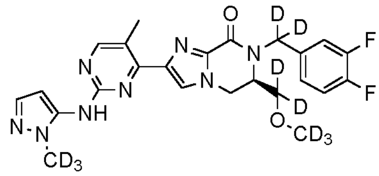 ,

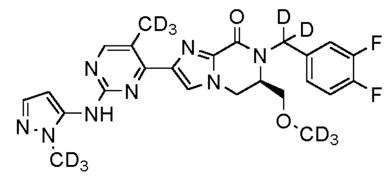,
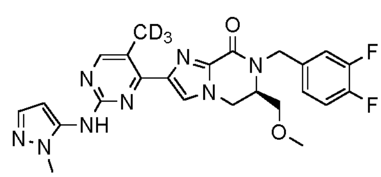,
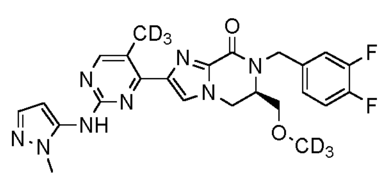,

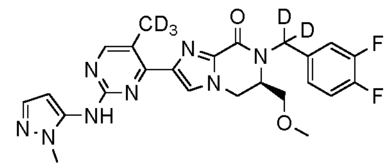,
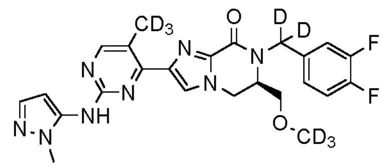,
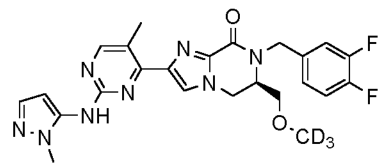,

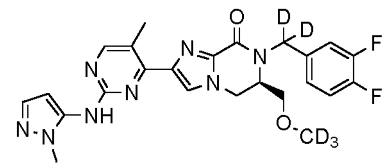,
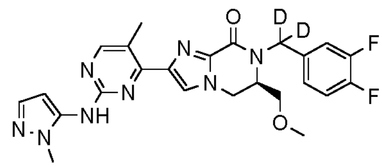,
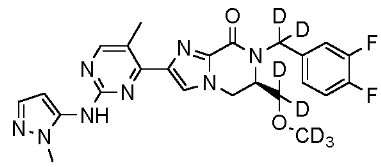,

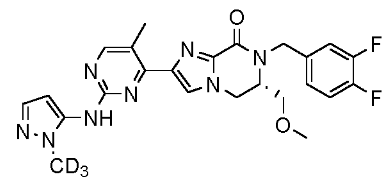,
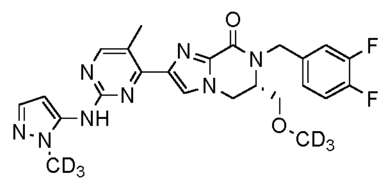,
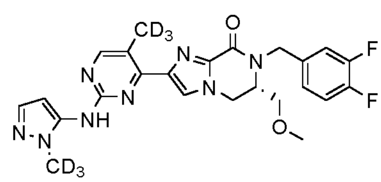,

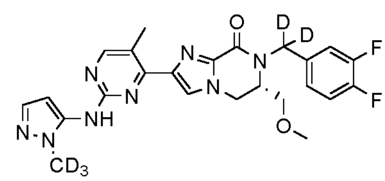,
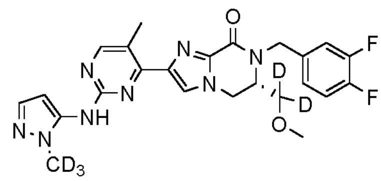,
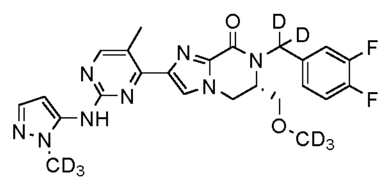,

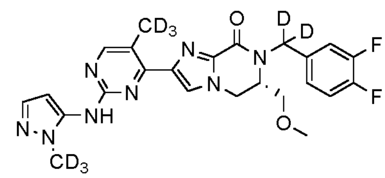,
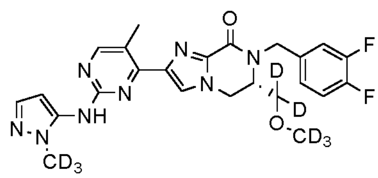,
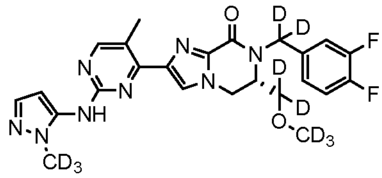,

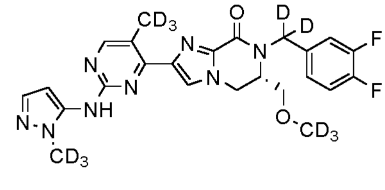,
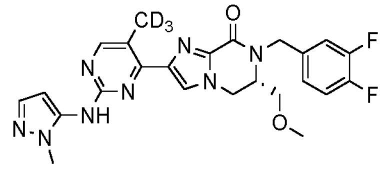,
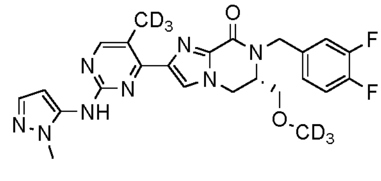,

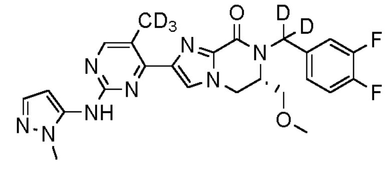,
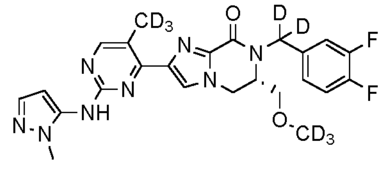,
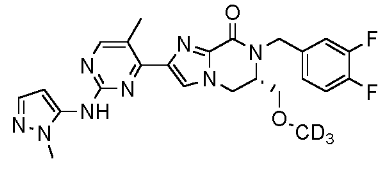,

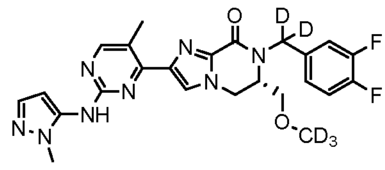 , 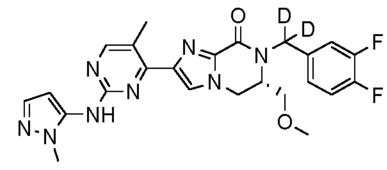 , 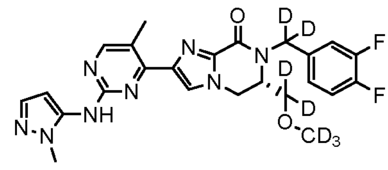 ,

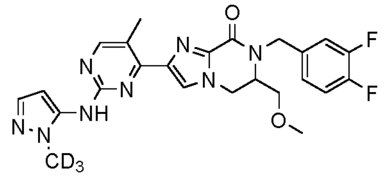 , 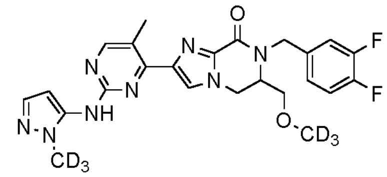 , 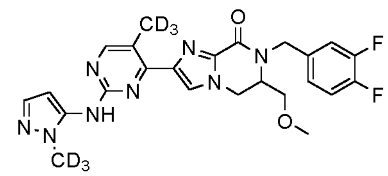 ,

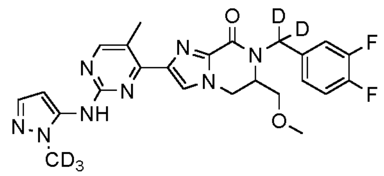 , 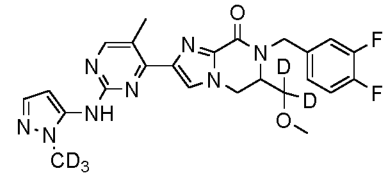 , 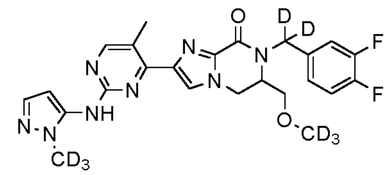 ,

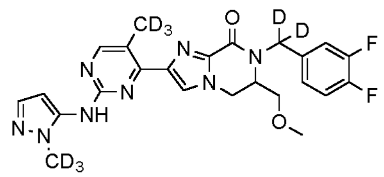 , 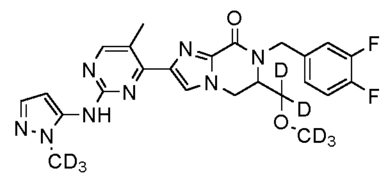 , 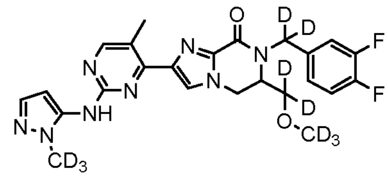 ,

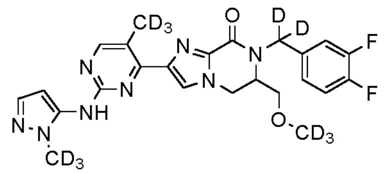 , 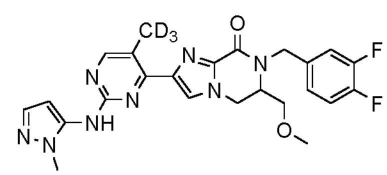 , 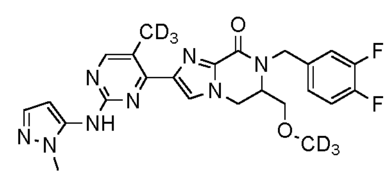 ,

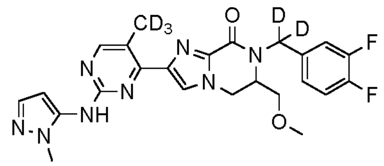 , 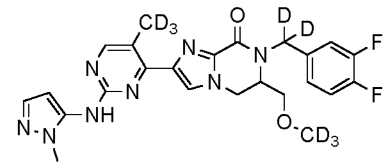 , 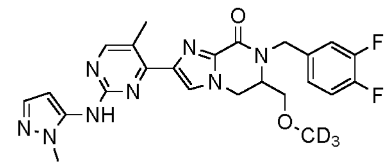 ,

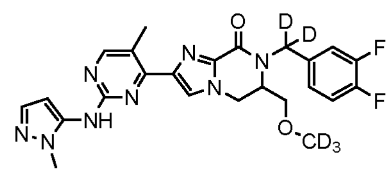 , 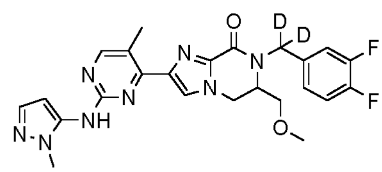 , 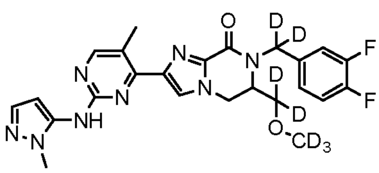 .

**11.** Pharmazeutische Zusammensetzung, umfassend (einen) pharmazeutisch verträgliche(n) Hilfsstoff(e) und eine Verbindung oder Tautomere, Stereoisomere, Kristallformen, pharmazeutisch verträgliche Salze, Hydrate oder Solvate davon nach einem der Ansprüche 1 bis 10; alternativ, die auch (ein) andere(s) therapeutische(s) Mittel umfasst; alternativ, wobei das/die andere(n) therapeutische(n) Mittel aus MEK-Inhibitoren ausgewählt ist/sind.

**12.** Verbindung oder die Tautomere, Stereoisomere, Kristallformen, pharmazeutisch verträglichen Salze, Hydrate oder

Solvate davon nach einem der Ansprüche 1 bis 10 oder die Zusammensetzung nach Anspruch 11 zur Verwendung bei der Behandlung und/oder Prävention von proliferativen Erkrankungen, ausgewählt aus nicht-kleinzelligem Lungenkrebs, Bauchspeicheldrüsenkrebs oder Darmkrebs.

## Revendications

1. Composé de formule (I), ou tautomères, stéréo-isomères, formes cristallines, sels, hydrates ou solvates pharmaceutiquement acceptables de celui-ci :

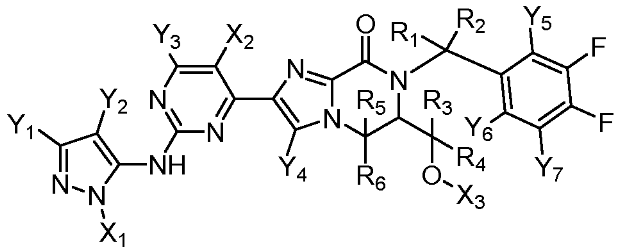

formule (I)

dans lequel,

$Y_1$, $Y_2$, $Y_3$, $Y_4$, $Y_5$, $Y_6$ et $Y_7$ sont chacun indépendamment choisis parmi l'hydrogène, le deutérium, l'halogène ou le trifluorométhyle ;
$R_1$, $R_2$, $R_3$, $R_4$, $R_5$ et $R_6$ sont chacun indépendamment choisis parmi l'hydrogène ou le deutérium ;
$X_1$, $X_2$ et $X_3$ sont chacun indépendamment choisis parmi $CH_3$, $CD_3$, $CHD_2$ ou $CH_2D$ ;
à condition que le composé ci-dessus ait au moins un deutérium.

2. Composé selon la revendication 1, qui est le composé de formule (II) :

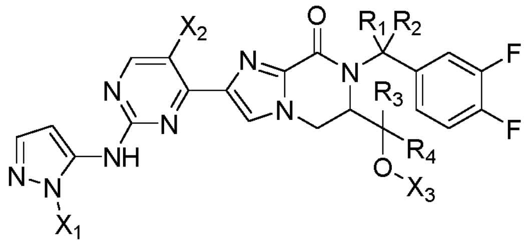

formule (II)

dans lequel,

$R_1$, $R_2$, $R_3$ et $R_4$ sont chacun indépendamment choisis parmi l'hydrogène ou le deutérium ;
$X_1$, $X_2$ et $X_3$ sont chacun indépendamment choisis parmi $CH_3$, $CD_3$, $CHD_2$ et $CH_2D$ ;
à condition que le composé ci-dessus ait au moins un deutérium ;
ou les tautomères, stéréo-isomères, formes cristallines, sels, hydrates ou solvates pharmaceutiquement acceptables de celui-ci.

3. Composé selon la revendication 2, qui est le composé de formule (III) :

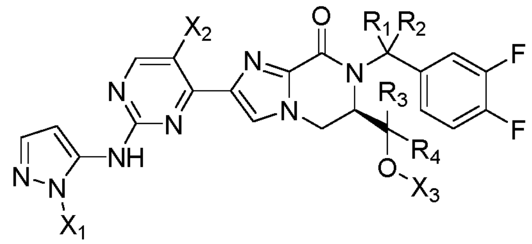

formule (III)

dans lequel,

$R_1$, $R_2$, $R_3$ et $R_4$ sont chacun indépendamment choisis parmi l'hydrogène ou le deutérium ;
$X_1$, $X_2$ et $X_3$ sont chacun indépendamment choisis parmi $CH_3$, $CD_3$, $CHD_2$ ou $CH_2D$ ;
à condition que le composé ci-dessus ait au moins un deutérium ;
ou les tautomères, stéréo-isomères, formes cristallines, sels, hydrates ou solvates pharmaceutiquement acceptables de celui-ci.

**4.** Composé selon la revendication 2, qui est le composé de formule (IV) :

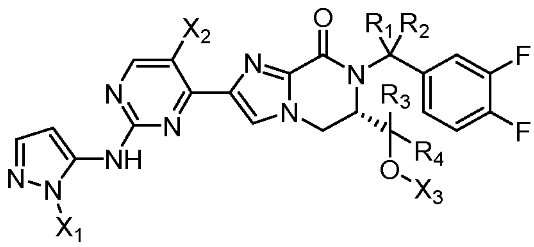

formule (IV)

dans lequel,

$R_1$, $R_2$, $R_3$ et $R_4$ sont chacun indépendamment choisis parmi l'hydrogène ou le deutérium ;
$X_1$, $X_2$ et $X_3$ sont chacun indépendamment choisis parmi $CH_3$, $CD_3$, $CHD_2$ ou $CH_2D$ ;
à condition que le composé ci-dessus ait au moins un deutérium ;
ou les tautomères, stéréo-isomères, formes cristallines, sels, hydrates ou solvates pharmaceutiquement acceptables de celui-ci.

**5.** Composé selon l'une quelconque des revendications 1 à 4, dans lequel $R_3$ et $R_4$ sont un hydrogène.

**6.** Composé selon l'une quelconque des revendications 1 à 5, dans lequel $X_2$ est $CH_3$.

**7.** Composé selon l'une quelconque des revendications 1 à 6, dans lequel $R_1$ et $R_2$ sont chacun un deutérium.

**8.** Composé selon l'une quelconque des revendications 1 à 7, dans lequel $X_1$ est $CD_3$.

**9.** Composé selon l'une quelconque des revendications 1 à 8, dans lequel $X_2$ est $CD_3$.

**10.** Composé selon la revendication 1, dans lequel le composé est choisi parmi les composés suivants :

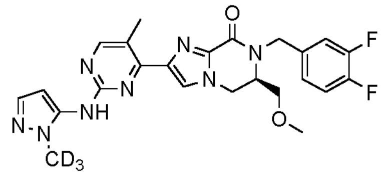,
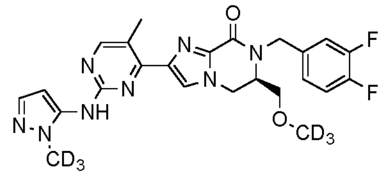,
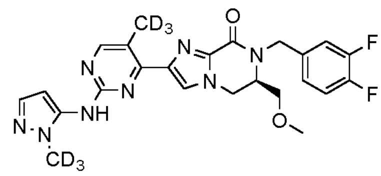,

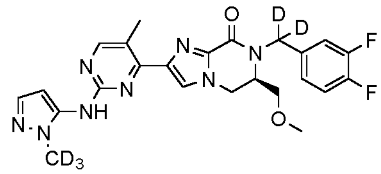,
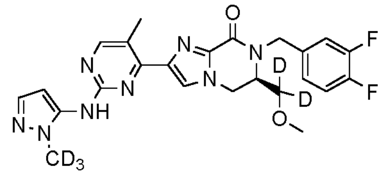,
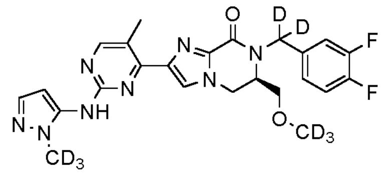,

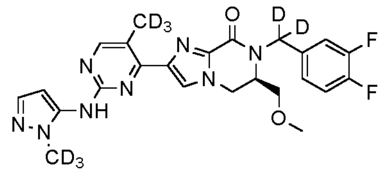,
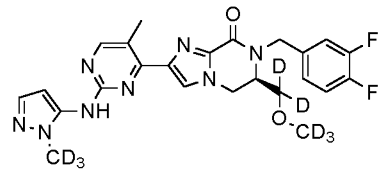,
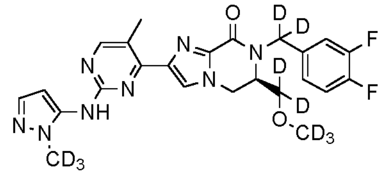,

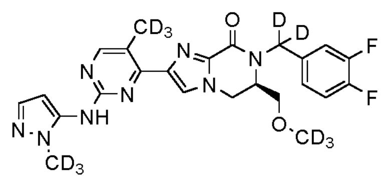,
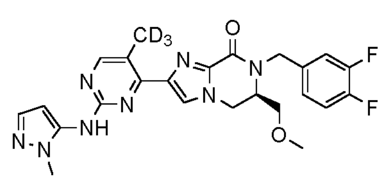,
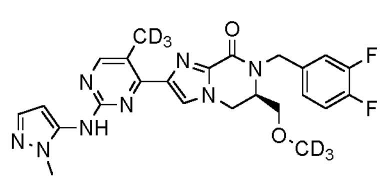,

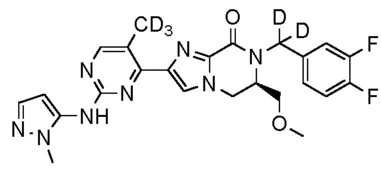,
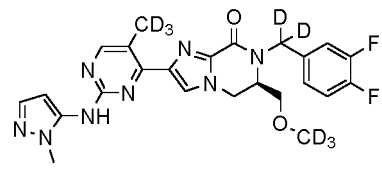,
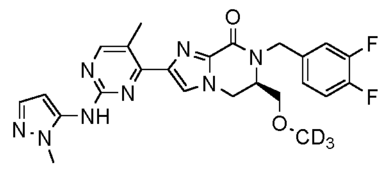,

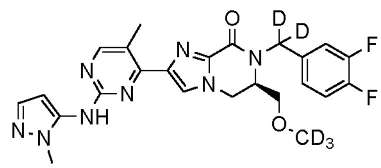,
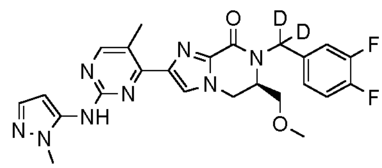,
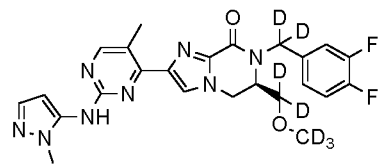,

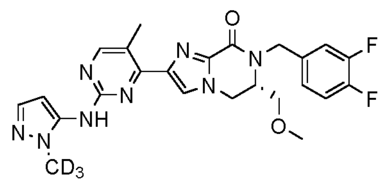,
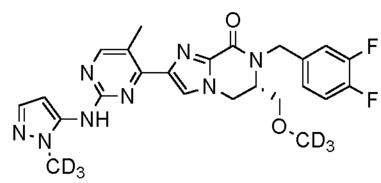,
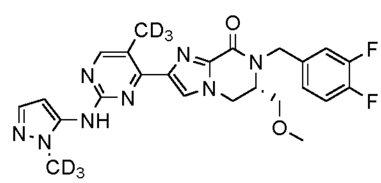,

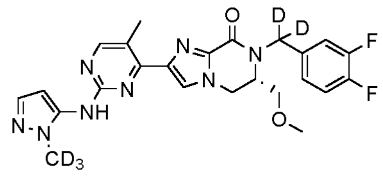,
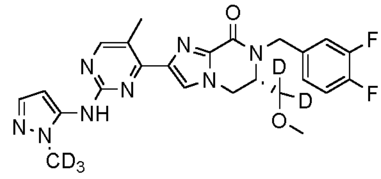,
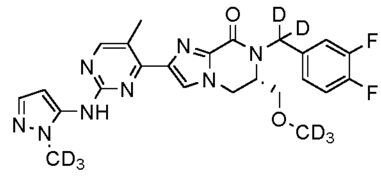,

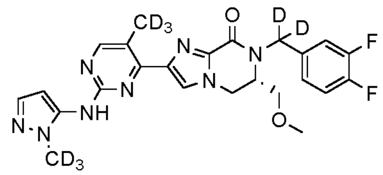,
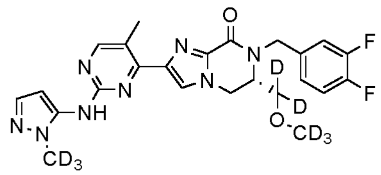,
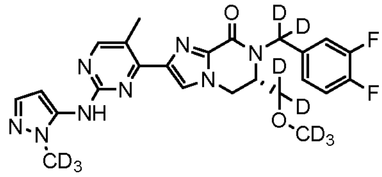,

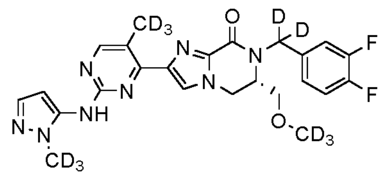,
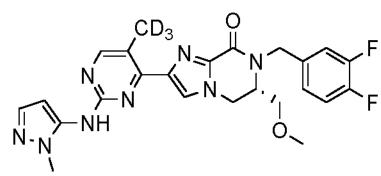,
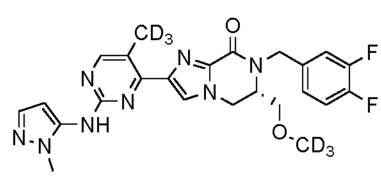,

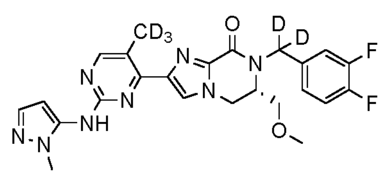,
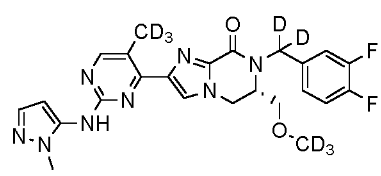,
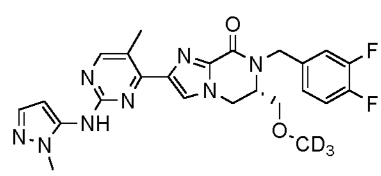,

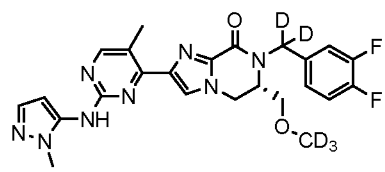,
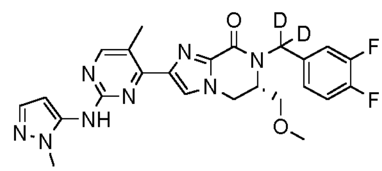,
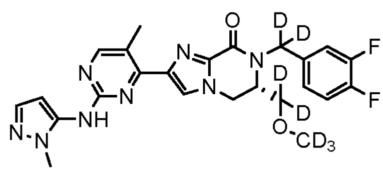,

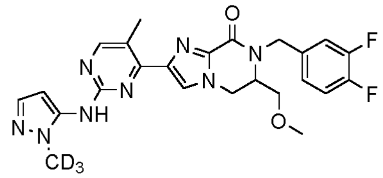,
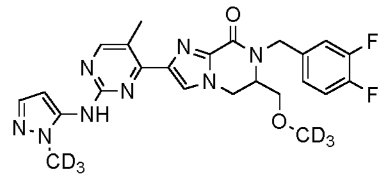,
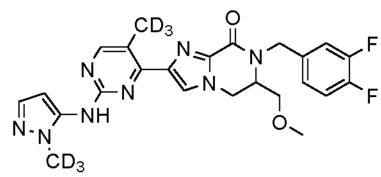,

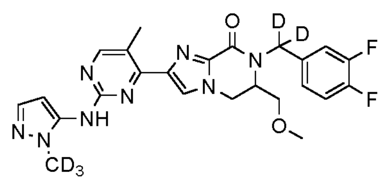,
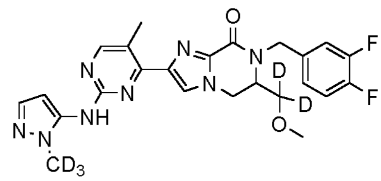,
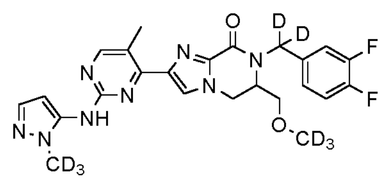,

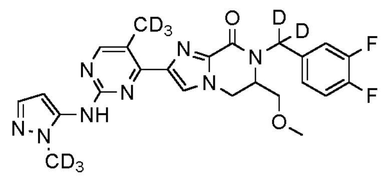, 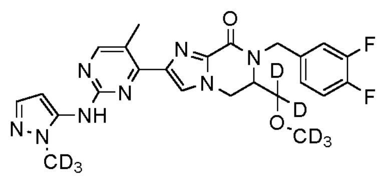, 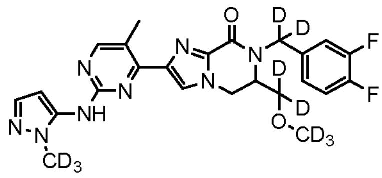,

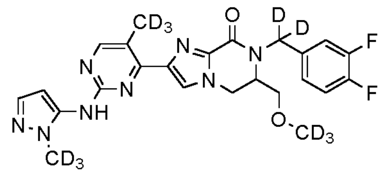, 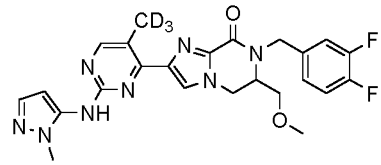, 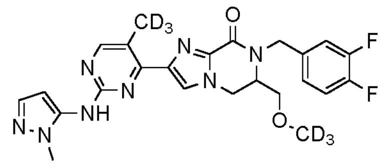,

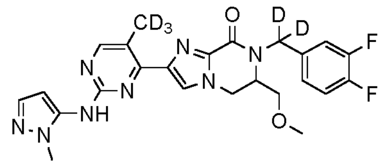, 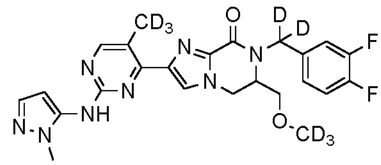, 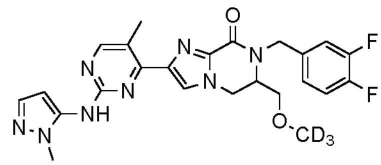,

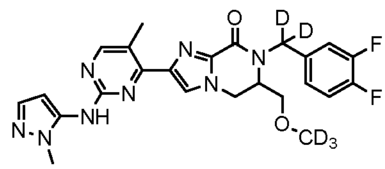, 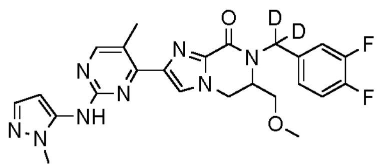, 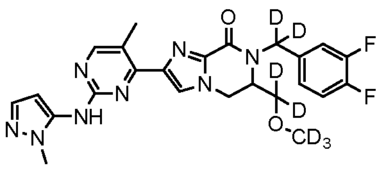.

**11.** Composition pharmaceutique, comprenant un (des) excipient(s) pharmaceutiquement acceptable (s) et un composé ou tautomères, stéréo-isomères, formes cristallines, sels, hydrates ou solvates pharmaceutiquement acceptables de celui-ci selon l'une quelconque des revendications 1 à 10 ; en variante, qui comprend également un (des) autre (s) agent(s) thérapeutique(s) ;
en variante, dans laquelle, l'autre ou les autres agents thérapeutiques est/sont choisis parmi les inhibiteurs de MEK.

**12.** Composé ou tautomères, stéréo-isomères, formes cristallines, sels, hydrates ou solvates pharmaceutiquement acceptables de celui-ci selon l'une quelconque des revendications 1 à 10 ou composition selon la revendication 11, destiné à être utilisé dans le traitement et/ou la prévention de maladies prolifératives choisies parmi le cancer du poumon à grandes cellules, le cancer du pancréas ou le cancer colorectal.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2017080979 A
- US 5376645 A
- WO 20150832840 A


### Non-patent literature cited in the description


- **YOON S ; SEGER R.** The extracellular signal-regulated kinase: multiple substrates regulate diverse cellular functions. *Growth Factors,* 2006, vol. 24, 21-44
- **BERGE et al.** *J. Pharmaceutical Sciences,* 1977, vol. 66, 1-19
- **WUTS ; GREENE.** Protective Groups in Organic Synthesis. John Wiley & Sons, 2006
- **JEAN JACQUES ; ANDRE COLLET ; SAMUE1 H. WILEN.** Enantiomers, Racemates and Resolutions. John Wiley And Sons, Inc, 1981
- Remington's Pharmaceutical Sciences. Mack Publishing Company
- **STERN et al.** *Critical Reviews in Oncology/Haematology,* 2005, vol. 54, 11-29
- *J. Med. Chem.,* 2004, vol. 47, 6658-6661